# EUROPEAN PATENT APPLICATION

(11) **EP 4 495 115 A1**
(43) Date of publication of application: **22.01.2025**
(21) Application number: 23769879.0
(22) Date of filing: 16.03.2023
(51) Int. Cl.: C07D 455/03, A61K 31/4375, A61P 35/00

(54) **TETRACYCLIC COMPOUND AND USE THEREOF**

(30) Priority: 16.03.2022 CN 202210256272; 16.03.2022 CN 202210256355; 05.05.2022 CN 202210482536; 05.05.2022 CN 202210481050; 05.05.2022 CN 202210482528; 12.12.2022 CN 202211590192
(71) Applicant: Shanghai Shijiang Biotechnology Co., Ltd, Shanghai 200333 (CN)
(72) Inventor: SHI, Yufeng, Nanjing, Jiangsu 210032 (CN); MA, Wenjiang, Nanjing, Jiangsu 210032 (CN); PEI, Gang, Nanjing, Jiangsu 210032 (CN); XIA, Peng, Nanjing, Jiangsu 210032 (CN); WANG, Yingcai, Nanjing, Jiangsu 210032 (CN)
(74) Representative: reuteler & cie SA
(86) International application number: PCT/CN2023/081936
(87) International publication number: WO 2023/174377

(57) **Abstract**

The present invention relates to a tetracyclic compound and a use thereof. Specifically, the present invention provides a use of a compound of formula I, or an optical isomer, a racemate, a solvate, a pharmaceutically acceptable salt, or a deuterated compound thereof in the preparation of a composition or a formulation for preventing and/or treating tumors. The compound of the present invention has an excellent precise treatment effect on tumors with low or no expression and low or no activity of mitochondrial membrane permeability pores, low or no expression and low or no activity of recombinant peptidylprolyl isomerase F, low or no expression of NNMT gene, high expression of DNA methylases, high expression of UHRF 1, a high methylation level at a nucleotide site of the NNMT gene, and/or a high methylation level at a DNA CpG site of a NNMT gene region.

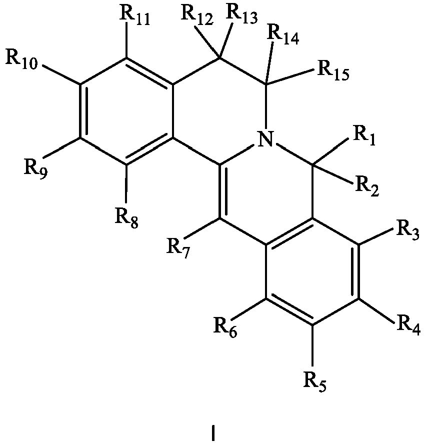

## Description

### FIELD OF THE INVENTION

The present invention relates to the field of medicine. Specifically, the present invention relates to tetracyclic compound and use thereof.

### BACKGROUND TECHNOLOGY

Tumor is a common disease that seriously endangers human health, and the mortality rate of malignant tumors is rising. Due to the heterogeneity of tumor, simply using the same treatment method or medication based on source or pathological characteristic of tumors can easily lead to improper treatment, which can delay valuable treatment time and opportunities for patient. Therefore, it is very necessary to take precision treatment according to the different features of tumor. With the development of biological technology, tumors are typed at the molecular level such as gene and protein level, etc, and more and more changes in tumor-related gene and the expression and activity of protein have been discovered, changes in tumor-related gene and the expression and activity of protein play an important role in the development of malignant tumors, the discovery and application of biomarkers can provide precise guidance for the application of related drug and make precision treatment of tumor possible, thereby achieving targeted administration of drug, significantly improving treatment effect, reducing the dose of drug and reducing toxic side effects.

Therefore, there is an urgent need in the art to develop a drug that can achieve precision treatment on tumor.

### SUMMARY OF THE INVENTION

The present invention provide a compound, the compound has excellent precision treatment effect on tumor with low expression, no expression, low activity or no activity of mitochondria permeability transition pore, low expression, no expression, low activity or no activity of peptidyl-prolyl cis-trans isomerase F, low or no expression of NNMT gene, high expression of DNA methylase, high expression of UHRFl, high methylation level of nucleotide site of NNMT gene, and/or high methylation level of DNA CpG site of NNMT gene.

In the first aspect of the present invention, it provides a use of the compound of formula I, or an optical isomer thereof, or a racemate thereof, or a solvate thereof, or a pharmaceutically acceptable salt thereof, or a deuterated compound thereof in the preparation of a composition or a preparation for preventing and/or treating tumor; 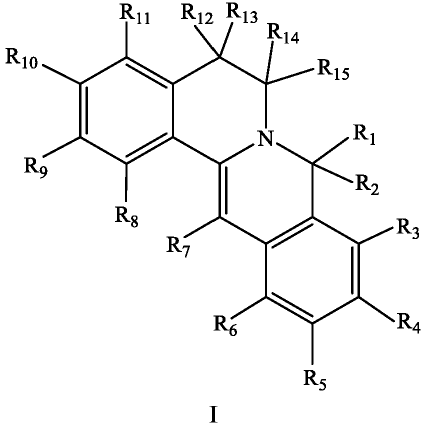 wherein,
R₁ and R₂ are each independently hydrogen, halogen, substituted or unsubstituted C1-C16 alkyl, substituted or unsubstituted C3-C16 cycloalkyl, substituted or unsubstituted 3-12 membered heterocycloalkyl, substituted or unsubstituted C1-C16 haloalkyl, substituted or unsubstituted C3-C16 halocycloalkyl, substituted or unsubstituted C6-C16 aryl-substituted or unsubstituted C1-C10 alkyl-, substituted or unsubstituted 5-16 membered heteroaryl-substituted or unsubstituted C1-C10 alkyl-, substituted or unsubstituted C2-C10 alkenyl-substituted or unsubstituted C1-C10 alkyl-;
R₃ and R₄ are each independently hydrogen, R₁₆-O-, R₁₆-S-;
R₅, R₆, R₇, R₈, R₁₁, R₁₂, R₁₃, R₁₄ and R₁₅ are each independently hydrogen, substituted or unsubstituted C1-C8 alkyl, halogen;
R₉ and R₁₀ are connected to form a substituted or unsubstituted 3-12 membered heterocycloalkane ring;
R₁₆ is hydrogen, substituted or unsubstituted C1-C16 alkyl, substituted or unsubstituted C3-C16 cycloalkyl.

In another preferred embodiment, the heterocyclic ring of the heterocycloalkyl, heteroaryl and heterocycloalkane ring has 1-4 (preferably 1, 2, 3 or 4) heteroatoms independently selected from the group consisting of N, O and S.

In another preferred embodiment, the heterocyclic ring of the heterocycloalkyl has 1-4 (preferably 1, 2, 3 or 4) heteroatoms independently selected from the group consisting of N, O and S.

In another preferred embodiment, the heterocyclic ring of the heteroaryl has 1-4 (preferably 1, 2, 3 or 4) heteroatoms independently selected from the group consisting of N, O and S.

In another preferred embodiment, the heterocyclic ring of the heterocycloalkane ring has 1-4 (preferably 1, 2, 3 or 4) heteroatoms independently selected from the group consisting of N, O and S.

In another preferred embodiment, each "substituted" means that one or more (preferably 1, 2, 3, 4, 5, 6, 7, or 8) hydrogen atoms on the ring or group are independently substituted by substituent selected from the group consisting of C1-C8 alkyl, C3-C12 cycloalkyl, C1-C8 haloalkyl, C3-C8 halocycloalkyl, C3-C8 cycloalkoxyl, C3-C8 cycloalkylthio, C3-C8 halocycloalkoxyl, C3-C8 halocycloalkylthio, halogen, nitro, -CN, hydroxyl, sulfhydryl, amino, C1-C4 carboxyl, C2-C4 ester group, C2-C4 amide group, C1-C8 alkoxyl, C1-C8 alkylthio, C1-C8 haloalkoxyl, C1-C8 haloalkylthio, C6-C12 aryl, C6-C12 aryl-O-, 5-10 membered heteroaryl, 5-10 membered heteroaryl-O-, 5-10 membered heterocycloalkyl.

In another preferred embodiment, each "substituted" means that one or more (preferably 1, 2, 3, 4, 5, 6, 7, or 8) hydrogen atoms on the ring or group are independently substituted by substituent selected from the group consisting of C1-C6 alkyl, C3-C10 cycloalkyl, C1-C6 haloalkyl, C3-C8 halocycloalkyl, C3-C8 cycloalkoxyl, C3-C8 cycloalkylthio, C3-C8 halocycloalkoxyl, C3-C8 halocycloalkylthio, halogen, nitro, -CN, hydroxyl, sulfhydryl, amino, C1-C4 carboxyl, C2-C4 ester group, C2-C4 amide group, C1-C6 alkoxyl, C1-C6 alkylthio, C1-C6 haloalkoxyl, C1-C6 haloalkylthio, C6-C12 aryl, C6-C12 aryl-O-, 5-10 membered heteroaryl, 5-10 membered heteroaryl-O-, 5-10 membered heterocycloalkyl.

In another preferred embodiment, each "substituted" means that one or more (preferably 1, 2, 3, 4, 5, 6, 7, or 8) hydrogen atoms on the ring or group are independently substituted by substituent selected from the group consisting of C1-C4 alkyl, C3-C10 cycloalkyl, C1-C4 haloalkyl, C3-C8 halocycloalkyl, C3-C8 cycloalkoxyl, C3-C8 cycloalkylthio, C3-C8 halocycloalkoxyl, C3-C8 halocycloalkylthio, halogen, nitro, -CN, hydroxyl, sulfhydryl, amino, C1-C4 carboxyl, C2-C4 ester group, C2-C4 amide group, C1-C4 alkoxyl, C1-C4 alkylthio, C1-C4 haloalkoxyl, C1-C4 haloalkylthio, C6-C12 aryl, C6-C12 aryl-O-, 5-10 membered heteroaryl, 5-10 membered heteroaryl-O-, 5-10 membered heterocycloalkyl.

In another preferred embodiment, each "substituted" means that one or more (preferably 1, 2, 3, 4, 5, 6, 7 or 8) hydrogen atoms on the ring or group are each independently substituted by a substituent.

In another preferred embodiment, at least one of R₁ and R₂ is not hydrogen.

In another preferred embodiment, R₁ is hydrogen, and R₂ is not hydrogen.

In another preferred embodiment, R₁ is not hydrogen, and R₂ is hydrogen.

In another preferred embodiment, both R₁ and R₂ are not hydrogen.

In another preferred embodiment, R₁ is not hydrogen.

In another preferred embodiment, R₂ is not hydrogen.

In another preferred embodiment, R₁ and R₂ are the same or different functional groups.

In another preferred embodiment, R₁ and R₂ are each independently hydrogen, halogen, substituted or unsubstituted C1-C12 alkyl, substituted or unsubstituted C3-C12 cycloalkyl, substituted or unsubstituted 3-12 membered heterocycloalkyl, substituted or unsubstituted C1-C12 haloalkyl, substituted or unsubstituted C3-C12 halocycloalkyl, substituted or unsubstituted C6-C12 aryl-substituted or unsubstituted C1-C8 alkyl-, substituted or unsubstituted 5-12 membered heteroaryl-substituted or unsubstituted C1-C8 alkyl-, substituted or unsubstituted C2-C8 alkenyl-substituted or unsubstituted C1-C8 alkyl-.

In another preferred embodiment, R₁ and R₂ are each independently hydrogen, halogen, substituted or unsubstituted C1-C10 alkyl, substituted or unsubstituted C3-C10 cycloalkyl, substituted or unsubstituted 3-10 membered heterocycloalkyl, substituted or unsubstituted C1-C8 haloalkyl, substituted or unsubstituted C3-C10 halocycloalkyl, substituted or unsubstituted C6-C12 aryl-substituted or unsubstituted C1-C6 alkyl-, substituted or unsubstituted 5-10 membered heteroaryl-substituted or unsubstituted C1-C6 alkyl-, substituted or unsubstituted C2-C8 alkenyl-substituted or unsubstituted C1-C8 alkyl-.

In another preferred embodiment, R₁ and R₂ are each independently hydrogen, halogen, substituted or unsubstituted C1-C8 alkyl, substituted or unsubstituted C3-C8 cycloalkyl, substituted or unsubstituted 3-8 membered heterocycloalkyl, substituted or unsubstituted C1-C6 haloalkyl, substituted or unsubstituted C3-C8 halocycloalkyl, substituted or unsubstituted C6-C10 aryl-substituted or unsubstituted C1-C4 alkyl-, substituted or unsubstituted 5-10 membered heteroaryl-substituted or unsubstituted C1-C4 alkyl-, substituted or unsubstituted C2-C6 alkenyl-substituted or unsubstituted C1-C6 alkyl-.

In another preferred embodiment, R₁ and R₂ are each independently hydrogen, halogen, substituted or unsubstituted C1-C8 alkyl, substituted or unsubstituted C3-C8 cycloalkyl, substituted or unsubstituted 3-6 membered heterocycloalkyl, substituted or unsubstituted C1-C6 haloalkyl, substituted or unsubstituted C3-C6 halocycloalkyl, substituted or unsubstituted C6-C10 aryl-substituted or unsubstituted C1-C4 alkyl-, substituted or unsubstituted 5-8 membered heteroaryl-substituted or unsubstituted C1-C4 alkyl-, substituted or unsubstituted C2-C4 alkenyl-substituted or unsubstituted C1-C4 alkyl-.

In another preferred embodiment, R₁ and R₂ are each independently hydrogen, halogen, substituted or unsubstituted C1-C8 alkyl, substituted or unsubstituted C3-C8 cycloalkyl, substituted or unsubstituted 3-6 membered heterocycloalkyl, substituted or unsubstituted C1-C4 haloalkyl, substituted or unsubstituted C3-C6 halocycloalkyl, substituted or unsubstituted C6-C8 aryl-substituted or unsubstituted C1-C4 alkyl-, substituted or unsubstituted 5-8 membered heteroaryl-substituted or unsubstituted C1-C4 alkyl-, substituted or unsubstituted C2-C4 alkenyl-substituted or unsubstituted C1-C4 alkyl-.

In another preferred embodiment, R₁ and R₂ are each independently hydrogen, halogen, substituted or unsubstituted C1-C6 alkyl, substituted or unsubstituted C3-C6 cycloalkyl, substituted or unsubstituted 3-8 membered heterocycloalkyl, substituted or unsubstituted C1-C2 haloalkyl, substituted or unsubstituted C3-C6 halocycloalkyl, substituted or unsubstituted C6-C8 aryl-substituted or unsubstituted C1-C2 alkyl-, substituted or unsubstituted 5-8 membered heteroaryl-substituted or unsubstituted C1-C2 alkyl-, substituted or unsubstituted C2-C4 alkenyl-substituted or unsubstituted C1-C2 alkyl-.

In another preferred embodiment, R₁ and R₂ are each independently hydrogen, substituted or unsubstituted methyl, substituted or unsubstituted ethyl, substituted or unsubstituted propyl, substituted or unsubstituted butyl, substituted or unsubstituted pentyl, substituted or unsubstituted hexyl, substituted or unsubstituted heptyl, substituted or unsubstituted octyl, substituted or unsubstituted ethylenyl-substituted or unsubstituted methyl-, substituted or unsubstituted ethylenyl-substituted or unsubstituted ethyl-, substituted or unsubstituted halomethyl, substituted or unsubstituted cyclopentyl, substituted or unsubstituted cyclohexyl.

In another preferred embodiment, R₁ and R₂ are each independently hydrogen, methyl, ethyl, propyl, butyl, pentyl, hexyl, ethylenyl-methyl-, ethylenyl-ethyl-, halomethyl, cyclopentyl, cyclohexyl.

In another preferred embodiment, the aryl is phenyl.

In another preferred embodiment, the propyl is n-propyl or isopropyl.

In another preferred embodiment, the propyl is

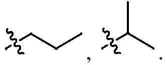

In another preferred embodiment, the butyl is n-butyl.

In another preferred embodiment, the butyl is

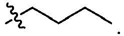

In another preferred embodiment, the hexyl is n- hexyl.

In another preferred embodiment, the hexyl is

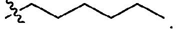

In another preferred embodiment, the ethylenyl-ethyl- is

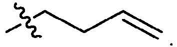

In another preferred embodiment, the cyclopentyl is

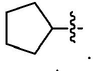

In another preferred embodiment, the cyclohexyl is

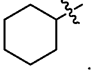

In another preferred embodiment, the halogen is fluorine, chlorine, bromine or iodine.

In another preferred embodiment, the halo is mono-halo, di-halo, tri-halo or full-halo.

In another preferred embodiment, halo is fluoro, chloro, bromo, iodo.

In another preferred embodiment, the halomethyl is trichloromethyl or trifluoromethyl.

In another preferred embodiment, the deuterated is mono-deuterated, di-deuterated, tri-deuterated or fully-deuterated.

In another preferred embodiment, R₃ and R₄ are each independently hydrogen, R₁₆-O-, R₁₆-S-.

In another preferred embodiment, R₅, R₆, R₇, R₈, R₁₁, R₁₂, R₁₃, R₁₄ and R₁₅ are each independently hydrogen, substituted or unsubstituted C1-C8 alkyl, halogen.

In another preferred embodiment, R₅, R₆, R₇, R₈, R₁₁, R₁₂, R₁₃, R₁₄ and R₁₅ are each independently hydrogen, substituted or unsubstituted C1-C6 alkyl, halogen.

In another preferred embodiment, R₅, R₆, R₇, R₈, R₁₁, R₁₂, R₁₃, R₁₄ and R₁₅ are each independently hydrogen, substituted or unsubstituted C1-C4 alkyl, halogen.

In another preferred embodiment, R₉ and R₁₀ are connected to form a substituted or unsubstituted 3-10 membered heterocycloalkane ring.

In another preferred embodiment, R₉ and R₁₀ are connected to form a substituted or unsubstituted 3-8 membered heterocycloalkane ring.

In another preferred embodiment, R₉ and R₁₀ are connected to form a substituted or unsubstituted 5-8 membered heterocycloalkane ring.

In another preferred embodiment, R₉ and R₁₀ are connected to form a substituted or unsubstituted 5-7 membered heterocycloalkane ring.

In another preferred embodiment, R₉ and R₁₀ are connected to form a substituted or unsubstituted 3 membered heterocycloalkane ring, substituted or unsubstituted 4 membered heterocycloalkane ring, substituted or unsubstituted 5 membered heterocycloalkane ring, substituted or unsubstituted 6 membered heterocycloalkane ring, substituted or unsubstituted 7 membered heterocycloalkane ring, substituted or unsubstituted 8 membered heterocycloalkane ring, substituted or unsubstituted 9 membered heterocycloalkane ring, substituted or unsubstituted 10 membered heterocycloalkane ring, substituted or unsubstituted 11 membered heterocycloalkane ring, substituted or unsubstituted 12 membered heterocycloalkane ring.

In another preferred embodiment, R₉ and R₁₀ are connected to form a substituted or unsubstituted 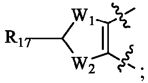
W₁ and W₂ are each independently O or S;
R₁₇ is hydrogen, substituted or unsubstituted C1-C8 alkyl.

In another preferred embodiment, W₁ is O or S.

In another preferred embodiment, W₂ is O or S.

In another preferred embodiment, R₁₆ is hydrogen, substituted or unsubstituted C1-C12 alkyl, substituted or unsubstituted C3-C12 cycloalkyl.

In another preferred embodiment, R₁₆ is hydrogen, substituted or unsubstituted C1-C10 alkyl, substituted or unsubstituted C3-C10 cycloalkyl.

In another preferred embodiment, R₁₆ is hydrogen, substituted or unsubstituted C1-C8 alkyl, substituted or unsubstituted C3-C8 cycloalkyl.

In another preferred embodiment, R₁₆ is hydrogen, substituted or unsubstituted C1-C6 alkyl, substituted or unsubstituted C3-C6 cycloalkyl.

In another preferred embodiment, R₁₆ is hydrogen, substituted or unsubstituted C1-C4 alkyl, substituted or unsubstituted C3-C6 cycloalkyl.

In another preferred embodiment, R₁₆ is hydrogen, substituted or unsubstituted C1-C2 alkyl, substituted or unsubstituted C3-C6 cycloalkyl.

In another preferred embodiment, R₁₆ is halogen, substituted or unsubstituted methyl, substituted or unsubstituted ethyl, substituted or unsubstituted propyl, substituted or unsubstituted butyl.

In another preferred embodiment, R₁₆ is halogen, methyl.

In another preferred embodiment, R₁₇ is hydrogen, substituted or unsubstituted C1-C8 alkyl.

In another preferred embodiment, R₁₇ is hydrogen, substituted or unsubstituted C1-C6 alkyl.

In another preferred embodiment, R₁₇ is hydrogen, substituted or unsubstituted C1-C4 alkyl.

In another preferred embodiment, the compound of formula I has no optical activity or has optical activity.

In another preferred embodiment, the compound of formula I has no chiral carbon atom or has chiral carbon atom.

In another preferred embodiment, the compound of formula I has one chiral carbon atom.

In another preferred embodiment, the configuration of the compound of formula I is racemate, R configuration (rectus), or S configuration (sinister).

In another preferred embodiment, the compound of formula I has the following structure of formula I-1: 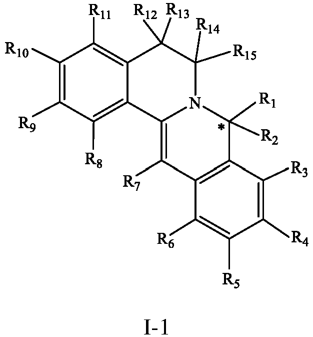 wherein, R₁, R₂, R₃, R₄, R₅, R₆, R₇, R₈, R₉, R₁₀, R₁₁, R₁₂, R₁₃, R₁₄ and R₁₅ are each independently defined as described above.

In another preferred embodiment, "*" represents the configuration of the compound.

In another preferred embodiment, "*" means that the configuration of the compound is racemate, R configuration (rectus), or S configuration (sinister).

In another preferred embodiment, "*" represents a chiral carbon atom.

In another preferred embodiment, the configuration of chiral carbon atom is R configuration (rectus), S configuration (sinister) or racemate.

In another preferred embodiment, the configuration of chiral carbon atom is R configuration (rectus) and/or S configuration.

In another preferred embodiment, the R configuration (rectus) and S configuration of chiral carbon atom refers to racemate.

In another preferred embodiment, the compound of formula I has the following structure of formula 1-2: 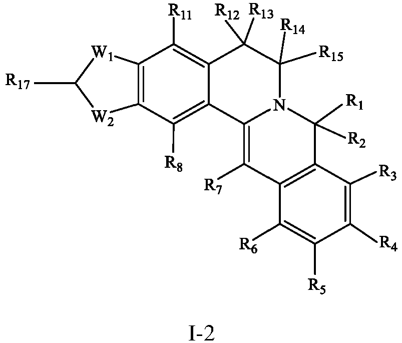 wherein, R₁, R₂, R₃, R₄, R₅, R₆, R₇, R₈, R₁₁, R₁₂, R₁₃, R₁₄, R₁₅, R₁₇, W₁ and W₂ are each independently defined as described above.

In another preferred embodiment, the compound of formula I has the following structure of formula 1-3: 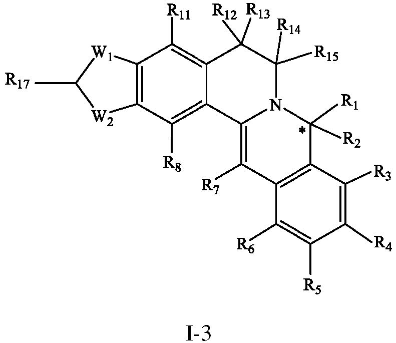 wherein, R₁, R₂, R₃, R₄, R₅, R₆, R₇, R₈, R₁₁, R₁₂, R₁₃, R₁₄, R₁₅, R₁₇, W₁, W₂ and * are each independently defined as described above.

In another preferred embodiment, the pharmaceutically acceptable salt of the compound of formula I comprises the salt formed by the compound of formula I and an acid.

In another preferred embodiment, the acid comprises one or more of hydrochloric acid, mucic acid, D-glucuronic acid, hydrobromic acid, hydrofluoric acid, hydroiodic acid, sulfuric acid, nitric acid, phosphoric acid, formic acid, acetic acid, trifluoroacetic acid, propionic acid, oxalic acid, malonic acid, succinic acid, fumaric acid, maleic acid, lactic acid, malic acid, tartaric acid, citric acid, picric acid, methanesulfonic acid, benzenemethanesulfonic acid, benzenesulfonic acid, aspartic acid and glutamic acid.

In another preferred embodiment, the pharmaceutically acceptable salt of the compound of formula I comprises the salt formed by the compound of formula I and one or more of hydrochloric acid, mucic acid, D-glucuronic acid, hydrobromic acid, hydrofluoric acid, hydroiodic acid, sulfuric acid, nitric acid, phosphoric acid, formic acid, acetic acid, trifluoroacetic acid, propionic acid, oxalic acid, malonic acid, succinic acid, fumaric acid, maleic acid, lactic acid, malic acid, tartaric acid, citric acid, picric acid, methane sulfonic acid, benzenemethanesulfonic acid, benzenesulfonic acid, aspartic acid and glutamic acid.

In another preferred embodiment, the pharmaceutically acceptable salt of the compound of formula I comprises the salt formed by the compound of formula I and hydrochloric acid, mucic acid, D-glucuronic acid, hydrobromic acid, hydrofluoric acid, hydroiodic acid, sulfuric acid, nitric acid, phosphoric acid, formic acid, acetic acid, trifluoroacetic acid, propionic acid, oxalic acid, malonic acid, succinic acid, fumaric acid, maleic acid, lactic acid, malic acid, tartaric acid, citric acid, picric acid, methanesulfonic acid, benzenemethanesulfonic acid, benzenesulfonic acid, aspartic acid or glutamic acid.

In another preferred embodiment, the salt root of the pharmaceutically acceptable salt of the compound of formula I comprises the salt root formed by the loss of a H⁺ in the acid.

In another preferred embodiment, the salt root of the pharmaceutically acceptable salt of the compound of formula I comprises the salt root formed by the loss of a H⁺ in hydrochloric acid, mucic acid, D-glucuronic acid, hydrobromic acid, hydrofluoric acid, hydroiodic acid, sulfuric acid, nitric acid, phosphoric acid, formic acid, acetic acid, trifluoroacetic acid, propionic acid, oxalic acid, malonic acid, succinic acid, fumaric acid, maleic acid, lactic acid, malic acid, tartaric acid, citric acid, picric acid, methanesulfonic acid, benzenemethanesulfonic acid, benzenesulfonic acid, aspartic acid or glutamic acid.

In another preferred embodiment, the salt root of the pharmaceutically acceptable salt of the compound of formula I comprises F-, Cl-, Br-, I-, HCOO-, CH3COO-, SO42-or NO3-.

In another preferred embodiment, the compound of formula I, or an optical isomer thereof, or a racemate thereof, or a solvate thereof, or a pharmaceutically acceptable salt thereof, or a deuterated compound thereof is selected from the following group:

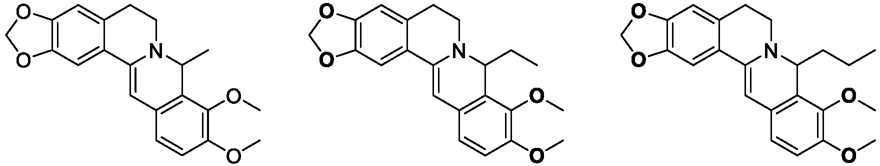

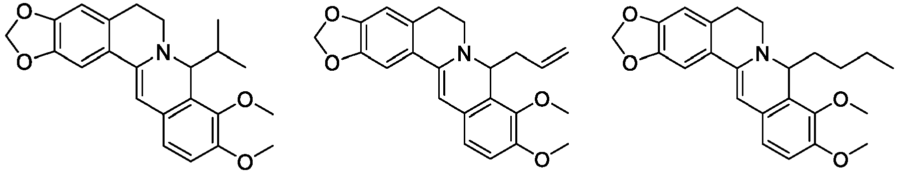

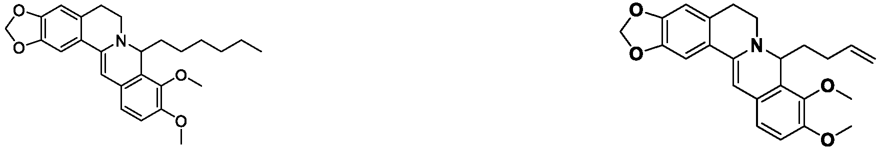

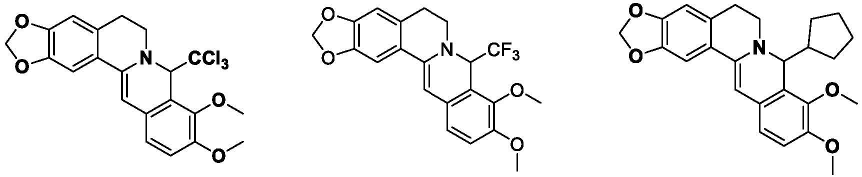

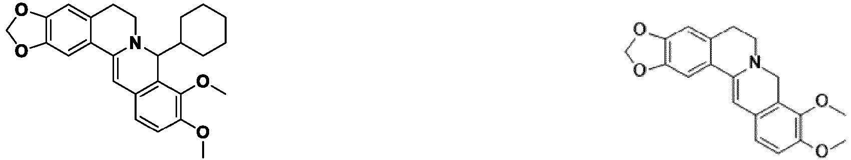

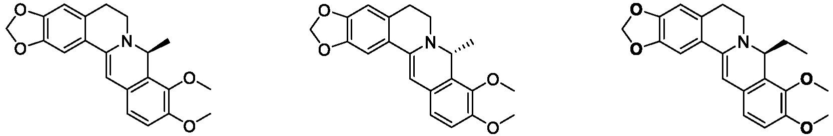

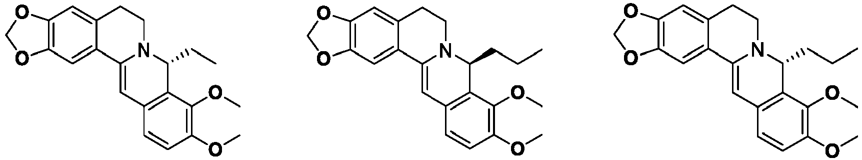

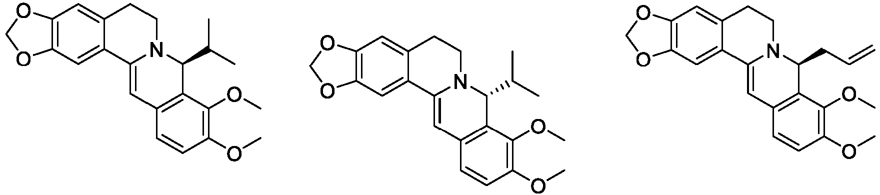

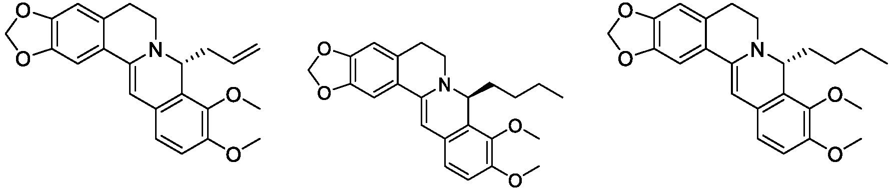

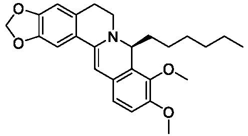

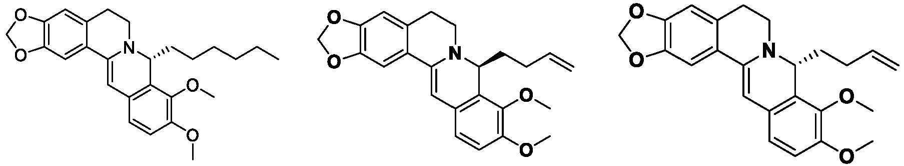

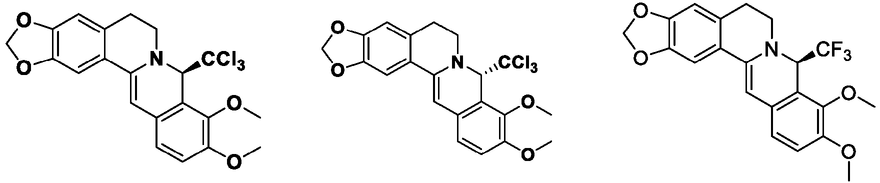

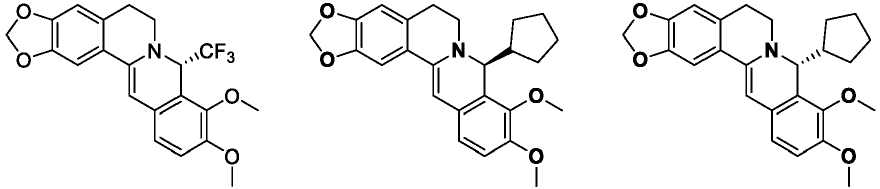

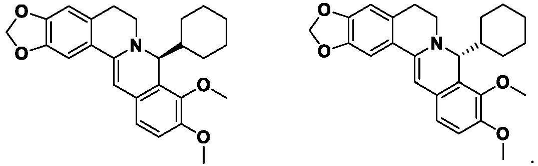

In another preferred embodiment, the tumor is human-derived tumor.

In another preferred embodiment, the tumor is human tumor.

In another preferred embodiment, the tumor comprises tumor with low expression, no expression, low activity or no activity of mitochondria permeability transition pore.

In another preferred embodiment, the tumor comprises tumor with low expression, no expression, low activity or no activity of peptidyl-prolyl cis-trans isomerase F.

In another preferred embodiment, the protein identification number of peptidyl-prolyl cis-trans isomerase F is UniProtKB/Swiss Prot: P30405, and the gene identification number of peptidyl-prolyl cis-trans isomerase F is NCBI Entrez Gene: 10105.

In another preferred embodiment, the tumor with low expression or low activity of mitochondria permeability transition pore means the expression level or activity level of mitochondria permeability transition pore in tumor cell is lower than the expression level or activity level of mitochondria permeability transition pore in the same cell or a normal cell (e.g., para-tumor tissue cell).

In another preferred embodiment, the low expression or low activity of mitochondria permeability transition pore means the ratio (H1/H0) of the expression level or activity level Hlof mitochondria permeability transition pore in a cell ( e.g., tumor cell ) to the expression level or activity level H0 of mitochondria permeability transition pore in the same cell or a normal cell (e.g., para-tumor tissue cell) is <1.0, preferably ≤ 0.8, more preferably ≤ 0.7, more preferably ≤ 0.6, more preferably ≤ 0.5, more preferably ≤ 0.4, more preferably ≤ 0.3, more preferably ≤ 0.2, more preferably ≤ 0.1, more preferably ≤ 0.05, more preferably ≤ 0.01, more preferably ≤ 0.005, more preferably ≤ 0.001, more preferably ≤ 0.0001, more preferably ≤ 0.00001, more preferably ≤ 0.000001, more preferably ≤ 0.0000001.

In another preferred embodiment, the cell comprises tumor cell.

In another preferred embodiment, the cell comprises the same type of cell.

In another preferred embodiment, the same cell comprises the same type of tumor cell.

In another preferred embodiment, the same cell comprises the cell (e.g., the same type of tumor cell) with normal expression, high expression, normal activity or high activity of mitochondria permeability transition pore.

In another preferred embodiment, the same cell comprises the same type of cell (e.g., the same type of tumor cell) with normal expression, high expression, normal activity or high activity of mitochondria permeability transition pore

In another preferred embodiment, the normal cell comprises normal tissue cell (e.g., tumor origin cell, tumor-adjacent cell or para-tumor tissue cell).

In another preferred embodiment, the normal cell comprises normal tissue cell (e.g., tumor origin cell, tumor-adjacent cell or para-tumor tissue cell) with normal expression or normal activity of mitochondria permeability transition pore.

In another preferred embodiment, H0 refers to the expression level or activity level H0 of mitochondria permeability transition pore in the cell with normal expression, high expression, normal activity or high activity of mitochondria permeability transition pore.

In another preferred embodiment, the cell with normal expression, high expression, normal activity or high activity of mitochondria permeability transition pore comprises the cell that is not sensitive to the compound of formula I, or an optical isomer thereof, or a racemate thereof, or a solvate thereof, or a pharmaceutically acceptable salt thereof, or a deuterated compound thereof.

In another preferred embodiment, the tumor with low expression or low activity of peptidyl-prolyl cis-trans isomerase F means the expression level or activity level of peptidyl-prolyl cis-trans isomerase F in tumor cell is lower than the expression level or activity level of peptidyl-prolyl cis-trans isomerase F in the same cell or a normal cell (e.g., para-tumor tissue cell).

In another preferred embodiment, the low expression or low activity of peptidyl-prolyl cis-trans isomerase F means the ratio (P1/P0) of the expression level or activity level Plof peptidyl-prolyl cis-trans isomerase F in a cell ( e.g., tumor cell) to the expression level or activity level P0 of peptidyl-prolyl cis-trans isomerase F in the same cell or a normal cell (e.g., para-tumor tissue cell) is <1.0, preferably ≤ 0.8, more preferably ≤ 0.7, more preferably ≤ 0.6, more preferably ≤ 0.5, more preferably ≤ 0.4, more preferably ≤ 0.3, more preferably ≤ 0.2, more preferably ≤ 0.1, more preferably ≤ 0.05, more preferably ≤ 0.01, more preferably ≤ 0.005, more preferably ≤ 0.001, more preferably ≤ 0.0001, more preferably ≤ 0.00001, more preferably ≤ 0.000001, more preferably ≤ 0.0000001.

In another preferred embodiment, the cell comprises the same type of cell.

In another preferred embodiment, the same cell comprises the same type of tumor cell.

In another preferred embodiment, the same cell comprises the cell (e.g., the same type of tumor cell) with normal expression, high expression, normal activity or high activity of peptidyl-prolyl cis-trans isomerase F.

In another preferred embodiment, the same cell comprises the same type of cell (e.g., the same type of tumor cell) with normal expression, high expression, normal activity or high activity of peptidyl-prolyl cis-trans isomerase F

In another preferred embodiment, the normal cell comprises normal tissue cell (e.g., tumor origin cell, tumor-adjacent cell or para-tumor tissue cell) with normal expression or normal activity of peptidyl-prolyl cis-trans isomerase F.

In another preferred embodiment, P0 refers to the expression level or activity level H0 of peptidyl-prolyl cis-trans isomerase F in the cell with normal expression, high expression, normal activity or high activity of peptidyl-prolyl cis-trans isomerase F.

In another preferred embodiment, the cell with normal expression, high expression, normal activity or high activity of peptidyl-prolyl cis-trans isomerase F comprises the cell that is not sensitive to the compound of formula I, or an optical isomer thereof, or a racemate thereof, or a solvate thereof, or a pharmaceutically acceptable salt thereof, or a deuterated compound thereof.

In another preferred embodiment, the mitochondria permeability transition pore inhibitor is administered to achieve low expression, no expression, low activity or no activity of mitochondria permeability transition pore in tumor.

In another preferred embodiment, the peptidyl-prolyl cis-trans isomerase F inhibitor is administered to achieve low expression, no expression, low activity or no activity of peptidyl-prolyl cis-trans isomerase F.

In another preferred embodiment, the inhibitor comprises specific inhibitor.

In another preferred embodiment, the mitochondria permeability transition pore inhibitor comprises inhibitor that can achieve low expression, no expression, low activity or no activity of mitochondria permeability transition pore in tumor.

In another preferred embodiment, the peptidyl-prolyl cis-trans isomerase F inhibitor comprises inhibitor that can achieve low expression, no expression, low activity or no activity of peptidyl-prolyl cis-trans isomerase F.

In another preferred embodiment, the mitochondria permeability transition pore inhibitor is selected from the group consisting of Cyclosporin A, CyP-D protein inhibitor, peroxide scavenger, and combinations thereof.

In another preferred embodiment, the peptidyl-prolyl cis-trans isomerase F inhibitor comprises shRNA.

In another preferred embodiment, the nucleotide sequence of shRNA is GTTCTTCATCTGCACCATAAA.

In another preferred embodiment, the tumor comprises tumor with low or no expression of NNMT gene.

In another preferred embodiment, the tumor comprises tumor with high expression of DNA methylase.

In another preferred embodiment, the DNA methylase is selected from the group consisting of DNMT1, DNMT3a, DNMT3b, and combinations thereof.

In another preferred embodiment, the tumor comprises tumor with high expression of DNMT1.

In another preferred embodiment, the tumor comprises tumor with high expression of DNMT3a.

In another preferred embodiment, the tumor comprises tumor with high expression of DNMT3b.

In another preferred embodiment, the tumor comprises tumor with high expression of UHRFl.

In another preferred embodiment, the tumor comprises tumor with high methylation level of nucleotide site of NNMT gene and/or high methylation level of DNA CpG site of NNMT gene.

In another preferred embodiment, the tumor comprises tumor with high methylation level of nucleotide site of NNMT gene.

In another preferred embodiment, the methylation of nucleotide site of NNMT gene comprises the methylation of cytosine nucleotide site of NNMT gene.

In another preferred embodiment, the methylation of nucleotide site of NNMT gene comprises the methylation of cytosine of NNMT gene.

In another preferred embodiment, the methylation of nucleotide site of NNMT gene comprises the methylation of the 5'carbon atom on the cytosine of the NNMT gene.

In another preferred embodiment, the tumor comprises tumor with high methylation level of DNA CpG site of NNMT gene.

In another preferred embodiment, the methylation of DNA CpG site of NNMT gene comprises the methylation of cytosine nucleotide site of DNA CpG site of NNMT gene.

In another preferred embodiment, the methylation of DNA CpG site of NNMT gene comprises the methylation of cytosine of DNA CpG site of NNMT gene.

In another preferred embodiment, the methylation of DNA CpG site of NNMT gene comprises the methylation of the 5'carbon atom on the cytosine of DNA CpG site of NNMT gene.

In another preferred embodiment, the NNMT gene is human-derived NNMT gene.

In another preferred embodiment, the NNMT gene is human NNMT gene.

In another preferred embodiment, the tumor with low or no expression of NNMT gene means that no NNMT protein can be detected in 1 µg of protein extracted from tumor using NNMT antibody, preferably in 5 µg of protein extracted from tumor, more preferably in 10 µg of protein extracted from tumor, more preferably in 100 µg of protein extracted from tumor, preferably in 1000 µg of protein extracted from tumor.

In another preferred embodiment, the tumor with low or no expression of NNMT gene means the expression level of NNMT gene in tumor cell is lower than that in the same type of cell or a normal cell.

In another preferred embodiment, the tumor with low or no expression of NNMT gene means the ratio (E1/E0) of the expression level E1 of NNMT gene in the tumor cell to the expression level E0 of NNMT gene in the same type of cell or a normal cell is < 1.0.

In another preferred embodiment, the low or no expression of NNMT gene means the ratio (E1/E0) of the expression E1 of NNMT gene in a cell ( e.g., tumor cell ) to the expression E0 of NNMT gene in the same type of cell or a normal cell is <1.0, preferably ≤ 0.7, more preferably ≤ 0.6, more preferably ≤ 0.5, more preferably ≤ 0.4, more preferably ≤ 0.3, more preferably ≤ 0.2, more preferably ≤ 0.1, more preferably ≤ 0.05, more preferably ≤ 0.01, more preferably ≤ 0.005, more preferably ≤ 0.001, more preferably ≤ 0.0001, more preferably ≤ 0.00001, more preferably ≤ 0.000001, more preferably ≤ 0.0000001.

In another preferred embodiment, the cell comprises tumor cell.

In another preferred embodiment, the same type of cell comprises the same type of tumor cell.

In another preferred embodiment, the same type of cell comprises the cell (e.g., the same type of tumor cell) with normal or high expression of NNMT gene.

In another preferred embodiment, the same type of cell comprises the same type of cell with normal or high expression of NNMT gene.

In another preferred embodiment, the normal cell comprises normal tissue cell (e.g., tumor origin cell, tumor-adjacent cell or para-tumor tissue cell).

In another preferred embodiment, the normal cell comprises normal tissue cell (e.g., tumor origin cell, tumor-adjacent cell or para-tumor tissue cell) with normal expression of NNMT gene.

In another preferred embodiment, E0 refers to the expression level of NNMT gene in the cell with normal or high expression of NNMT gene.

In another preferred embodiment, the cell with normal or high expression of NNMT gene comprises the cell that is not sensitive to the compound of formula I, or an optical isomer thereof, or a racemate thereof, or a solvate thereof, or a pharmaceutically acceptable salt thereof, or a deuterated compound thereof

In another preferred embodiment, the tumor with high expression of DNA methylase means that DNA methylase can be detected in 20 µg of protein extracted from tumor using DNA methylase antibody, preferably in 5 µg of protein extracted from tumor, more preferably in 1 µg of protein extracted from tumor, more preferably in 0.2 µg of protein extracted from tumor, more preferably in 0.05 µg of protein extracted from tumor, more preferably in 0.01 µg of protein extracted from tumor.

In another preferred embodiment, the tumor with high expression of DNA methylase means the expression level of DNA methylase in tumor cell is higher than that in the same type of cell or a normal cell.

In another preferred embodiment, the tumor with high expression of DNA methylase means the ratio (A1/A0) of the expression level A1 of DNA methylase in the tumor cell to the expression level A0 of DNA methylase in the same type of cell or a normal cell is > 1.0, preferably ≥ 1.2, more preferably ≥ 1.5, more preferably ≥ 2, more preferably ≥ 3, more preferably ≥ 5, more preferably ≥ 8, more preferably ≥ 10, more preferably ≥ 15, more preferably ≥ 20, more preferably ≥ 30, more preferably ≥ 50, such as 2-50.

In another preferred embodiment, the same type of cell comprises the same type of tumor cell.

In another preferred embodiment, the same type of cell comprises the cell (e.g., the same type of tumor cell) with normal or low expression of DNA methylase.

In another preferred embodiment, the same type of cell comprises the same type of cell with normal or low expression of DNA methylase.

In another preferred embodiment, the normal cell comprises normal tissue cell (e.g., tumor origin cell, tumor-adjacent cell or para-tumor tissue cell).

In another preferred embodiment, the normal cell comprises normal tissue cell (e.g., tumor origin cell, tumor-adjacent cell or para-tumor tissue cell) with normal expression of DNA methylase.

In another preferred embodiment, A0 refers to the expression level of DNA methylase in the cell with normal or low expression of DNA methylase.

In another preferred embodiment, the cell with normal or low expression of DNA methylase comprises the cell that is not sensitive to the compound of formula I, or an optical isomer thereof, or a racemate thereof, or a solvate thereof, or a pharmaceutically acceptable salt thereof, or a deuterated compound thereof

In another preferred embodiment, the tumor with high expression of DNMT1 means that DNMT1 protein can be detected in 20 µg of protein extracted from tumor using DNMT1 antibody, preferably in 5 µg of protein extracted from tumor, more preferably in 1 µg of protein extracted from tumor, more preferably in 0.2 µg of protein extracted from tumor, more preferably in 0.05 µg of protein extracted from tumor, more preferably in 0.01 µg of protein extracted from tumor.

In another preferred embodiment, the tumor with high expression of DNMT1 means the expression level of DNMT1 in tumor type of cell is higher than that in the same type of cell or a normal cell.

In another preferred embodiment, the tumor with high expression of DNMT1 means the ratio (B1/B0) of the expression level B1 of DNMT1 in the tumor cell to the expression level B0 of DNMT1 in the same type of cell or a normal cell is > 1.0, preferably ≥ 1.2, more preferably ≥ 1.5, more preferably ≥ 2, more preferably ≥ 3, more preferably ≥ 5, more preferably ≥ 8, more preferably ≥ 10, more preferably ≥ 15, more preferably ≥ 20, more preferably ≥ 30, more preferably ≥ 50, such as 2-50.

In another preferred embodiment, the same type of cell comprises the same type of tumor cell.

In another preferred embodiment, the same type of cell comprises the cell (e.g., the same type of tumor cell ) with normal or low expression of DNMT1.

In another preferred embodiment, the same type of cell comprises the same type of cell with normal or low expression of DNMT1.

In another preferred embodiment, the normal cell comprises normal tissue cell (e.g., tumor origin cell, tumor-adjacent cell or para-tumor tissue cell).

In another preferred embodiment, the normal cell comprises normal tissue cell (e.g., tumor origin cell, tumor-adjacent cell or para-tumor tissue cell) with normal expression of DNMT1.

In another preferred embodiment, B0 refers to the expression level of DNMT1 in the cell with normal or low expression of DNMT1.

In another preferred embodiment, the cell with normal or low expression of DNMT1 comprises the cell that is not sensitive to the compound of formula I, or an optical isomer thereof, or a racemate thereof, or a solvate thereof, or a pharmaceutically acceptable salt thereof, or a deuterated compound thereof

In another preferred embodiment, the tumor with high expression of DNMT3a means that DNMT3a protein can be detected in 20 µg of protein extracted from tumor using DNMT3a antibody, preferably in 5 µg of protein extracted from tumor, more preferably in 1 µg of protein extracted from tumor, more preferably in 0.2 µg of protein extracted from tumor, more preferably in 0.05 µg of protein extracted from tumor, more preferably in 0.01 µg of protein extracted from tumor.

In another preferred embodiment, the tumor with high expression of DNMT3a means the expression level of DNMT3a in tumor cell is higher than that in the same type of cell or a normal cell.

In another preferred embodiment, the tumor with high expression of DNMT3a means the ratio (C1/C0) of the expression level C1 of DNMT3a in the tumor cell to the expression level C0 of DNMT3a in the same type of cell or a normal cell is > 1.0, preferably ≥ 1.2, more preferably ≥ 1.5, more preferably ≥ 2, more preferably ≥ 3, more preferably ≥ 5, more preferably ≥ 8, more preferably ≥ 10, more preferably ≥ 15, more preferably ≥ 20, more preferably ≥ 30, more preferably ≥ 50, such as 2-50.

In another preferred embodiment, the same type of cell comprises the same type of tumor cell.

In another preferred embodiment, the same type of cell comprises the cell (e.g., the same type of tumor cell ) with normal or low expression of DNMT3a.

In another preferred embodiment, the same type of cell comprises the same type of cell with normal or low expression of DNMT3a.

In another preferred embodiment, the normal cell comprises normal tissue cell (e.g., tumor origin cell, tumor-adjacent cell or para-tumor tissue cell).

In another preferred embodiment, the normal cell comprises normal tissue cell (e.g., tumor origin cell, tumor-adjacent cell or para-tumor tissue cell) with normal expression of DNMT3a.

In another preferred embodiment, C0 refers to the expression level of DNMT3a in the cell with normal or low expression of DNMT3a.

In another preferred embodiment, the cell with normal or low expression of DNMT3a comprises the cell that is not sensitive to the compound of formula I, or an optical isomer thereof, or a racemate thereof, or a solvate thereof, or a pharmaceutically acceptable salt thereof, or a deuterated compound thereof.

In another preferred embodiment, the tumor with high expression of DNMT3b means that DNMT3b protein can be detected in 20 µg of protein extracted from tumor using DNMT3b antibody, preferably in 5 µg of protein extracted from tumor, more preferably in 1 µg of protein extracted from tumor, more preferably in 0.2 µg of protein extracted from tumor, more preferably in 0.05 µg of protein extracted from tumor, more preferably in 0.01 µg of protein extracted from tumor.

In another preferred embodiment, the tumor with high expression of DNMT3b means the expression level of DNMT3b in tumor type of cell is higher than that in the same type of cell or a normal cell.

In another preferred embodiment, the tumor with high expression of DNMT3b means the ratio (D1/D0) of the expression level D1 of DNMT3b in the tumor cell to the expression level D0 of DNMT3b in the same type of cell or a normal cell is > 1.0, preferably ≥ 1.2, more preferably ≥ 1.5, more preferably ≥ 2, more preferably ≥ 3, more preferably ≥ 5, more preferably ≥ 8, more preferably ≥ 10, more preferably ≥ 15, more preferably ≥ 20, more preferably ≥ 30, more preferably ≥ 50, such as 2-50.

In another preferred embodiment, the same type of cell comprises the same type of tumor cell.

In another preferred embodiment, the same type of cell comprises the cell (e.g., the same type of tumor cell) with normal or low expression of DNMT3b.

In another preferred embodiment, the same type of cell comprises the same type of cell with normal or low expression of DNMT3b.

In another preferred embodiment, the normal cell comprises normal tissue cell (e.g., tumor origin cell, tumor-adjacent cell or para-tumor tissue cell).

In another preferred embodiment, the normal cell comprises normal tissue cell (e.g., tumor origin cell, tumor-adjacent cell or para-tumor tissue cell) with normal expression of DNMT3b

In another preferred embodiment, D0 refers to the expression level of DNMT3b in the cell with normal or low expression of DNMT3b.

In another preferred embodiment, the cell with normal or low expression of DNMT3b comprises the cell that is not sensitive to the compound of formula I, or an optical isomer thereof, or a racemate thereof, or a solvate thereof, or a pharmaceutically acceptable salt thereof, or a deuterated compound.

In another preferred embodiment, the tumor with high expression of UHRF1 means that UHRF1 protein can be detected in 20 µg of protein extracted from tumor using UHRF1 antibody, preferably in 5 µg of protein extracted from tumor, more preferably in 1 µg of protein extracted from tumor, more preferably in 0.2 µg of protein extracted from tumor, more preferably in 0.05 µg of protein extracted from tumor, more preferably in 0.01 µg of protein extracted from tumor.

In another preferred embodiment, the tumor with high expression of UHRF1 means the expression level of UHRF1 in tumor type of cell is higher than that in the same type of cell or a normal cell.

In another preferred embodiment, the tumor with high expression of UHRF1 means the ratio (F1/F0) of the expression level F1 of UHRF1 in the tumor cell to the expression level F0 of UHRF1 in the same type of cell or a normal cell is > 1.0, preferably ≥ 1.2, more preferably ≥ 1.5, more preferably ≥ 2, more preferably ≥ 3, more preferably ≥ 5, more preferably ≥ 8, more preferably ≥ 10, more preferably ≥ 15, more preferably ≥ 20, more preferably ≥ 30, more preferably ≥ 50, such as 2-50.

In another preferred embodiment, the same type of cell comprises the same type of tumor cell.

In another preferred embodiment, the same type of cell comprises the cell (e.g., the same type of tumor cell) with normal or low expression of UHRF1.

In another preferred embodiment, the same type of cell comprises the same type of cell with normal or low expression of UHRF1.

In another preferred embodiment, the normal cell comprises normal tissue cell (e.g., tumor origin cell, tumor-adjacent cell or para-tumor tissue cell).

In another preferred embodiment, the normal cell comprises normal tissue cell (e.g., tumor origin cell, tumor-adjacent cell or para-tumor tissue cell) with normal expression of UHRF1

In another preferred embodiment, F0 refers to the expression level of UHRF1 in the cell with normal or low expression of UHRF1.

In another preferred embodiment, the cell with normal or low expression of UHRF1 comprises the cell that is not sensitive to the compound of formula I, or an optical isomer thereof, or a racemate thereof, or a solvate thereof, or a pharmaceutically acceptable salt thereof, or a deuterated compound.

In another preferred embodiment, the high methylation level of nucleotide site of NNMT gene means the methylation level of nucleotide site of NNMT gene in a cell ( e.g., tumor cell) is higher than that in the same type of cell or a normal cell.

In another preferred embodiment, the high methylation level of nucleotide site of NNMT gene means the ratio (L1/L0) of the methylation level L1 of nucleotide site of NNMT gene in a cell ( e.g., tumor cell ) to the methylation level L0 of nucleotide site of NNMT gene in the same type of cell or a normal cell (e.g., para-tumor tissue cell) is > 1.0, preferably ≥ 1.2, more preferably ≥ 1.5, more preferably ≥ 2, more preferably ≥ 3, more preferably ≥ 5, more preferably ≥ 8, more preferably ≥ 10, more preferably ≥ 15, more preferably ≥ 20, more preferably ≥ 30, more preferably ≥ 50, such as 2-50.

In another preferred embodiment, the high methylation level of nucleotide site of NNMT gene means the methylation level of nucleotide site of NNMT gene in a cell ( e.g., tumor cell ) is ≥ 1%, more preferably ≥ 3%, more preferably ≥ 5%, more preferably ≥ 10%, more preferably ≥ 15%, more preferably ≥ 20%, more preferably ≥ 25%, more preferably ≥ 30%, more preferably ≥ 40%, more preferably ≥ 50%.

In another preferred embodiment, the cell comprises tumor cell.

In another preferred embodiment, the same type cell comprises the same type of tumor cell.

In another preferred embodiment, the same type of cell comprises the cell (e.g., the same type of tumor cell) with normal or low methylation level of nucleotide site of NNMT gene.

In another preferred embodiment, the same type of cell comprises the same type of cell with normal or low methylation level of nucleotide site of NNMT gene.

In another preferred embodiment, the normal cell comprises normal tissue cell (e.g., tumor origin cell, tumor-adjacent cell or para-tumor tissue cell).

In another preferred embodiment, the normal cell comprises normal tissue cell (e.g., tumor origin cell, tumor-adjacent cell or para-tumor tissue cell) with normal methylation level of nucleotide site of NNMT gene.

In another preferred embodiment, the cell with normal or low methylation level of nucleotide site of NNMT gene comprises the cell that is not sensitive to the compound of formula I, or an optical isomer thereof, or a racemate thereof, or a solvate thereof, or a pharmaceutically acceptable salt thereof, or a deuterated compound.

In another preferred embodiment, the high methylation level of nucleotide site of NNMT gene means the methylation level (M%) of nucleotide site of NNMT gene in a cell ( e.g., tumor cell) is ≥ 3% and ≤ M1%, wherein M1 is any positive integer from 3 to 100.

In another preferred embodiment, M1 is 5, 10, 15, 20, 25, 30, 35, 40, 45, 50, 55, 60, 65, 70, 80, 85, 90, 95 or 100.

In another preferred embodiment, the methylation level of nucleotide site of NNMT gene refers to the ratio of the number of methylated nucleotides to the number of all nucleotides in the NNMT gene.

In another preferred embodiment, the methylation level of nucleotide site of NNMT gene comprises the methylation level of nucleotide site in promoter region of NNMT gene.

In another preferred embodiment, the nucleotide sequence of the promoter region of NNMT gene is as shown in SEQ ID NO: 1.

In another preferred embodiment, the methylation level of nucleotide site of NNMT gene comprises the methylation level of nucleotide sites from 1050 bp before the transcription start site to 499 bp after the transcription start site in NNMT gene.

In another preferred embodiment, the sites from 1050 bp before the transcription start site to 499 bp after the transcription start site in NNMT gene is sites 951-2500 of nucleotide sequence as shown in SEQ ID NO: 1.

In another preferred embodiment, the methylation level of nucleotide site of NNMT gene comprises the methylation level of nucleotide sites from 1050 bp to 193 bp before the transcription start site in NNMT gene.

In another preferred embodiment, the sites from 1050 bp to 193 bp before the transcription start site in NNMT gene is sites 951-1808 of nucleotide sequence as shown in SEQ ID NO: 1.

In another preferred embodiment, the methylation level of nucleotide site of NNMT gene comprises the methylation level of nucleotide sites from 840 bp to 469 bp before the transcription start site in NNMT gene.

In another preferred embodiment, the sites from 840 bp to 469 bp before the transcription start site in NNMT gene is sites 1161-1532 of nucleotide sequence as shown in SEQ ID NO: 1.

In another preferred embodiment, the methylation level of nucleotide site of NNMT gene comprises the methylation level of nucleotide site between any two sites (including the two sites itself) selected from group consisting of site 114165695, site 114165730, site 114165769, site 114165804, site 114165938, site 114166050 and site 114166066 on human chromosome 11.

In another preferred embodiment, the methylation level of nucleotide site of NNMT gene comprises the methylation level of nucleotide site of one or more (e.g., 2, 3, 4, 5, 6, or 7 ) of site 114165695, site 114165730, site 114165769, site 114165804, site 114165938, site 114166050 and site 114166066 on human chromosome 11.

In another preferred embodiment, the methylation level of nucleotide site of NNMT gene comprises the methylation level of nucleotide sites selected from group consisting of site 114165695 on human chromosome 11, site 114165730 on human chromosome 11, site 114165769 on human chromosome 11, site 114165804 on human chromosome 11, site 114165938 on human chromosome 11, site 114166050 on human chromosome 11, site 114166066 on human chromosome 11, and combinations thereof.

In another preferred embodiment, the methylation level of nucleotide site of NNMT gene comprises the methylation level of nucleotide site between any two sites (including the two sites itself) selected from group consisting of site 1161, site 1196, site 1235, site 1270, site 1404, site 1516 and site 1532 in nucleotide sequence of SEQ ID NO: 1.

In another preferred embodiment, the methylation level of nucleotide site of NNMT gene comprises the methylation level of nucleotide site of one or more (e.g., 2, 3, 4, 5, 6, or 7 ) of site 1161, site 1196, site 1235, site 1270, site 1404, site 1516 and site 1532 in nucleotide sequence of SEQ ID NO: 1.

In another preferred embodiment, the methylation level of DNA CpG site of NNMT gene comprises the methylation level of nucleotide sites selected from group consisting of site 1161 in SEQ ID NO: 1, site 1196 in SEQ ID NO: 1, site 1235 in SEQ ID NO: 1, site 1270 in SEQ ID NO: 1, site 1404 in SEQ ID NO: 1, site 1516 in SEQ ID NO: 1, site 1532 in SEQ ID NO: 1, and combinations thereof.

In another preferred embodiment, the high methylation level of DNA CpG site of NNMT gene means the methylation level of DNA CpG site of NNMT gene in a cell ( e.g., tumor cell ) is higher than that in the same type of cell or a normal cell.

In another preferred embodiment, the high methylation level of DNA CpG site of NNMT gene means the ratio (W1/W0) of the methylation level W1 of DNA CpG site of NNMT gene in a cell ( e.g., tumor cell) to the methylation level W0 of DNA CpG site of NNMT gene in the same type of cell or a normal cell (e.g., para-tumor tissue cell) is > 1.0, preferably ≥ 1.2, more preferably ≥ 1.5, more preferably ≥ 2, more preferably ≥ 3, more preferably ≥ 5, more preferably ≥ 8, more preferably ≥ 10, more preferably ≥ 15, more preferably ≥ 20, more preferably ≥ 30, more preferably ≥ 50, such as 2-50.

In another preferred embodiment, the high methylation level of DNA CpG site of NNMT gene means the methylation level of DNA CpG site of NNMT gene in a cell ( e.g., tumor cell) is ≥ 1%, more preferably ≥ 3%, more preferably ≥ 5%, more preferably ≥ 10%, more preferably ≥ 15%, more preferably ≥ 20%, more preferably ≥ 25%, more preferably ≥ 30%, more preferably ≥ 40%, more preferably ≥ 50%.

In another preferred embodiment, the cell comprises tumor cell.

In another preferred embodiment, the same type of cell comprises the same type of tumor cell.

In another preferred embodiment, the same type of cell comprises the cell (e.g., the same type of tumor cell) with normal or low methylation level of DNA CpG site of NNMT gene.

In another preferred embodiment, the same type of cell comprises the same type of cell with normal or low methylation level of DNA CpG site of NNMT gene.

In another preferred embodiment, the normal cell comprises normal tissue cell (e.g., tumor origin cell, tumor-adjacent cell or para-tumor tissue cell).

In another preferred embodiment, the normal cell comprises normal tissue cell (e.g., tumor origin cell, tumor-adjacent cell or para-tumor tissue cell) with normal methylation level of DNA CpG site of NNMT gene.

In another preferred embodiment, the cell with normal or low methylation level of DNA CpG site of NNMT gene comprises the cell that is not sensitive to the compound of formula I, or an optical isomer thereof, or a racemate thereof, or a solvate thereof, or a pharmaceutically acceptable salt thereof, or a deuterated compound thereof

In another preferred embodiment, the high methylation level of DNA CpG site of NNMT gene means the methylation level (M%) of DNA CpG site of NNMT gene in a cell ( e.g., tumor cell) is ≥ 3% and ≤ M2%, wherein M2 is any positive integer from 3 to 100.

In another preferred embodiment, M2 is 5, 10, 15, 20, 25, 30, 35, 40, 45, 50, 55, 60, 65, 70, 80, 85, 90, 95 or 100.

In another preferred embodiment, the methylation level of CpG site refers to the ratio of the number of methylated CpG nucleotides to the number of all nucleotides in a gene.

In another preferred embodiment, the methylation level of DNA CpG site of NNMT gene refers to the ratio of the number of methylated CpG nucleotides to the number of all nucleotides in the NNMT gene.

In another preferred embodiment, the methylation level of CpG site refers to the ratio of the number of methylated CpG nucleotides to the number of all CpG nucleotides in a gene.

In another preferred embodiment, the methylation level of DNA CpG site of NNMT gene refers to the ratio of the number of methylated DNA CpG nucleotides to the number of all CpG nucleotides in the NNMT gene.

In another preferred embodiment, the methylation level of DNA CpG site refers to the ratio of the number of methylated CpG sites to the number of all CpG sites in a DNA.

In another preferred embodiment, the methylation level of DNA CpG site refers to the ratio of the number of methylated CpG nucleotides to the number of all nucleotides in a DNA.

In another preferred embodiment, the methylation level of DNA CpG site refers to the ratio of the number of methylated CpG nucleotides to the number of all CpG nucleotides in a DNA.

In another preferred embodiment, the methylation level of DNA CpG site of NNMT gene refers to the ratio of the number of methylated DNA CpG sites to the number of all DNA CpG sites in the NNMT gene.

In another preferred embodiment, the methylation level of DNA CpG site of NNMT gene refers to the ratio of the number of methylated DNA CpG nucleotides to the number of all DNA CpG nucleotides in the NNMT gene.

In another preferred embodiment, the methylation level of DNA CpG site of NNMT gene comprises the methylation level of DNA CpG site in promoter region of NNMT gene.

In another preferred embodiment, the nucleotide sequence of the promoter region of NNMT gene is as shown in SEQ ID NO: 1.

In another preferred embodiment, the methylation level of DNA CpG site of NNMT gene comprises the methylation level of DNA CpG sites from 1050 bp before the transcription start site to 499 bp after the transcription start site in NNMT gene.

In another preferred embodiment, the sites from 1050 bp before the transcription start site to 499 bp after the transcription start site in NNMT gene is sites 951-2500 of nucleotide sequence as shown in SEQ ID NO: 1.

In another preferred embodiment, the methylation level of DNA CpG site of NNMT gene comprises the methylation level of the DNA CpG sites from 1050 bp to 193 bp before the transcription start site in NNMT gene.

In another preferred embodiment, the sites from 1050 bp to 193 bp before the transcription start site in NNMT gene is sites 951-1808 of nucleotide sequence as shown in SEQ ID NO: 1.

In another preferred embodiment, the methylation level of DNA CpG site of NNMT gene comprises the methylation level of DNA CpG sites from 840 bp to 469 bp before the transcription start site in NNMT gene.

In another preferred embodiment, the sites from 840 bp to 469 bp before the transcription start site in NNMT gene is sites 1161-1532 of nucleotide sequence as shown in SEQ ID NO: 1.

In another preferred embodiment, the methylation level of DNA CpG site of NNMT gene comprises the methylation level of DNA CpG site between any two sites (including the two sites itself) selected from group consisting of site 114165695, site 114165730, site 114165769, site 114165804, site 114165938, site 114166050 and site 114166066 on human chromosome 11.

In another preferred embodiment, the methylation level of DNA CpG site of NNMT gene comprises the methylation level of site of one or more (e.g., 2, 3, 4, 5, 6, or 7) of site 114165695, site 114165730, site 114165769, site 114165804, site 114165938, site 114166050 and site 114166066 on human chromosome 11.

In another preferred embodiment, the methylation level of DNA CpG site of NNMT gene comprises the methylation level of sites selected from group consisting of site 114165695 on human chromosome 11, site 114165730 on human chromosome 11, site 114165769 on human chromosome 11, site 114165804 on human chromosome 11, site 114165938 on human chromosome 11, site 114166050 on human chromosome 11, site 114166066 on human chromosome 11, and combinations thereof.

In another preferred embodiment, the methylation level of DNA CpG site of NNMT gene comprises the methylation level of DNA CpG site between any two sites (including the two sites itself) selected from group consisting of site 1161, site 1196, site 1235, site 1270, site 1404, site 1516 and site 1532 in nucleotide sequence of SEQ ID NO: 1.

In another preferred embodiment, the methylation level of DNA CpG site of NNMT gene comprises the methylation level of the site of one or more (e.g., 2, 3, 4, 5, 6, or 7) of site 1161, site 1196, site 1235, site 1270, site 1404, site 1516 and site 1532 in nucleotide sequence of SEQ ID NO: 1.

In another preferred embodiment, the methylation level of DNA CpG site of NNMT gene comprises the methylation level of sites selected from group consisting of site 1161 in SEQ ID NO: 1, site 1196 in SEQ ID NO: 1, site 1235 in SEQ ID NO: 1, site 1270 in SEQ ID NO: 1, site 1404 in SEQ ID NO: 1, site 1516 in SEQ ID NO: 1, site 1532 in SEQ ID NO: 1, and combinations thereof.

In another preferred embodiment, the NNMT gene inhibitor is administered to achieve low or no expression of the NNMT gene in tumor.

In another preferred embodiment, the DNA methylase promoter is administered to achieve high expression of DNA methylase in tumor.

In another preferred embodiment, the DNMT1 promoter is administered to achieve high expression of DNMT1 in tumor.

In another preferred embodiment, the DNMT3a promoter is administered to achieve high expression of DNMT3a in tumor.

In another preferred embodiment, the DNMT3b promoter is administered to achieve high expression of DNMT3b in tumor.

In another preferred embodiment, the UHRFl promoter is administered to achieve high expression of UHRFl in tumor.

In another preferred embodiment, the methylation promoter of nucleotide site of NNMT gene is administered to achieve high methylation level of nucleotide site of NNMT gene in tumor.

In another preferred embodiment, the methylation promoter of DNA CpG site of NNMT gene is administered to achieve high methylation level of DNA CpG site of NNMT gene in tumor.

In another preferred embodiment, the inhibitor comprises specific inhibitor.

In another preferred embodiment, the promoter comprises specific promoter.

In another preferred embodiment, the NNMT gene inhibitor comprises inhibitor that can achieve low or no expression of the NNMT gene in tumor.

In another preferred embodiment, the DNA methylase promoter comprises promoter that can achieve high expression of DNA methylase in tumor.

In another preferred embodiment, the DNMT1 promoter comprises promoter that can achieve high expression of DNMT1 in tumor.

In another preferred embodiment, the DNMT3a promoter comprises promoter that can achieve high expression of DNMT3a in tumor.

In another preferred embodiment, the DNMT3b promoter comprises promoter that can achieve high expression of DNMT3b in tumor.

In another preferred embodiment, the UHRFl promoter comprises promoter that can achieve high expression of UHRFl in tumor.

In another preferred embodiment, the methylation promoter of nucleotide site of NNMT gene comprises promoter that can achieve high methylation level of nucleotide site of NNMT gene in tumor.

In another preferred embodiment, the methylation promoter of DNA CpG site of NNMT gene comprises promoter that can achieve high methylation promoter of DNA CpG site of NNMT gene in tumor.

In another preferred embodiment, the tumor is selected from the group consisting of lung cancer, renal carcinoma, breast cancer, colon cancer, lymphoma, leukemia, pancreatic cancer, brain tumor, liver cancer, prostate cancer, and combinations thereof.

In another preferred embodiment, the lung cancer is selected from the group consisting of non-small cell lung cancer, small cell lung cancer, and combinations thereof.

In another preferred embodiment, the lung cancer cell comprises NCI-H82 cell.

In another preferred embodiment, the colon cancer comprises colon adenocarcinoma.

In another preferred embodiment, the colon cancer cell comprises SW48 cell.

In another preferred embodiment, the breast cancer cell comprises MDA-MB-453 cell.

In another preferred embodiment, the breast cancer comprises triple negative breast cancer.

In another preferred embodiment, the lymphoma is selected from the group consisting of B-cell lymphoma, skin T-cell lymphoma, and combinations thereof.

In another preferred embodiment, the lymphoma comprises diffuse large B-cell lymphoma.

In another preferred embodiment, the brain tumor is selected from the group consisting of brain glioblastoma, neuroglioma, brain medulloblastoma, brain neuroblastoma, and combination thereof.

In another preferred embodiment, the brain medulloblastoma comprises cerebellar medulloblastoma.

In another preferred embodiment, the brain glioblastoma comprises glioblastoma multiforme.

In another preferred embodiment, the brain tumor comprises glioblastoma.

In another preferred embodiment, the brain tumor comprises glioma.

In another preferred embodiment, the brain tumor comprises a malignant glioma in cranial fossa.

In another preferred embodiment, the brain tumor comprises medulloblastoma.

In another preferred embodiment, the brain tumor cell comprises Daoy cell.

In another preferred embodiment, the brain tumor cell comprises one or more of GB-1 cell, SF126 cell, D341 Med cell, Kelly cell and NB-1 cell.

In another preferred embodiment, the renal carcinoma is selected from the group consisting of clear cell renal cell adenocarcinoma, renal carcinoma Wilms, and combination thereof.

In another preferred embodiment, the renal carcinoma comprises clear cell renal cell adenocarcinoma.

In another preferred embodiment, the renal carcinoma comprises renal carcinoma Wilms.

In another preferred embodiment, the renal carcinoma cell comprises renal carcinoma Wilms cell.

In another preferred embodiment, the renal carcinoma cell comprises one or more of G-401 cell and 786-O cell.

In another preferred embodiment, the pancreatic cancer comprises pancreatic ductal carcinoma.

In another preferred embodiment, the pancreatic cancer cell comprises CFPAC-1 cell.

In another preferred embodiment, the leukemia is selected from the group consisting of T-lymphocyte leukemia, myeloid leukemia, and combinations thereof.

In another preferred embodiment, the T-lymphocytic leukemia comprises acute T-lymphocytic leukemia.

In another preferred embodiment, the myeloid leukemia comprises type M4 of acute myeloid leukemia.

In another preferred embodiment, the myeloid leukemia comprises FAB type M4 of acute myeloid leukemia.

In another preferred embodiment, the expression comprises protein expression and/or mRNA expression.

In another preferred embodiment, the composition or preparation is a pharmaceutical composition or pharmaceutical preparation.

In another preferred embodiment, the composition or preparation further comprises a pharmaceutically acceptable carrier.

In another preferred embodiment, the expression is mRNA expression or protein expression.

In another preferred embodiment, the level is mRNA level or protein level.

In another preferred embodiment, the dosage form of the composition or preparation is a solid preparation, liquid preparation or semi-solid preparation.

In another preferred embodiment, the dosage form of the composition or preparation is oral preparation, external preparation or injection preparation.

In another preferred embodiment, the dosage form of the composition or preparation is tablet, injection, infusion, paste, gel, solution, microsphere or film.

In the second aspect of the present invention, it provides a marker for determining whether the compound of formula I, or an optical isomer thereof, or a racemate thereof, or a solvate thereof, or a pharmaceutically acceptable salt thereof, or a deuterated compound thereof according to the first aspect of the present invention is suitable for use in the prevention and/or treatment of patient tumor, the marker comprises mitochondria permeability transition pore, peptidyl-prolyl cis-trans isomerase F, NNMT gene, DNA methylase, UHRF1, the methylation of nucleotide site of NNMT gene, and/or the methylation of DNA CpG site of NNMT gene.

In another preferred embodiment, the marker comprises the expression level or activity of mitochondria permeability transition pore, the expression level or activity of peptidyl-prolyl cis-trans isomerase F, the expression level of NNMT gene, the expression level of DNA methylase, the expression level of UHRF1, the methylation level of nucleotide site of NNMT gene, and/or the methylation level of DNA CpG site of NNMT gene.

In another preferred embodiment, the compound of formula I, or an optical isomer thereof, or a racemate thereof, or a solvate thereof, or a pharmaceutically acceptable salt thereof, or a deuterated compound thereof according to the first aspect of the present invention is suitable for use in the prevention and/or treatment of patient tumor with low expression, no expression, low activity or no activity of mitochondria permeability transition pore, low expression, no expression, low activity or no activity of peptidyl-prolyl cis-trans isomerase F, low or no expression of NNMT gene, high expression of DNA methylase, high expression of UHRFl, high methylation level of nucleotide site of NNMT gene, and/or high methylation level of DNA CpG site of NNMT gene.

In another preferred embodiment, the compound of formula I, or an optical isomer thereof, or a racemate thereof, or a solvate thereof, or a pharmaceutically acceptable salt thereof, or a deuterated compound thereof according to the first aspect of the present invention is not suitable for use in the prevention and/or treatment of patient tumor with high expression or high activity of mitochondria permeability transition pore, high expression or high activity of peptidyl-prolyl cis-trans isomerase F, high expression of NNMT gene, low expression of DNA methylase, low expression of UHRFl, low methylation level of nucleotide site of NNMT gene, and/or low methylation level of DNA CpG site of NNMT gene.

In another preferred embodiment, "the compound of formula I, or an optical isomer thereof, or a racemate thereof, or a solvate thereof, or a pharmaceutically acceptable salt thereof, or a deuterated compound thereof according to the first aspect of the present invention is suitable for use in the prevention and/or treatment of patient tumor" comprises "the patient tumor is sensitive to the compound of formula I, or an optical isomer thereof, or a racemate thereof, or a solvate thereof, or a pharmaceutically acceptable salt thereof, or a deuterated compound thereof according to the first aspect of the present invention".

In another preferred embodiment, "the compound of formula I, or an optical isomer thereof, or a racemate thereof, or a solvate thereof, or a pharmaceutically acceptable salt thereof, or a deuterated compound thereof according to the first aspect of the present invention is not suitable for use in the prevention and/or treatment of patient tumor" comprises "the patient tumor is not sensitive to the compound of formula I, or an optical isomer thereof, or a racemate thereof, or a solvate thereof, or a pharmaceutically acceptable salt thereof, or a deuterated compound thereof according to the first aspect of the present invention".

In another preferred embodiment, the tumor with low expression, no expression, low activity or no activity of mitochondria permeability transition pore is as described in the first aspect of the invention.

In another preferred embodiment, the tumor with low expression, no expression, low activity or no activity of peptidyl-prolyl cis-trans isomerase F is as described in the first aspect of the invention.

In another preferred embodiment, the tumor with low or no expression of NNMT gene is as described in the first aspect of the invention.

In another preferred embodiment, the DNA methylase is selected from the group consisting of DNMT1, DNMT3a, DNMT3b, and combinations thereof.

In another preferred embodiment, the tumor with high expression of DNA methylase (e.g., DNMT1) is as described in the first aspect of the invention.

In another preferred embodiment, the tumor with high expression of UHRFl is as described in the first aspect of the invention.

In another preferred embodiment, the tumor with high methylation level of nucleotide site of NNMT gene is as described in the first aspect of the invention.

In another preferred embodiment, the tumor with high methylation level of DNA CpG site of NNMT gene is as described in the third aspect of the invention.

In another preferred embodiment, the high expression or high activity of mitochondria permeability transition pore means the ratio (H1/H0) of the expression level or activity level Hlof mitochondria permeability transition pore in a cell ( e.g., tumor cell ) to the expression level or activity level H0 of mitochondria permeability transition pore in the same cell or a normal cell (e.g., para-tumor tissue cell) is >1.0, preferably ≥1.2, more preferably ≥1.5, more preferably ≥2, more preferably ≥3, more preferably ≥5, more preferably ≥8, more preferably ≥ 10, more preferably ≥ 15, more preferably ≥20, more preferably ≥30, more preferably ≥50, such as 2-50.

In another preferred embodiment, the high expression or high activity of peptidyl-prolyl cis-trans isomerase F means the ratio (P1/P0) of the expression level or activity level Plof peptidyl-prolyl cis-trans isomerase F in a cell ( e.g., tumor cell) to the expression level or activity level P0 of peptidyl-prolyl cis-trans isomerase F in the same cell or a normal cell (e.g., para-tumor tissue cell) is > 1.0, preferably ≥ 1.2, more preferably ≥ 1.5, more preferably ≥2, more preferably ≥3, more preferably ≥5, more preferably ≥8, more preferably ≥10, more preferably ≥15, more preferably ≥20, more preferably ≥30, more preferably ≥50, such as 2-50.

In another preferred embodiment, the high expression of NNMT gene means the ratio (E1/E0) of the expression E1 of NNMT gene in a cell ( e.g., tumor cell) to the expression E0 of NNMT gene in the same type of cell or a normal cell is >1.0, preferably ≥1.2, more preferably ≥1.5, more preferably ≥2, more preferably ≥3, more preferably ≥5, more preferably ≥8, more preferably ≥10, more preferably ≥15, more preferably ≥20, more preferably ≥30, more preferably ≥50, such as 2-50.

In another preferred embodiment, the tumor with low expression of DNA methylase means the ratio (A1/A0) of the expression level A1 of DNA methylase in the tumor cell to the expression level A0 of DNA methylase in the same type of cell or a normal cell is <1.0, preferably ≤ 0.7, more preferably ≤ 0.6, more preferably ≤ 0.5, more preferably ≤ 0.4, more preferably ≤ 0.3, more preferably ≤ 0.2, more preferably ≤ 0.1, more preferably ≤ 0.05, more preferably ≤ 0.01, more preferably ≤ 0.005, more preferably ≤ 0.001, more preferably ≤ 0.0001, more preferably ≤ 0.00001, more preferably ≤ 0.000001, more preferably ≤ 0.0000001.

In another preferred embodiment, the tumor with low expression of DNMT1 means the ratio (B1/B0) of the expression level B1 of DNMT1 in the tumor cell to the expression level B0 of DNMT1 in the same type of cell or a normal cell is <1.0, preferably ≤ 0.7, more preferably ≤ 0.6, more preferably ≤ 0.5, more preferably ≤ 0.4, more preferably ≤ 0.3, more preferably ≤ 0.2, more preferably ≤ 0.1, more preferably ≤ 0.05, more preferably ≤ 0.01, more preferably ≤ 0.005, more preferably ≤ 0.001, more preferably ≤ 0.0001, more preferably ≤ 0.00001, more preferably ≤ 0.000001, more preferably ≤ 0.0000001.

In another preferred embodiment, the tumor with low expression of DNMT3a means the ratio (C1/C0) of the expression level C1 of DNMT3a in the tumor cell to the expression level C0 of DNA methylase in the same type of cell or a normal cell is <1.0, preferably ≤ 0.7, more preferably ≤ 0.6, more preferably ≤ 0.5, more preferably ≤ 0.4, more preferably ≤ 0.3, more preferably ≤ 0.2, more preferably ≤ 0.1, more preferably ≤ 0.05, more preferably ≤ 0.01, more preferably ≤ 0.005, more preferably ≤ 0.001, more preferably ≤ 0.0001, more preferably ≤ 0.00001, more preferably ≤ 0.000001, more preferably ≤ 0.0000001.

In another preferred embodiment, the tumor with low expression of DNMT3b means the ratio (D1/D0) of the expression level D1 of DNMT3b in the tumor cell to the expression level D0 of DNA methylase in the same type of cell or a normal cell is <1.0, preferably ≤ 0.7, more preferably ≤ 0.6, more preferably ≤ 0.5, more preferably ≤ 0.4, more preferably ≤ 0.3, more preferably ≤ 0.2, more preferably ≤ 0.1, more preferably ≤ 0.05, more preferably ≤ 0.01, more preferably ≤ 0.005, more preferably ≤ 0.001, more preferably ≤ 0.0001, more preferably ≤ 0.00001, more preferably ≤ 0.000001, more preferably ≤ 0.0000001.

In another preferred embodiment, the tumor with low expression of UHRF1 means the ratio (F1/F0) of the expression level F1 of UHRF1 in the tumor cell to the expression level F0 of UHRF1 in the same type of cell or a normal cell is <1.0, preferably ≤ 0.7, more preferably ≤ 0.6, more preferably ≤ 0.5, more preferably ≤ 0.4, more preferably ≤ 0.3, more preferably ≤ 0.2, more preferably ≤ 0.1, more preferably ≤ 0.05, more preferably ≤ 0.01, more preferably ≤ 0.005, more preferably ≤ 0.001, more preferably ≤ 0.0001, more preferably ≤ 0.00001, more preferably ≤ 0.000001, more preferably ≤ 0.0000001.

In another preferred embodiment, the low methylation level of nucleotide site of NNMT gene means the ratio (L1/L0) of the methylation level L1 of nucleotide site of NNMT gene in a cell ( e.g., tumor cell ) to the methylation level L0 of nucleotide site of NNMT gene in the same type of cell or a normal cell (e.g., para-tumor tissue cell) is <1.0, preferably ≤ 0.7, more preferably ≤ 0.6, more preferably ≤ 0.5, more preferably ≤ 0.4, more preferably ≤ 0.3, more preferably ≤ 0.2, more preferably ≤ 0.1, more preferably ≤ 0.05, more preferably ≤ 0.01, more preferably ≤ 0.005, more preferably ≤ 0.001, more preferably ≤ 0.0001, more preferably ≤ 0.00001, more preferably ≤ 0.000001, more preferably ≤ 0.0000001.

In another preferred embodiment, the low methylation level of DNA CpG site of NNMT gene means the ratio (W1/W0) of the methylation level W1 of DNA CpG site of NNMT gene in a cell ( e.g., tumor cell) to the methylation level W0 of DNA CpG site of NNMT gene in the same type of cell or a normal cell (e.g., para-tumor tissue cell) is <1.0, preferably ≤ 0.7, more preferably ≤ 0.6, more preferably ≤ 0.5, more preferably ≤ 0.4, more preferably ≤ 0.3, more preferably ≤ 0.2, more preferably ≤ 0.1, more preferably ≤ 0.05, more preferably ≤ 0.01, more preferably ≤ 0.005, more preferably ≤ 0.001, more preferably ≤ 0.0001, more preferably ≤ 0.00001, more preferably ≤ 0.000001, more preferably ≤ 0.0000001.

In the third aspect of the present invention, it provides a detection kit, which comprises:
(i) a detection reagent for detecting the expression level or activity of mitochondria permeability transition pore, the expression level or activity of peptidyl-prolyl cis-trans isomerase F, the expression level of NNMT gene, the expression level of DNA methylase, the expression level of UHRF1, the methylation level of nucleotide site of NNMT gene, and/or the methylation level of DNA CpG site of NNMT gene.

In another preferred embodiment, the test sample of the detection kit comprises tumor cell.

In another preferred embodiment, the level comprises protein level and/or mRNA level.

In another preferred embodiment, the expression comprises mRNA expression and/or protein expression.

In the fourth aspect of the present invention, it provides a use of the detection kit according to the third aspect of the present invention in the preparation of concomitant diagnose kit for determining whether the compound of formula I, or an optical isomer thereof, or a racemate thereof, or a solvate thereof, or a pharmaceutically acceptable salt thereof, or a deuterated compound thereof according to the first aspect of the present invention is suitable for use in the prevention and/or treatment of patient tumor.

In another preferred embodiment, the concomitant diagnose kit further comprises instruction or label.

In another preferred embodiment, the instruction or label records that the compound of formula I, or an optical isomer thereof, or a racemate thereof, or a solvate thereof, or a pharmaceutically acceptable salt thereof, or a deuterated compound thereof according to the first aspect of the present invention is suitable for use in the prevention and/or treatment of patient tumor with low expression, no expression, low activity or no activity of mitochondria permeability transition pore, low expression, no expression, low activity or no activity of peptidyl-prolyl cis-trans isomerase F, low or no expression of NNMT gene, high expression of DNA methylase, high expression of UHRFl, high methylation level of nucleotide site of NNMT gene, and/or high methylation level of DNA CpG site of NNMT gene.

In another preferred embodiment, the instruction or label records that the compound of formula I, or an optical isomer thereof, or a racemate thereof, or a solvate thereof, or a pharmaceutically acceptable salt thereof, or a deuterated compound thereof according to the first aspect of the present invention is not suitable for use in the prevention and/or treatment of patient tumor with high expression or high activity of mitochondria permeability transition pore, high expression or high activity of peptidyl-prolyl cis-trans isomerase F, high expression of NNMT gene, low expression of DNA methylase, low expression of UHRFl, low methylation level of nucleotide site of NNMT gene, and/or low methylation level of DNA CpG site of NNMT gene.

In another preferred embodiment, "the compound of formula I, or an optical isomer thereof, or a racemate thereof, or a solvate thereof, or a pharmaceutically acceptable salt thereof, or a deuterated compound thereof according to the first aspect of the present invention is suitable for use in the prevention and/or treatment of patient tumor" is as described in the second aspect of the invention.

In another preferred embodiment, "the compound of formula I, or an optical isomer thereof, or a racemate thereof, or a solvate thereof, or a pharmaceutically acceptable salt thereof, or a deuterated compound thereof according to the first aspect of the present invention is not suitable for use in the prevention and/or treatment of patient tumor" is as described in the second aspect of the invention.

In the fifth aspect of the present invention, it provides a medicine kit, which comprises:
(i) a detection reagent for detecting the expression level or activity of mitochondria permeability transition pore, the expression level or activity of peptidyl-prolyl cis-trans isomerase F, the expression level of NNMT gene, the expression level of DNA methylase, the expression level of UHRF1, the methylation level of nucleotide site of NNMT gene, and/or the methylation level of DNA CpG site of NNMT gene; and
(ii) the compound of formula I, or an optical isomer thereof, or a racemate thereof, or a solvate thereof, or a pharmaceutically acceptable salt thereof, or a deuterated compound thereof according to the first aspect of the present invention.

In another preferred embodiment, the expression level or activity of mitochondria permeability transition pore, the expression level or activity of peptidyl-prolyl cis-trans isomerase F, the expression level of NNMT gene, the expression level of DNA methylase, the expression level of UHRF1, the methylation level of nucleotide site of NNMT gene, and/or the methylation level of DNA CpG site of NNMT gene comprises the expression level or activity of mitochondria permeability transition pore, the expression level or activity of peptidyl-prolyl cis-trans isomerase F, the expression level of NNMT gene, the expression level of DNA methylase, the expression level of UHRF1, the methylation level of nucleotide site of NNMT gene, and/or the methylation level of DNA CpG site of NNMT gene in the tumor.

In another preferred embodiment, the detection sample comprises tumor.

In another preferred embodiment, the medicine kit further comprises instruction or label.

In another preferred embodiment, the instruction or label records that the compound of formula I, or an optical isomer thereof, or a racemate thereof, or a solvate thereof, or a pharmaceutically acceptable salt thereof, or a deuterated compound thereof according to the first aspect of the present invention is suitable for use in the prevention and/or treatment of patient tumor with low expression, no expression, low activity or no activity of mitochondria permeability transition pore, low expression, no expression, low activity or no activity of peptidyl-prolyl cis-trans isomerase F, low or no expression of NNMT gene, high expression of DNA methylase, high expression of UHRFl, high methylation level of nucleotide site of NNMT gene, and/or high methylation level of DNA CpG site of NNMT gene.

In another preferred embodiment, the instruction or label records that the compound of formula I, or an optical isomer thereof, or a racemate thereof, or a solvate thereof, or a pharmaceutically acceptable salt thereof, or a deuterated compound thereof according to the first aspect of the present invention is not suitable for use in the prevention and/or treatment of patient tumor with high expression or high activity of mitochondria permeability transition pore, high expression or high activity of peptidyl-prolyl cis-trans isomerase F, high expression of NNMT gene, low expression of DNA methylase, low expression of UHRFl, low methylation level of nucleotide site of NNMT gene, and/or low methylation level of DNA CpG site of NNMT gene.

In the sixth aspect of the present invention, it provides a method for preventing and/or treating tumor, which comprises administering the compound of formula I, or an optical isomer thereof, or a racemate thereof, or a solvate thereof, or a pharmaceutically acceptable salt thereof, or a deuterated compound thereof according to the first aspect of the present invention to a subject in need, thereby preventing and/or treating tumor.

In another preferred embodiment, the tumor is as described in the first aspect of the invention.

In another preferred embodiment, the subject is human and non-human mammals (rodent, rabbit, monkey, livestock, dog, cat, etc.).

In another preferred embodiment, the method comprises:
firstly achieving low expression, no expression, low activity or no activity of mitochondria permeability transition pore, low expression, no expression, low activity or no activity of peptidyl-prolyl cis-trans isomerase F, low or no expression of NNMT gene, high expression of DNA methylase, high expression of UHRFl, high methylation level of nucleotide site of NNMT gene, and/or high methylation level of DNA CpG site of NNMT gene in the subject tumor, then administering the compound of formula I, or an optical isomer thereof, or a racemate thereof, or a solvate thereof, or a pharmaceutically acceptable salt thereof, or a deuterated compound thereof to prevent and/or treat tumor.

In another preferred embodiment, the method comprises:
firstly administering mitochondria permeability transition pore inhibitor, peptidyl-prolyl cis-trans isomerase F inhibitor, NNMT gene inhibitor, DNA methylase promoter, UHRF1 promoter, methylation promoter of nucleotide site of NNMT gene, and/or methylation promoter of DNA CpG site of NNMT gene to achieve low expression, no expression, low activity or no activity of mitochondria permeability transition pore, low expression, no expression, low activity or no activity of peptidyl-prolyl cis-trans isomerase F, low or no expression of NNMT gene, high expression of DNA methylase, high expression of UHRFl, high methylation level of nucleotide site of NNMT gene, and/or high methylation level of DNA CpG site of NNMT gene in the subject tumor, then administering the compound of formula I, or an optical isomer thereof, or a racemate thereof, or a solvate thereof, or a pharmaceutically acceptable salt thereof, or a deuterated compound thereof to prevent and/or treat tumor.

In another preferred embodiment, the mitochondria permeability transition pore inhibitor, peptidyl-prolyl cis-trans isomerase F inhibitor, NNMT gene inhibitor, DNA methylase promoter, UHRF1 promoter, methylation promoter of nucleotide site of NNMT gene, and/or methylation promoter of DNA CpG site of NNMT gene is as described in the first aspect of the invention.

In the seventh aspect of the present invention, it provides a device or system, the device or system comprises:
(i) a detection module, the detection module is used to detect the expression level or activity of mitochondria permeability transition pore, the expression level or activity of peptidyl-prolyl cis-trans isomerase F, the expression level of NNMT gene, the expression level of DNA methylase, the expression level of UHRF1, the methylation level of nucleotide site of NNMT gene, and/or the methylation level of DNA CpG site of NNMT gene;
(ii) an output module, the output module comprises the information as follows:
   the compound of formula I, or an optical isomer thereof, or a racemate thereof, or a solvate thereof, or a pharmaceutically acceptable salt thereof, or a deuterated compound thereof according to the first aspect of the present invention is suitable for use in the prevention and/or treatment of patient tumor with low expression, no expression, low activity or no activity of mitochondria permeability transition pore, low expression, no expression, low activity or no activity of peptidyl-prolyl cis-trans isomerase F, low or no expression of NNMT gene, high expression of DNA methylase, high expression of UHRFl, high methylation level of nucleotide site of NNMT gene, and/or high methylation level of DNA CpG site of NNMT gene; and/or
   the compound of formula I, or an optical isomer thereof, or a racemate thereof, or a solvate thereof, or a pharmaceutically acceptable salt thereof, or a deuterated compound thereof according to the first aspect of the present invention is not suitable for use in the prevention and/or treatment of patient tumor with high expression or high activity of mitochondria permeability transition pore, high expression or high activity of peptidyl-prolyl cis-trans isomerase F, high expression of NNMT gene, low expression of DNA methylase, low expression of UHRFl, low methylation level of nucleotide site of NNMT gene, and/or low methylation level of DNA CpG site of NNMT gene.

In another preferred embodiment, the device comprises a gene detector or protein detector.

In another preferred embodiment, the device or system further comprises sample injection module.

In another preferred embodiment, the injection module is used to inject tumor cell extract.

In another preferred embodiment, the device or system further comprises data processing module.

In another preferred embodiment, the expression level or activity of mitochondria permeability transition pore, the expression level or activity of peptidyl-prolyl cis-trans isomerase F, the expression level of NNMT gene, the expression level of DNA methylase, the expression level of UHRF1, the methylation level of nucleotide site of NNMT gene, and/or the methylation level of DNA CpG site of NNMT gene can be obtained by the procession of the data processing module.

In the eighth aspect of the present invention, it provides a use of mitochondria permeability transition pore inhibitor, peptidyl-prolyl cis-trans isomerase F inhibitor, NNMT gene inhibitor, DNA methylase promoter, UHRF1 promoter, methylation promoter of nucleotide site of NNMT gene, and/or methylation promoter of DNA CpG site of NNMT gene in the preparation of a composition or a preparation for enhancing the anti-tumor effect of anti-tumor drug.

In another preferred embodiment, the mitochondria permeability transition pore inhibitor comprises inhibitor that can achieve low expression, no expression, low activity or no activity of mitochondria permeability transition pore in tumor.

In another preferred embodiment, the peptidyl-prolyl cis-trans isomerase F inhibitor comprises inhibitor that can achieve low expression, no expression, low activity or no activity of peptidyl-prolyl cis-trans isomerase F.

In another preferred embodiment, the NNMT gene inhibitor comprises inhibitor that can achieve low or no expression of the NNMT gene in tumor.

In another preferred embodiment, the DNA methylase promoter comprises promoter that can achieve high expression of DNA methylase in tumor.

In another preferred embodiment, the DNA methylase is selected from the group consisting of DNMT1, DNMT3a, DNMT3b, and combinations thereof.

In another preferred embodiment, the DNA methylase promoter comprises DNMT1 promoter.

In another preferred embodiment, the DNMT1 promoter comprises promoter that can achieve high expression of DNMT1 in tumor.

In another preferred embodiment, the DNA methylase promoter comprises DNMT3a promoter.

In another preferred embodiment, the DNMT3a promoter comprises promoter that can achieve high expression of DNMT3a in tumor.

In another preferred embodiment, the DNA methylase promoter comprises DNMT3b promoter.

In another preferred embodiment, the DNMT3b promoter comprises promoter that can achieve high expression of DNMT3b in tumor.

In another preferred embodiment, the UHRFl promoter comprises promoter that can achieve high expression of UHRFl in tumor.

In another preferred embodiment, the methylation promoter of nucleotide site of NNMT gene comprises promoter that can achieve high methylation level of nucleotide site of NNMT gene in tumor.

In another preferred embodiment, the methylation promoter of DNA CpG site of NNMT gene comprises promoter that can achieve high methylation level of nucleotide site of NNMT gene in tumor.

In another preferred embodiment, the inhibitor comprises specific inhibitor.

In another preferred embodiment, the promoter comprises specific promoter.

In another preferred embodiment, the anti-tumor drug comprises the compound of formula I, or an optical isomer thereof, or a racemate thereof, or a solvate thereof, or a pharmaceutically acceptable salt thereof, or a deuterated compound thereof according to the first aspect of the present invention.

In another preferred embodiment, the tumor is as described in the first aspect of the invention.

In another preferred embodiment, the mitochondria permeability transition pore inhibitor is selected from the group consisting of Cyclosporin A, CyP-D protein inhibitor, peroxide scavenger, and combinations thereof.

In another preferred embodiment, the peptidyl-prolyl cis-trans isomerase F inhibitor comprises shRNA.

In another preferred embodiment, the nucleotide sequence of shRNA is GTTCTTCATCTGCACCATAAA.

In another preferred embodiment, the composition or preparation is a pharmaceutical composition or pharmaceutical preparation.

In another preferred embodiment, the composition or preparation further comprises a pharmaceutically acceptable carrier.

In another preferred embodiment, the dosage form of the composition or preparation is a solid preparation, liquid preparation or semi-solid preparation.

In another preferred embodiment, the dosage form of the composition or preparation is oral preparation, external preparation or injection preparation.

In another preferred embodiment, the injection preparation is intravenous injection preparation.

In another preferred embodiment, the dosage form of the composition or preparation is tablet, injection, infusion, paste, gel, solution, microsphere or film.

In the ninth aspect of the present invention, it provides an active ingredient combination, the active ingredient combination comprises:
(1) a first active ingredient, the first active ingredient comprises anti-tumor drug; and
(2) a second active ingredient, the second active ingredient comprises mitochondria permeability transition pore inhibitor, peptidyl-prolyl cis-trans isomerase F inhibitor, NNMT gene inhibitor, DNA methylase promoter, UHRF1 promoter, methylation promoter of nucleotide site of NNMT gene, and/or methylation promoter of DNA CpG site of NNMT gene.

In another preferred embodiment, the anti-tumor drug comprises the compound of formula I, or an optical isomer thereof, or a racemate thereof, or a solvate thereof, or a pharmaceutically acceptable salt thereof, or a deuterated compound thereof according to the first aspect of the present invention.

In another preferred embodiment, the mitochondria permeability transition pore inhibitor, peptidyl-prolyl cis-trans isomerase F inhibitor, NNMT gene inhibitor, DNA methylase promoter, UHRF1 promoter, methylation promoter of nucleotide site of NNMT gene, and/or methylation promoter of DNA CpG site of NNMT gene is as described in the eighth aspect of the invention.

In another preferred embodiment, the molar ratio of the first active ingredient to the second active ingredient is 0.01-600:1, preferably 0.05-500:1, more preferably 0.1-400:1, more preferably 0.2-200:1, more preferably 0.5-100:1, more preferably 0.5-80:1, most preferably 1-50:1.

In another preferred embodiment, at least one active ingredient is independent in the active ingredient combination.

In another preferred embodiment, the first active ingredient and the second active ingredient are independent of each other in the active ingredient combination.

In the tenth aspect of the present invention, it provides a composition, the composition comprises:
(1) a first active ingredient, the first active ingredient comprises anti-tumor drug; and
(2) a second active ingredient, the second active ingredient comprises mitochondria permeability transition pore inhibitor, peptidyl-prolyl cis-trans isomerase F inhibitor, NNMT gene inhibitor, DNA methylase promoter, UHRF1 promoter, methylation promoter of nucleotide site of NNMT gene, and/or methylation promoter of DNA CpG site of NNMT gene.

In another preferred embodiment, the anti-tumor drug comprises the compound of formula I, or an optical isomer thereof, or a racemate thereof, or a solvate thereof, or a pharmaceutically acceptable salt thereof, or a deuterated compound thereof according to the first aspect of the present invention.

In another preferred embodiment, the mitochondria permeability transition pore inhibitor, peptidyl-prolyl cis-trans isomerase F inhibitor, NNMT gene inhibitor, DNA methylase promoter, UHRF1 promoter, methylation promoter of nucleotide site of NNMT gene, and/or methylation promoter of DNA CpG site of NNMT gene is as described in the eighth aspect of the invention.

In another preferred embodiment, the composition is a pharmaceutical composition.

In another preferred embodiment, the pharmaceutical composition further comprises a pharmaceutically acceptable carrier.

In another preferred embodiment, the dosage form of the composition or preparation is a solid preparation, liquid preparation or semi-solid preparation.

In another preferred embodiment, the dosage form of the composition or preparation is oral preparation, external preparation or injection preparation.

In another preferred embodiment, the injection preparation is intravenous injection preparation.

In another preferred embodiment, the dosage form of the composition or preparation is tablet, injection, infusion, paste, gel, solution, microsphere or film.

In another preferred embodiment, the content of the first active ingredient is 0.01-99.99 wt%, preferably 0.1-99.9 wt%, more preferably 1-99 wt%, more preferably 10-99 wt%, most preferably 20-99 wt%, based on the total weight of the active ingredient in the composition.

In another preferred embodiment, the content of the second active ingredient is 0.01-99.99 wt%, preferably 0.1-99.9 wt%, more preferably 1-99 wt%, more preferably 10-99 wt%, most preferably 20-99 wt%, based on the total weight of the active ingredient in the composition.

In the eleventh aspect of the present invention, it provides a medicine kit, the medicine kit comprises:
(A) a first preparation comprising a first active ingredient, the first active ingredient comprises anti-tumor drug; and
(B) a second preparation comprising a second active ingredient, the second active ingredient comprises mitochondria permeability transition pore inhibitor, peptidyl-prolyl cis-trans isomerase F inhibitor, NNMT gene inhibitor, DNA methylase promoter, UHRF1 promoter, methylation promoter of nucleotide site of NNMT gene, and/or methylation promoter of DNA CpG site of NNMT gene.

In another preferred embodiment, the anti-tumor drug comprises the compound of formula I, or an optical isomer thereof, or a racemate thereof, or a solvate thereof, or a pharmaceutically acceptable salt thereof, or a deuterated compound thereof according to the first aspect of the present invention.

In another preferred embodiment, the mitochondria permeability transition pore inhibitor, peptidyl-prolyl cis-trans isomerase F inhibitor, NNMT gene inhibitor, DNA methylase promoter, UHRF1 promoter, methylation promoter of nucleotide site of NNMT gene, and/or methylation promoter of DNA CpG site of NNMT gene is as described in the eighth aspect of the invention.

In another preferred embodiment, the medicine kit further comprises a user manual.

In another preferred embodiment, the first preparation and the second preparation are independent preparation of each other.

In another preferred embodiment, the first preparation and the second preparation are combined preparation.

In another preferred embodiment, the user manual records that the first preparation and the second preparation are used together to enhance anti-tumor activity of anti-tumor drug.

In another preferred embodiment, the method for using together means the second preparation comprising a second active ingredient is administered firstly, then the first preparation comprising a first active ingredient is administered.

In the twelfth aspect of the present invention, it provides a method for inhibiting tumor cell, which comprises contacting the tumor cell with the compound of formula I, or an optical isomer thereof, or a racemate thereof, or a solvate thereof, or a pharmaceutically acceptable salt thereof, or a deuterated compound thereof according to the first aspect of the present invention, thereby inhibiting tumor cell.

In another preferred embodiment, the method is vitro method.

In another preferred embodiment, the method is non-therapeutic and non-diagnostic method.

In another preferred embodiment, the contact is performed in vitro culture.

In another preferred embodiment, the tumor is as described in the first aspect of the invention.

In another preferred embodiment, the method comprises:
firstly achieving low expression, no expression, low activity or no activity of mitochondria permeability transition pore, low expression, no expression, low activity or no activity of peptidyl-prolyl cis-trans isomerase F, low or no expression of NNMT gene, high expression of DNA methylase, high expression of UHRFl, high methylation level of nucleotide site of NNMT gene, and/or high methylation level of DNA CpG site of NNMT gene in the tumor cell, then contacting the tumor cell with the compound of formula I, or an optical isomer thereof, or a racemate thereof, or a solvate thereof, or a pharmaceutically acceptable salt thereof, or a deuterated compound thereof according to the first aspect of the present invention, thereby inhibiting tumor cell.

In another preferred embodiment, the method comprises:
firstly administering mitochondria permeability transition pore inhibitor, peptidyl-prolyl cis-trans isomerase F inhibitor, NNMT gene inhibitor, DNA methylase promoter, UHRF1 promoter, methylation promoter of nucleotide site of NNMT gene, and/or methylation promoter of DNA CpG site of NNMT gene to achieve low expression, no expression, low activity or no activity of mitochondria permeability transition pore, low expression, no expression, low activity or no activity of peptidyl-prolyl cis-trans isomerase F, low or no expression of NNMT gene, high expression of DNA methylase, high expression of UHRFl, high methylation level of nucleotide site of NNMT gene, and/or high methylation level of DNA CpG site of NNMT gene in the tumor cell, then contacting the tumor cell with the compound of formula I, or an optical isomer thereof, or a racemate thereof, or a solvate thereof, or a pharmaceutically acceptable salt thereof, or a deuterated compound thereof according to the first aspect of the present invention, thereby inhibiting tumor cell.

In another preferred embodiment, the mitochondria permeability transition pore inhibitor, peptidyl-prolyl cis-trans isomerase F inhibitor, NNMT gene inhibitor, DNA methylase promoter, UHRF1 promoter, methylation promoter of nucleotide site of NNMT gene, and/or methylation promoter of DNA CpG site of NNMT gene is as described in the eighth aspect of the invention.

In the thirteenth aspect of the present invention, it provides a use of medicine kit according to the fifth aspect of the present invention in the preparation of medicine box for preventing and/or treating tumor.

In another preferred embodiment, the medicine box further comprises instruction or label.

In another preferred embodiment, the instruction or label records that the compound of formula I, or an optical isomer thereof, or a racemate thereof, or a solvate thereof, or a pharmaceutically acceptable salt thereof, or a deuterated compound thereof according to the first aspect of the present invention is suitable for use in the prevention and/or treatment of patient tumor with low expression, no expression, low activity or no activity of mitochondria permeability transition pore, low expression, no expression, low activity or no activity of peptidyl-prolyl cis-trans isomerase F, low or no expression of NNMT gene, high expression of DNA methylase, high expression of UHRFl, high methylation level of nucleotide site of NNMT gene, and/or high methylation level of DNA CpG site of NNMT gene.

In another preferred embodiment, the instruction or label records that the compound of formula I, or an optical isomer thereof, or a racemate thereof, or a solvate thereof, or a pharmaceutically acceptable salt thereof, or a deuterated compound thereof according to the first aspect of the present invention is not suitable for use in the prevention and/or treatment of patient tumor with high expression or high activity of mitochondria permeability transition pore, high expression or high activity of peptidyl-prolyl cis-trans isomerase F, high expression of NNMT gene, low expression of DNA methylase, low expression of UHRFl, low methylation level of nucleotide site of NNMT gene, and/or low methylation level of DNA CpG site of NNMT gene.

It should be understood that, in the present invention, each of the technical features specifically described above and below can be combined with each other, thereby constituting new or preferred technical solutionse.

### DESCRIPTION OF THE DRAWINGS

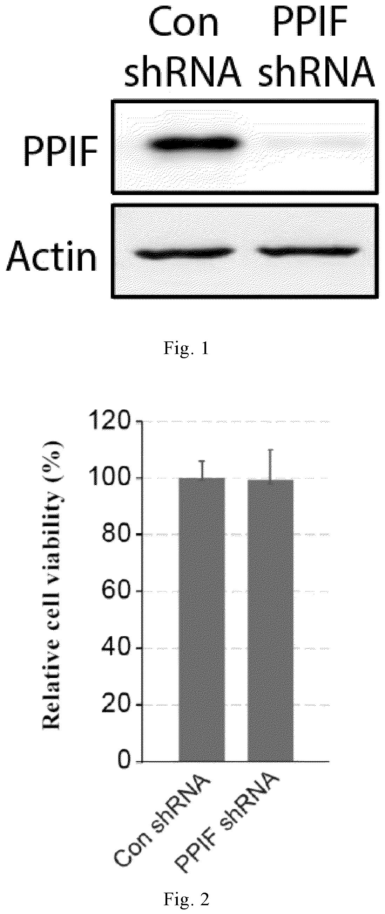

Fig.1 shows the expression content of PPIF protein using Western Blot test, wherein, Con shRNA refers to the expression content of PPIF protein in Daoy cell transfected with empty virus vector without carrying shRNA that can specificly induce the degradation of PPIF mRNA; PPIF shRNA refers to the expression content of PPIF protein in Daoy cell transfected with virus vector carrying shRNA that can specificly induce the degradation of PPIF mRNA.
Fig.2 shows the relative cell viability of Daoy cell having inactive mPTP and Daoy cell having active mPTP, wherein, Con shRNA refers to the relative viability of Daoy cell transfected with empty virus vector without carrying shRNA that can specificly induce the degradation of PPIF mRNA; PPIF shRNA refers to the relative viability of Daoy cell transfected with virus vector carrying shRNA that can specificly induce the degradation of PPIF mRNA.

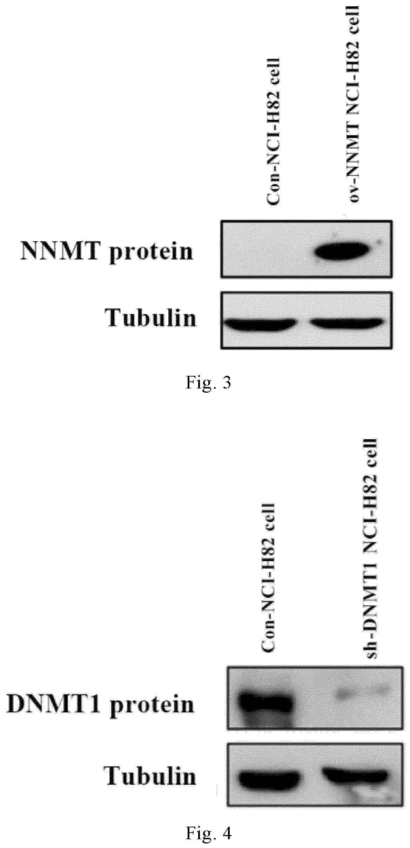

Fig.3 shows the expression content of NNMT protein in Con-NCI-H82 cell and ov-NNMT NCI-H82 cell using Western Blot test, wherein, Con-NCI-H82 refers to the expression content of NNMT protein in NCI-H82 cell transfected with empty virus vector without carrying NNMT gene and shRNA, which is used as control; ov-NNMT NCI-H82 refers to the expression content of NNMT protein in NCI-H82 cell transfected with virus vector carrying NNMT gene.
Fig.4 shows the expression content of DNMT1 protein in Con-NCI-H82 cell and sh-DNMT1 NCI-H82 cell using Western Blot test, wherein, Con-NCI-H82 refers to the expression content of DNMT1 protein in NCI-H82 cell transfected with empty virus vector without carrying NNMT gene and shRNA, which is used as control; sh-DNMT1 NCI-H82 refers to the expression content of DNMT1 protein in NCI-H82 cell transfected with virus vector carrying shRNA.

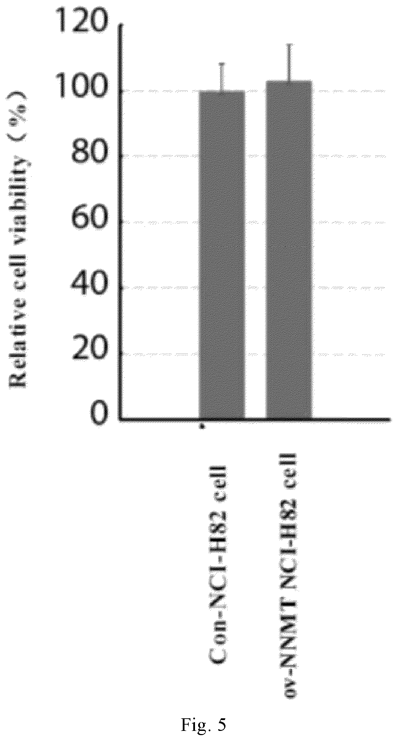

Fig.5 shows the relative cell viability of Con-NCI-H82 cell and ov-NNMT NCI-H82 cell, wherein, Con-NCI-H82 refers to the cell viability of NCI-H82 cell transfected with empty virus vector without carrying NNMT gene and shRNA, which is used as control; ov-NNMT NCI-H82 refers to the cell viability of NCI-H82 cell transfected with virus vector carrying NNMT gene.

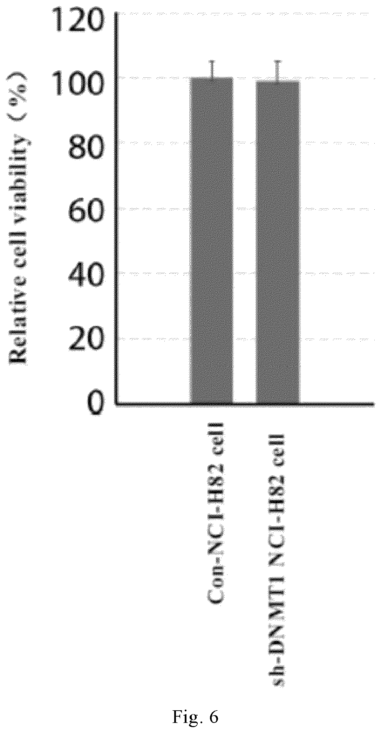

Fig.6 shows the relative cell viability of Con-NCI-H82 cell and sh-DNMT1 NCI-H82 cell, wherein, Con-NCI-H82 refers to the cell viability of NCI-H82 cell transfected with empty virus vector without carrying NNMT gene and shRNA, which is used as control; sh-DNMT1 NCI-H82 refers to the cell viability of NCI-H82 cell transfected with virus vector carrying shRNA.

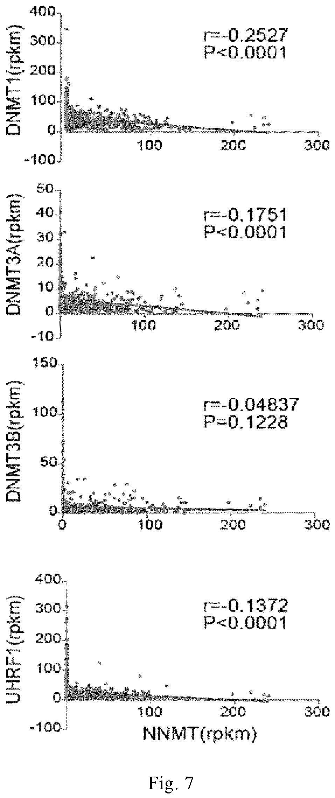

Fig.7 shows the correlation between the expression of NNMT and the expression of DNMT1, UHRF1, DNMT3a and DNMT3b in tumor cells.

### EMBODIMENTS FOR CARRYING OUT THE INVENTION

Based on an extensive and intensive research, the inventors have unexpectedly developed a compound, the compound has excellent precision treatment effect on tumor with low expression, no expression, low activity or no activity of mitochondria permeability transition pore, low expression, no expression, low activity or no activity of peptidyl-prolyl cis-trans isomerase F, low or no expression of NNMT gene, high expression of DNA methylase, high expression of UHRFl, high methylation level of nucleotide site of NNMT gene, and/or high methylation level of DNA CpG site of NNMT gene. The expression level or activity of mitochondria permeability transition pore, the expression level or activity of peptidyl-prolyl cis-trans isomerase F, the expression level of NNMT gene, the expression level of DNA methylase, the expression level of UHRF1, the methylation level of nucleotide site of NNMT gene, and/or the methylation level of DNA CpG site of NNMT gene can be used as a marker for determining whether the compound of present invention is suitable for use in the prevention and/or treatment of patient tumor. On this basis, the inventors has completed the present invention.

### Terms

Unless otherwise defined, the meanings of all technical and scientific terms used in the invention are the same as those generally understood by the skilled in the art.

As used herein, the term "comprise", " comprising", and "containing" are used interchangeably, which not only comprise open definitions, but also semi-closed and closed definitions. In other words, the term comprises "consisting of" and "essentially consisting of".

As used herein, the term "anti-tumor cancer" and "anti-tumor drug" are used interchangeably.

As used herein, the term "cancer" and "tumor" are used interchangeably.

As used herein, the term "a cell" refers to a cell (e.g., single tumor cell) or a group of cells containing multiple similar cells ((e.g., a tumor tissue).

As used herein, "the compound of present invention is suitable for use in the prevention and/or treatment of patient tumor" comprises "patient tumor is sensitive to compound of present invention".

As used herein, "the compound of present invention is not suitable for use in the prevention and/or treatment of patient tumor" comprises "patient tumor is not sensitive to compound of present invention".

As used herein, the term "IC50" and "IC₅₀" are used interchangeably, which refers to 50% inhibiting concentration, ie, the concentration of the inhibitor when 50% inhibitory effect is achieved.

As used herein, the term "methylation of CpG site", "methylation of CpG nucleotide" and "CpG methylation" are used interchangeably.

As used herein, the term "P/S" refers to adding penicillin and streptomycin into the culture medium.

As used herein, "the expression level or activity of mitochondria permeability transition pore, the expression level or activity of peptidyl-prolyl cis-trans isomerase F, the expression level of NNMT gene, the expression level of DNA methylase, the expression level of UHRF1, the methylation level of nucleotide site of NNMT gene, and/or the methylation level of DNA CpG site of NNMT gene" refers to one or more of the expression level or activity of mitochondria permeability transition pore, the expression level or activity of peptidyl-prolyl cis-trans isomerase F, the expression level of NNMT gene, the expression level of DNA methylase, the expression level of UHRF1, the methylation level of nucleotide site of NNMT gene and the methylation level of DNA CpG site of NNMT gene.

As used herein, "the low expression, no expression, low activity or no activity of mitochondria permeability transition pore, low expression, no expression, low activity or no activity of peptidyl-prolyl cis-trans isomerase F, low or no expression of NNMT gene, high expression of DNA methylase, high expression of UHRFl, high methylation level of nucleotide site of NNMT gene, and/or high methylation level of DNA CpG site of NNMT gene" refers to one or more of the low expression, no expression, low activity or no activity of mitochondria permeability transition pore, low expression, no expression, low activity or no activity of peptidyl-prolyl cis-trans isomerase F, low or no expression of NNMT gene, high expression of DNA methylase, high expression of UHRFl, high methylation level of nucleotide site of NNMT gene and high methylation level of DNA CpG site of NNMT gene.

As used herein, "the high expression or high activity of mitochondria permeability transition pore, high expression or high activity of peptidyl-prolyl cis-trans isomerase F, high expression of NNMT gene, low expression of DNA methylase, low expression of UHRFl, low methylation level of nucleotide site of NNMT gene, and/or low methylation level of DNA CpG site of NNMT gene" refers to one or more of the high expression or high activity of mitochondria permeability transition pore, high expression or high activity of peptidyl-prolyl cis-trans isomerase F, high expression of NNMT gene, low expression of DNA methylase, low expression of UHRFl, low methylation level of nucleotide site of NNMT gene and low methylation level of DNA CpG site of NNMT gene.

As used herein, mitochondria permeability transition pore is abbreviated as mPTP.

As used herein, peptidyl-prolyl cis-trans isomerase F is abbreviated as PPIF.

As used herein, the term "high methylation level of DNA CpG site", "high level of DNA CpG site methylation" and "high methylation of DNA CpG site" are used interchangeably.

As used herein, the term "low methylation level of DNA CpG site", "low level of DNA CpG site methylation" and "low methylation of DNA CpG site" are used interchangeably.

As used herein, the term "high methylation level of nucleotide site", "high level of nucleotide site methylation" and "high methylation of nucleotide site" are used interchangeably.

As used herein, the term "low methylation level of nucleotide site", "low level of nucleotide site methylation" and "low methylation of nucleotide site" are used interchangeably.

As used herein, the term "methylation of CpG site", "methylation of CpG nucleotide" and "CpG methylation" are used interchangeably.

As used herein, "the compound of present invention is suitable for use in the prevention and/or treatment of patient tumor" comprises "patient tumor is sensitive to compound of present invention".

As used herein, "the compound of present invention is not suitable for use in the prevention and/or treatment of patient tumor" comprises "patient tumor is not sensitive to compound of present invention".

As used herein, the term "NNMT" refers to Nicotinamide N-Methyltransferase.

As used herein, the term "bp" refers to base pair.

As used herein, the term "SST" refers to the transcription start site.

As used herein, the term "Chr11" refers to human chromosome 11 according to human genome version GCF_ 000001405.25 (GRCh37. p13).

As used herein, the term "human chromosome 11" refers to human chromosome 11 according to human genome version GCF_ 000001405.25 (GRCh37. p13).

As used herein, the terms "before the transcription start site" and "after the transcription start site" do not comprise the transcription start site itself.

As used herein, the terms "site 114165695 on human chromosome 11" refers to nucleotide of site 114165695 on human chromosome 11; "site 114165730 on human chromosome 11" refers to nucleotide of site 114165730 on human chromosome 11; "site 114165769 on human chromosome 11" refers to nucleotide of site 114165769 on human chromosome 11; "site 114165804 on human chromosome 11" refers to nucleotide of site 114165804 on human chromosome 11; "site 114165938 on human chromosome 11" refers to nucleotide of site 114165938 on human chromosome 11; "site 114166050 on human chromosome 11" refers to nucleotide of site 114166050 on human chromosome 11; "site 114166066 on human chromosome 11" refers to nucleotide of site 114166066 on human chromosome 11.

As used herein, the gene expression comprises the protein expression of the gene and/or the mRNA expression of the gene.

As used herein, the term "DNMT3a" and "DNMT3A" are used interchangeably, which refers to DNA methyltransferase 3a.

As used herein, the term "DNMT3b" and "DNMT3B" are used interchangeably, which refers to DNA methyltransferase 3b.

As used herein, the term "DNMT1" refers to DNA methyltransferase 1.

As used herein, the term "UHRF1" refers to ubiquitin-like with PHD and ring finger domain 1.

As used herein, the term "MS-ESI" refers to Electro Spray Ionization-Mass Spectroscopy.

As used herein, the term "1H NMR" refers to H Nuclear Magnetic Resonance Spectra.

It should be understood that the skilled in the art can choose the substituents and substituted forms on the compound of the present invention to obtain chemically stable compounds, the compound can be synthesized by the techniques known in the art and the methods described below. If the compound is substituted by more than one substituents, it should be understood that the substituents can be on the same carbon or different carbons, as long as a stable structure is obtained.

As used herein, the term "substitute" or "substituted" means the hydrogen atom on the group is substituted by non-hydrogen atom group, but it needs to meet its valence requirements and the substituted compound is chemically stable, that is, the substituted compound does not spontaneously undergo transformations such as cyclization and elimination, etc.

As used herein, the term "deuterated" refers to one or more hydrogen atoms in a compound or group are substituted by deuterium. The deuterated can be mono-substituted, di-substituted, multi-substituted or fully-substituted.

As used herein, the term "solvate" refers to a complex formed by the coordination of a compound with a solvent molecule in a specific ratio.

As used herein, "R₁", "R1" and "R¹" have the same meaning and can be used interchangeably, the other similar definitions have the same meaning.

As used herein, " 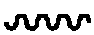 " denotes the linking site of the group.

As used herein, the term "alkyl" refers to a saturated hydrocarbon group with linear chain (ie, unbranched chain) or branched chain, or a combination of linear and branched chains, the alkyl only have carbon and hydrogen atoms. When the number of carbon atoms is limited in front of the alkyl (e.g., C1-C6 alkyl), it refers to the number of carbon atoms (e.g., 1-6) in the alkyl, for example, C1-C4 alkyl refers to an alkyl having 1-4 carbon atoms. Representative examples comprise but are not limited to methyl, ethyl, propyl, isopropyl, butyl, isobutyl, sec-butyl, tert-butyl, or the like.

As used herein, the term "alkenyl" refers to a hydrocarbon group formed by the loss of one hydrogen atom connected to the double bond in linear or branched alkene with one or more double bonds. When the number of carbon atoms is limited in front of the alkenyl (e.g., C2-C6 alkenyl), it refers to the number of carbon atoms (e.g., 1-6) in the alkenyl, for example, C2-C4 alkenyl refers to an alkenyl having 2-4 carbon atoms in the alkenyl. Representative examples comprise but are not limited to ethylenyl (e.g.,CH₂=CH-), butenyl (e.g.,C (CH₃) ₂=CH-), or the like.

As used herein, the term "halogen" refers to F, Cl, Br or I.

As used herein, the term "halo" means the group is substituted by halogen.

As used herein, the term "haloalkyl" means that one or more (preferably 1, 2, 3 or 4) hydrogens on the alkyl are substituted by halogen, the alkyl and halogen are as defined above. When the number of carbon atoms is limited in front of the haloalkyl (e.g., C1-C8 haloalkyl), it refers to the number of carbon atoms (e.g., 1-8) in the haloalkyl, for example, C1-C6 haloalkyl refers to an haloalkyl having 1-6 carbon atoms. Representative examples comprise but are not limited to -CF₃, -CHF₂, monofluoroisopropyl, difluorobutyl, or the like.

As used herein, the term "cycloalkyl" refers to a cyclic group having a saturated or partially saturated monocyclic ring, bicyclic ring or polycyclic ring (fused ring, bridged ring or spiro ring). When the number of carbon atoms is limited in front of the cycloalkyl (e.g., C3-C12 cycloalkyl), it refers to the number of ring carbon atoms (e.g., 3-12) in the cycloalkyl. For example, C3-C8 cycloalkyl refers to a saturated or partially saturated monocycloalkyl or dicycloalkyl having 3-8 ring carbon atoms, comprising cyclopropyl, cyclobutyl, cyclopentyl, cycloheptyl, or the like. The term "spirocycloalkyl" refers to a bicyclic or polycyclic group that shares a carbon atom (referred to spiro-atom) between single rings, the spirocycloalkyl can contain one or more double bonds, but no ring has a fully conjugated π electron system. Fused cycloalkyl refers to all carbon bicyclic or polycyclic group in which each rings in the system shares an adjacent pair of carbon atoms with other rings, one or more rings can have one or more double bonds, but no ring has a fully conjugated π electron system. Bridged cycloalkyl refers to all carbon polycyclic group in which any two rings share two non-directly connected carbon atoms, the bridged cycloalkyl can have one or more double bonds, but no ring has a fully conjugated π electron system. The representative examples of cycloalkyl comprise but are not limited to

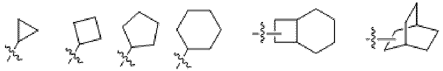

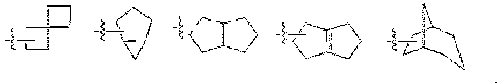

As used herein, the term "halocycloalkyl" means that one or more (preferably 1, 2, 3 or 4) hydrogens on cycloalkyl are substituted by halogen, the cycloalkyl and halogen are as defined above, When the number of carbon atoms is limited in front of the halocycloalkyl (e.g, C3-C8 halocycloalkyl), it refers to the number of ring carbon atoms (e.g., 3-8) in the halocycloalkyl, for example, C3-C8 halocycloalkyl refers to an halocycloalkyl having 3-8 ring carbon atoms. Representative examples comprises but are not limited to monofluorocyclopropyl, monochlorocyclobutyl, monofluorocyclopentyl, difluorocycloheptyl, or the like.

As used herein, the term "alkoxyl" refers to R-O- group, wherein R is alkyl, the alkyl is as defined above. When the number of carbon atoms is limited in front of the alkoxyl, for example, C1-C8 alkoxyl means that the alkyl in the alkoxyl has 1-8 carbon atoms. Representative examples of alkoxyl comprise but are not limited to methoxyl, ethoxyl, n-propoxyl, isopropoxyl, tert-butoxyl, or the like.

As used herein, the term "alkylthio" refers to R-S- group, wherein R is alkyl, the alkyl is as defined above. When the number of carbon atoms is limited in front of the alkylthio, for example, C1-C8 alkylthio means that the alkyl in the alkylthio has 1-8 carbon atoms. Representative examples of alkylthio comprise but are not limited to methylthio, ethylthio, n-propylthio, isopropylthio, tert-butylthio, or the like.

As used herein, the term "haloalkoxyl" refers to haloalkyl-O-, wherein the haloalkyl is as defined above, for example, C1-C6 haloalkoxyl refers to a haloalkoxyl having 1-6 carbon atoms. Representative examples comprise but are not limited to monofluoromethoxyl, monofluoroethoxyl, bisfluorobutoxyl, or the like.

As used herein, the term "haloalkylthio" refers to haloalkyl-S-, wherein the haloalkyl is as defined above, for example, C1-C6 haloalkylthio refers to a haloalkylthio having 1-6 carbon atoms. Representative examples of haloalkylthio comprise but are not limited to monofluoromethylthio, monofluoroethylthio, difluorobutylthio, or the like.

As used herein, the term "cycloalkoxyl" refers to R-O-, wherein R is cycloalkyl, the cycloalkyl is as defined above. When the number of carbon atoms is limited in front of the cycloalkoxyl, for example, C3-C8 cycloalkoxyl means that the cycloalkyl in the cycloalkoxyl has 3-8 ring carbon atoms. Representative examples of cycloalkoxyl comprise but are not limited to cyclopropyloxyl, cyclobutoxyl, or the like.

As used herein, the term "cycloalkylthio" refers to R-S- group, wherein R is cycloalkyl, the cycloalkyl is as defined above. When the number of carbon atoms is limited in front of the cycloalkylthio, for example, C3-C8 cycloalkylthio means that the cycloalkyl in the cycloalkylthio has 3-8 ring carbon atoms. Representative examples of cycloalkylthio comprise but are not limited to cyclopropylthio, cyclobutythio, or the like.

As used herein, the term "halocycloalkoxyl" means that one or more (preferably 1, 2, 3 or 4) hydrogens on the cycloalkoxyll are substituted by halogen, the cycloalkoxyl and halogen are as defined above. When the number of carbon atoms is limited in front of the halocycloalkoxyl (e.g., C3-C8 halocycloalkoxyl), it refers to the number of ring carbon atoms (e.g., 3-8) in the halocycloalkoxyl, for example, C3-C8 halocycloalkoxyl refers to an halocycloalkoxyl having 3-8 ring carbon atoms. Representative examples comprises but are not limited to monofluorocyclopropyl-O-, monochlorocyclobutyl-O-, monofluorocyclopentyl-O-, difluorocycloheptyl-0 -, or similar functional groups, or the like.

As used herein, the term "halocycloalkylthio" means that one or more (preferably 1, 2, 3 or 4) hydrogens on the cycloalkoxyll are substituted by halogen, the cycloalkoxyl and halogen are as defined above. When the number of carbon atoms is limited in front of the halocycloalkylthio (e.g., C3-C8 halocycloalkylthio), it refers to the number of ring carbon atoms (e.g., 3-8) in the halocycloalkylthio, for example, C3-C8 halocycloalkylthio refers to an halocycloalkylthio having 3-8 ring carbon atoms. Representative examples comprises but are not limited to monofluorocyclopropyl-S-, monochlorocyclobutyl-S-, monofluorocyclopentyl-S-, difluorocycloheptyl-S-, or similar functional groups, or the like.

As used herein, the term "heterocycloalkane ring" refers to fully saturated or partially unsaturated ring (comprising but not limited to such as 3-7 membered monocyclic ring, 7-11 membered bicyclic ring, or 8-16 membered tricyclic ring), at least one heteroatom is present in a ring with at least one carbon atom. When the number of members is limited in front of the heterocycloalkane ring, it refers to the number of ring atoms in the heterocycloalkane ring, for example, 3-16 membered heterocycloalkane ring refers to a heterocycloalkane ring having 3-16 ring atoms. Each heterocyclic ring having heteroatoms can have one or more (e.g., 1, 2, 3 or 4) heteroatoms, each of heteroatoms is independently selected from the group consisting of nitrogen atom, oxygen atom or sulfur atom, wherein the nitrogen atom or sulfur atom can be oxidized, and the nitrogen atom can also be quaternized. Representative examples of monocyclic heterocycloalkane ring comprise but are not limited to azetidine ring, oxetane ring, tetrahydrofuran ring, piperidine ring, piperazine ring. Polycyclic heterocycloalkane ring comprises spiro, fused and bridged ring, the spiro, fused and bridged heterocycloalkane ring is optionally linked with other rings by single bond, or further linked with other cycloalkane ring and heterocycloalkane ring by any two or more atoms on the ring.

As used herein, the term "heterocycloalkyl" refers to fully saturated or partially unsaturated cyclic (comprising but not limited to such as 3-7 membered monocyclic ring, 7-11 membered bicyclic ring, or 8-16 membered tricyclic ring) group, at least one heteroatom is present in a ring with at least one carbon atom. When the number of members is limited in front of the heterocycloalkyl, it refers to the number of ring atoms in the heterocycloalkyl, for example, 3-16 membered heterocycloalkyl refers to a heterocycloalkyl having 3-16 ring atoms. Each heterocyclic ring having heteroatoms can have one or more (e.g., 1, 2, 3 or 4) heteroatoms, each of heteroatoms is independently selected from the group consisting of nitrogen atom, oxygen atom or sulfur atom, wherein the nitrogen atom or sulfur atom can be oxidized, and the nitrogen atom can also be quaternized. Representative examples of monocyclic heterocycloalkyl comprise but are not limited to azetidinyl, oxetanyl, tetrahydrofuranyl, piperidinyl, piperazinyl. Polycyclic heterocycloalkyl comprises spiro, fused and bridged heterocyclyl, the spiro, fused and bridged heterocycloalkyl is optionally linked with other groups by single bond, or further linked with other cycloalkane rings and heterocycloalkane ring by any two or more atoms on the ring.

As used herein, the term "aryl" refers to an full carbon monocyclic ring or fused polycyclic ring (i.e., a ring that shares adjacent carbon atom pairs) group with a conjugated π electron system, which is aromatic cyclic hydrocarbon compound group. When the number of carbon atoms is limited in front of the aryl, for example, C6-C12 aryl means that the aryl has 6-12 ring carbon atoms, such as phenyl and naphthyl.

As used herein, the term "heteroaryl" refers to aromatic heterocyclic ring group having one to more (preferably 1, 2, 3 or 4) heteroatoms, the heteroaryl can be monocyclic ring, or polycyclic ring (bicyclic, tricyclic or polycyclic ring) fused together or covalently connected. Each of heterocyclic ring having heteroatom can have one or more (e.g., 1, 2, 3, 4) heteroatoms independently selected from the group consisting of oxygen, sulfur and nitrogen. When the number of members is limited in front of the heteroaryl, it refers to the number of ring atoms of the heteroaryl, for example, 5-12 membered heteroaryl refers to a heteroaryl having 5-12 ring atoms. Representative examples comprise but are not limited to pyrrolyl, pyrazolyl, imidazolyl, thiazolyl, furanyl, pyridyl, pyrimidinyl, etc.

As used herein, the term "carboxyl" refers to -COOH or -alkyl-COOH, the alkyl is as defined above. For example, "C2-C4 carboxyl" refers to -C1-C3 alkyl-COOH. Representative examples of carboxyl comprise but are not limited to -COOH, -CH₂COOH, or the like.

As used herein, the term "ester group" refers to R-C(O)-O- or -C(O)-O-R, wherein, R is alkyl, the alkyl is defined as above. For example, "C₂-C₄ ester group" refers to C₁-C₃ alkyl-C(O)-O- or - C(O)-O-Ci-Cs alkyl. Representative examples of ester group comprise but are not limited to CH₃C(O)O-, C₂H₅C(O)O-, (CH₃)₂CHC(O)O-, -C(O)OCH₃, -C(O)OC₂H₅, or the like.

As used herein, the term "amide group" refers to R-C(O)-N- or -C(O)-N-R, wherein, R is alkyl, the alkyl is defined as above. For example, "C₂-C₄ amide group" refers to C₁-C₃ alkyl-C(O)-N- or - C(O)-N-C₁-C₃ alkyl. Representative examples of amide group comprise but are not limited to CH₃C(O)-N-, C₂H₅C(O)-N-, (CH₃)₂CHC(O)-N-, -C(O)-N-CH₃, -C(O)-N-C₂H₅, or the like.

As used herein, the term "acyl" refers to 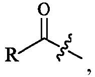 wherein R is alkyl, the alkyl is as defined above. When the number of carbon atoms is limited in front of the acyl (e.g, C2-C6 acyl), it refers to the number of carbon atoms (e.g., 2-6) in the acyl, for example, C2-C6 acyl refers to acyl having 2-6 carbon atoms. For example, the C₂-C₄ acyl refers to C₁-C₃ alkyl -C(O)-, representative examples comprises but are not limited to CH₃C(O)-, C₂H₅C (O)- , or the like.

As used herein, the term "-C(O)-" and ` 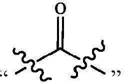 are used interchangeably.

As used alone or as part of other substituent, the term "amino" refers to -NH₂.

As used alone or as part of other substituent, the term 'nitro' refers to -NO₂.

As used alone or as part of other substituent, the term "cyano" refers to -CN.

As used alone or as part of other substituent, the term "hydroxyl" refers to -OH.

As used alone or as part of other substituent, the term "sulfhydryl" refers to -SH.

In present invention, it should be understood that all substituents are unsubstituted, unless explicitly described herein as "substituted". The specific substituent is the substituent as described above or the substituent in each Example. The "substituted" means that one or more hydrogen atoms on specific group are substituted by specific substituent. Preferably, each "substituted" means that one or more (preferably 1, 2, 3, 4, 5, 6, 7, or 8) hydrogen atoms on the ring or group are independently substituted by substituent selected from the group consisting of C1-C8 alkyl, C3-C12 cycloalkyl, C1-C8 haloalkyl, C3-C8 halocycloalkyl, C3-C8 cycloalkoxyl, C3-C8 cycloalkylthio, C3-C8 halocycloalkoxyl, C3-C8 halocycloalkylthio, halogen, nitro, -CN, hydroxyl, sulfhydryl, amino, C1-C4 carboxyl, C2-C4 ester group, C2-C4 amide group, C1-C8 alkoxyl, C1-C8 alkylthio, C1-C8 haloalkoxyl, C1-C8 haloalkylthio, C6-C12 aryl, C6-C12 aryl-O-, 5-10 membered heteroaryl, 5-10 membered heteroaryl-O-, 5-10 membered heterocycloalkyl.

In the present invention, the term "prevention" refers to a method of preventing the occurrence of disease and/or its accompanying symptoms, or protecting a subject from getting disease. The term 'prevention' also comprises delaying the occurrence of disease and/or its accompanying symptoms and reducing the risk of getting disease for subject.

In the present invention, the term "treatment" comprises delaying and terminating the progression of the disease, or eliminating the disease, and it does not require 100% inhibition, elimination and reversal. In some embodiments, compared to the level observed in the absence of the compound of present invention, the compound of present invention alleviates, inhibits and/or reverses related disease (e.g., tumor) and its accompanying symptoms, for example, by at least about 10%, at least about 30%, at least about 50%, at least about 80%, at least about 90%, or 100%.

### Compound

As used herein, the terms "compound of the present invention", "compound of formula I of the present invention" and "compound of formula I" are used interchangeably, and refer to a compound of formula I, or an optical isomer thereof, or a racemate thereof, or a solvate thereof, or a pharmaceutically acceptable salt thereof, or a deuterated compound thereof. It should be understood that the term also comprises a mixture of the above components.

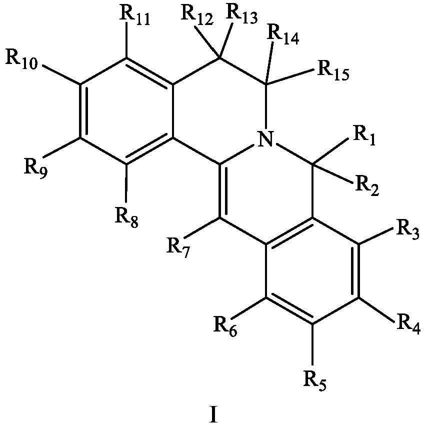

Specifically, the compound of formula I, or an optical isomer thereof, or a racemate thereof, or a solvate thereof, or a pharmaceutically acceptable salt thereof, or a deuterated compound thereof is as described above in the first aspect of the present invention.

In the present invention, the configuration of the compound of formula I can be racemate, R configuration (rectus) or S configuration (sinister). The structure and determination method of the racemate, R configuration (rectus) or S configuration (sinister) of the compound of the present invention are well known to the skilled in the art. For example, the structure of the racemate, R configuration (rectus) or S configuration (sinister) of a compound is as follows:

| racemate | R configuration (rectus) or S configuration (sinister) |
|---|---|
| 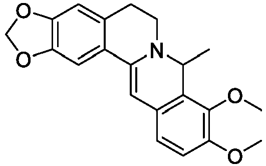 | 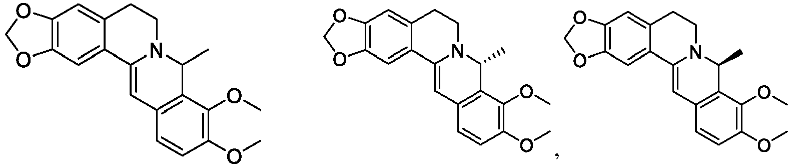 |

The racemate, R configuration (rectus) or S configuration (sinister) of other compounds is the like.

Typically, the compound of formula I of the present invention is the compound prepared in the Examples of the present invention.

The compound of formula I of the present invention can be prepared using the known organic synthesis method in the art.

The term "pharmaceutically acceptable salt" refers to a salt formed by the compound of the present invention and an acid or a base, the salt is suitable for use as a drug. Pharmaceutically acceptable salt comprises inorganic salt and organic salt. A preferred type of salt is the salt formed by the compound of the present invention and an acid. Acids suitable for salt formation comprise but are not limited to inorganic acid such as hydrochloric acid, hydrobromic acid, hydrofluoric acid, sulfuric acid, nitric acid, phosphoric acid and the like; organic acid such as formic acid, acetic acid, trifluoroacetic acid, trifluoroformic acid, propionic acid, oxalic acid, malonic acid, succinic acid, fumaric acid, maleic acid, lactic acid, malic acid, tartaric acid, citric acid, picric acid, methanesulfonic acid, benzenemethanesulfonic acid, benzenesulfonic acid and the like; and acidic amino acid such as aspartic acid and glutamic acid. A preferred type of salt is a metal salt formed by the compound of the present invention and a base. Bases suitable for salt formation comprise but are not limited to inorganic base such as sodium hydroxide, potassium hydroxide, sodium carbonate, sodium bicarbonate, sodium phosphate and the like; and organic base such as ammonia, triethylamine, diethylamine and the like.

The compound of formula I in present invention can be converted into its pharmaceutically acceptable salt using conventional methods. For example, a solution of corresponding acid can be added into the solution of above compounds, and the solvent is removed after the salt is formed, thereby forming the corresponding salt of the compound of the present invention.

The compounds of the present invention is preferably prepared as described in the Examples of the present invention.

### Mitochondria permeability transition pore

In present invention, the mitochondria permeability transition pore is abbreviated as mPTP.

The compound of present invention has excellent treatment effect on tumor with low expression, no expression, low activity or no activity of mitochondria permeability transition pore, the tumor with low expression, no expression, low activity or no activity of mitochondria permeability transition pore is sensitive to the compound of present invention.

Preferably, the tumor with low expression, no expression, low activity or no activity of mitochondria permeability transition pore is as described above in the first aspect of the invention.

In the present invention, the expression level or activity of mitochondria permeability transition pore can be determined using conventional methods, such as measuring the activity of mPTP, or measuring the expression level of mPTP at the protein or mRNA leve.

### Peptidyl-prolyl cis-trans isomerase F

In present invention, the peptidyl-prolyl cis-trans isomerase F is abbreviated as PPIF.

The compound of present invention has excellent treatment effect on tumor with low expression, no expression, low activity or no activity of peptidyl-prolyl cis-trans isomerase F, the tumor with low expression, no expression, low activity or no activity of peptidyl-prolyl cis-trans isomerase F is sensitive to the compound of present invention.

Preferably, the tumor with low expression, no expression, low activity or no activity of mitochondria permeability transition pore is as described above in the first aspect of the invention.

In the present invention, the expression level or activity of mitochondria permeability transition can be determined using conventional methods, such as measuring the activity of mPTP, or measuring the expression level of mPTP at the protein or mRNA leve.

### NNMT gene

In the present invention, the English name of NNMT is Nicotinamide N-Methyltransferase. Different databases have different identification numbers for NNMT gene as follows: HGNC: 7861; Entrez Gene: 4837; Ensembl: ENSG00000166741; OMIM: 600008; UniProtKB: P40261

According to version GCF_000001405.25 (GRCh37.p13) of human genome, the NNMT gene is located at 114,128,528 bp to 114,184,258 bp on human chromosome 11, the total length of DNA sequence of NNMT gene is 55,731 bp, the NNMT gene comprises promoter region, exon region and intron region, the transcription start site of NNMT gene is at site 114,166,535 bp.

The promoter region of NNMT gene is the nucleotide sequence from the 114164535 bp to 114167034 bp on human chromosome 11, i.e. the sequence from 2000 bp before the transcription start site (bold section) and 499 bp after the transcription start site (non-bold section) in NNMT gene, the total length of promoter region of NNMT gene is 2500 bp, The nucleotide sequence of the promoter region of NNMT gene is as shown in SEQ ID NO: 1 as follows:
**SEQ ID NO: 1:**

TATCCAAGAGCTATCAGCACTCCCATGTTTATTGTAGCACTGTTCACAATAGC
CAAGATTTGGAAGTACTCTAAGTGTCCATTAGCAGATGAATGGATAAAGACAATG
TGGTAATACACATAATGGAGTACTATTCAGTCATAAAGAAGAATTAGATCCTGTC
ATTTGCAATAACATGGATGGAACTGGAGGTCATAATGTTGAGTGAAATAAACCAG
GCACAGAAAGACAAACTTTGCATGTTCTCACTTATTTATGGGAGCTAAAAACTAA
AATAACTGAACTCACAGAGATAGAGAGTAGAAGGATGGTTACGAGAGGATGGGA
AGGGTAGCGAGGTGGGTAGGGGGGATGTGGGGATCATTAATGGGTATAAAAAT

AGTTAGAGGCCAGGCGCAGTGGCTCACGCCTGTAATCCCAGCACTTTGGGAGGC
CGAGGTAGGCGGAACACCTGAGGAGTTCAAGACCAGCCTGGCCAATATGATGAA
ACCCCGTCTCTACTAAAAATACAAAAATTAGCTGGGCGTGATGGTGTGCACCTGT
AGTCCCAGCTGCTTGGGAGGCTGAGGCAGGAGAATCGCTGGAACCCAAGAGGTG
AAGGTTGCAGTGAGCTGAGATCGCGTCACTGCACTCCAGCCTGGGTGACAGAGT
GAGACTCCACATCAAAAAAAAAAAAAAAAAGTTAGAAAGATTGAATAAGACCTAA
TATTTGCTAGCACAACAGGGTGAATATAGTAAAAAATAATTTATTTGTACCTTCA
AAAATAACTAGACAAGTATAATTGGGTTGTTTGTAACACACAAAAAATAAGTACT
TGAAGTGGTGGATACCCCATTTACCCTGATGTGATTATTTTGTATTGCAGGCCTC
TATCAGAATATCTCATGTAACCCATAAATATATACACCTACTCTGTACCCACAAAA
AGTTTTTAAAAAGAAAAATAAATAGCAACCGAAAAAAAAAGAGAGGGAGAAAAG
AAAAAAGAAAAAAAAATCAAGTGCCTGGCTGGGTAGAATAAATTCTAAGGCCAC
AATGTTACTGACCATGGGTTTTTTGGCTCTCAGTGTATAGAAATTGACACAAGGC
CAATAGTCTTCCCAAACATGCTTTACTGGAACTTACGCCCTGGCATAAGGGCCAC
AACAAAAGAGAGAGCGAATTCTCTGGCTTGCTGACTCCTTGGAAAAAACCGGTA
GGGATTTTTTTATTAGGCAAAGCACAGGAATTGACGTCAGAGGCAGGATGTGCT
GCTGGGCAAAGCATACGAGAAGTGGGGTATGCAGGTCAGCATTACTTGGTTGCA
ATGGTTATCTTGAGGAATGGGCCAACTGGTGGTCTGGCCAGTGGCAACAAGGCT
GTAAATCAATTATTCAGCATTCCTTCCCAAGGTGGGACACCCGGCAACATTGTTT
ATCTCCTAAGGCCAGTTCCTGGAATTAAGTGAAAGGATGACTAATGGACATGTTG
TCAGTGAGGTAGTGGTGTGGGTTTTGTGACCAGTGGGAATGCACGAAAGAATGC
TTTAGCGGGGAGTGAGCTGAAGCCAAGCCCCATCCCTACTCTGTCTCAAAGTGA
GTTCAGAAAAGGGGATTTAAAGAATTCTTTTTTTTTTTTTTTTTTTTTTTGAGACA
GAGTCTTGCTCTGTCGCCCAGGCTGGAGTGCAGTGGCGCCATCTTGGCTCACTG
CAAGCTCCGCCCCCCGGGTTCATGCCATTCTCCTGCCTCAGCCTCCCAAGTAGCT
GGGACTGCAGGTGCCTACCACCAAGCCCAGCTAATTTTTTGTATTTTTTTTTTAG
TAGAGACGGGGTTTCACCATGTTAGCCAGGATGGTCTCGATCTCCTGACCTCGTG
ATCTGCCCGCCTTAGCCTCCCAAAGTGCTGGGATTACAGGCATGAGCCTCCGCC
CCCGGCCTTAAATAATTCTTAAAGGAAGTAAAGTTAACTTTGAAAGAACTATCAG
GATTTGGATTGACTGAAAGGAGTGGGGAAGCTTAGGGAGGAGGTGCTTGCCAGAC
ACTGGGTCATGGCAGTGGTCGGTGAAGCTGCAGTTGCCTAGGGCAGGGATGGAGAGA
GAGTCTGGGCATGAGGAGAGGGTCTCGGGATGTTTGGCTGGACTAGATTTTACAGAA
AGCCTTATCCAGGCTTTTAAAATTACTCTTTCCAGACTTCATCTGAGACTCCTTCTTCA
GCCAACATTCCTTAGCCCTGAATACATTTCCTATCCTCATCTTTCCCTTCTTTTTTTTCC
TTTCTTTTACATGTTTAAATTTAAACCATTCTTCGTGACCCCTTTTCTTGGGAGATTCAT
GGCAAGAACGAGAAGAATGATGGTGCTTGTTAGGGGATGTCCTGTCTCTCTGAACTTT
GGGGTCCTATGCATTAAATAATTTTCCTGACGAGCTCAAGTGCTCCCTCTGGTCTACAA
TCCCTGGCGGCTGGCCTTCATCCCTTGGGCAAGCATTGCATACAGCTCATGGCCCTCCC
TCTACCATACCC.

In the present invention, the nucleotide sites from 1050 bp before the transcription start site to 499 bp after the transcription start site in NNMT gene is sites 951-2500 of nucleotide sequence as shown in SEQ ID NO: 1.

In the present invention, the nucleotide sites from 1050 bp to 193 bp before the transcription start site in NNMT gene is sites 951-1808 of nucleotide sequence as shown in SEQ ID NO: 1.

In the present invention, the nucleotide sites from 840 bp to 469 bp before the transcription start site in NNMT gene is sites 1161-1532 of nucleotide sequence as shown in SEQ ID NO: 1.

In present invention, the site 114165695, site 114165730, site 114165769, site 114165804, site 114165938, site 114166050 and site 114166066 on the human chromosome 11 correspond to the nucleotide site in SEQ ID NO: 1 as shown in Table 1:

**Table 1**

| Site on the human chromosome 11 | Corresponding to the nucleotide site in SEQ ID NO: 1 |
|---|---|
| site 114165695 | site 1161 |
| site 114165730 | site 1196 |
| site 114165769 | site 1235 |
| site 114165804 | site 1270 |
| site 114165938 | site 1404 |
| site 114166050 | site 1516 |
| site 114166066 | site 1532 |

### DNA methylation

DNA methylation is a form of chemical modification of DNA, which can change genetic performance under no change of DNA sequence. Many studies have shown that DNA methylation can cause changes in chromatin structure, DNA conformation, DNA stability and the way DNA interacts with protein, thereby regulating gene expression.

DNA methylation is one of the earliest discovered and most deeply studied epigenetic regulatory mechanisms. Broadly speaking, DNA methylation refers to the chemical modification process in which a specific base in the DNA sequence is modified with a methyl by covalent bonding with S-adenosyl methionine (SAM) as methyl donor under the catalysis of DNA methyltransferase (DNMT). This DNA methylation can occur at position C-5 of cytosine, position N-6 of adenine and position N-7 of guanine. DNA methylation in general studies mainly refers to the methylation process that occurs at the carbon atom of position C-5 on cytosine in CpG dinucleotides, the product is called 5-methylcytosine (5-mC). The 5-methylcytosine (5-mC) is the main form of DNA methylation in eukaryotic organisms such as plants and animals. DNA methylation, as a relatively stable modification state, can be passed on to new generations of DNA during DNA replication process under the action of DNA methyltransferase, which is an important epigenetic mechanism.

There are two types of DNA methylation reactions. One type is that the two unmethylated strands in the DNA are methylated, which is called denovo methylation; the other type is that the unmethylated strand of double-stranded DNA with one methylated strand and one unmethylated strand is methylated, which is called maintenance methylation.

Typically, DNA methylation is the methylation of DNA CpG site. The distribution of CpG binucleotide is very uneven in the human genome, while CpG remains or is higher than normal level in some regions of the genome. The CpG site rich region (also known as CpG island) is mainly located in the promoter region and exon regions of the gene, which is a region rich in CpG dinucleotide. About 60% of the promoters of the gene contains CpG island. The CpG is the abbreviation of cytosine (C)-phosphate (p)-guanine (G).

Gene expression is regulated by various signaling pathways, transcription factors and epigenetic modifications in the cell. DNA methylation modification is an important way in which epigenetic modifications regulate gene expression. The level of DNA methylation in a specific gene region often affects the expression level of the gene. Compared to the regulation of gene expression by signal transduction pathways and transcription factors, the effect of DNA methylation modification on gene expression is more stable in epigenetic modification, which is not easily affected by the extracellular environment. DNA methylation modification can be easily and accurately detected using existing technologies, so the DNA methylation is an ideal biomarker.

### Tumor

The compound of present invention can be used for preventing and/or treating tumor.

As used herein, the term "tumor" and "cancer" are used interchangeably.

In a preferred embodiment of the present invention, the tumor comprises tumor with low expression, no expression, low activity or no activity of mitochondria permeability transition pore. Typically, the tumor with low expression, no expression, low activity or no activity of mitochondria permeability transition pore is as described above in the first aspect of the present invention.

In a preferred embodiment of the present invention, the tumor comprises tumor with low expression, no expression, low activity or no activity of peptidyl-prolyl cis-trans isomerase F. Typically, the tumor with low expression, no expression, low activity or no activity of peptidyl-prolyl cis-trans isomerase F is as described above in the first aspect of the present invention.

In a preferred embodiment of the present invention, the tumor comprises tumor with low or no expression of NNMT gene. Typically, the tumor with low or no expression of NNMT gene is as described above in the first aspect of the present invention.

In a preferred embodiment of the present invention, the tumor comprises tumor with high expression of DNA methylase. Typically, the tumor with high expression of DNA methylase is as described above in the first aspect of the present invention.

The DNA methylase of present invention comprises but is not limited to DNMT1, DNMT3a, DNMT3b, and combinations thereof. Preferably, the DNA methylase comprises DNMT1.

In a preferred embodiment of the present invention, the tumor comprises tumor with high expression of DNMT1. Typically, the tumor with high expression of DNMT1 is as described above in the first aspect of the present invention.

In a preferred embodiment of the present invention, the tumor comprises tumor with high expression of DNMT3a. Typically, the tumor with high expression of DNMT3a is as described above in the first aspect of the present invention.

In a preferred embodiment of the present invention, the tumor comprises tumor with high expression of DNMT3b. Typically, the tumor with high expression of DNMT3b is as described above in the first aspect of the present invention.

In a preferred embodiment of the present invention, the tumor comprises tumor with high expression of UHRF1(ubiquitin-like with PHD and ring finger domain 1). Typically, the tumor with high expression of UHRF1 is as described above in the first aspect of the present invention.

In a preferred embodiment of the present invention, the tumor comprises tumor with high methylation level of nucleotide site of NNMT gene. Typically, the tumor with high methylation level of nucleotide site of NNMT gene is as described above in the first aspect of the present invention.

In a preferred embodiment of the present invention, the tumor comprises tumor with high methylation level of DNA CpG site of NNMT gene. Typically, the tumor with high methylation level of DNA CpG site of NNMT gene is as described above in the first aspect of the present invention.

Typically, the tumor of present invention is as described above in the first aspect of the present invention.

In present invention, the type of tumors corresponding to tumor cell lines are shown in Table 2

**Table 2**

| Tumor cell line | The corresponding type of tumor |
|---|---|
| NCI-H82 | Human small cell lung cancer cell |
| G-401 | Human renal carcinoma Wilms cell |
| MDA-MB-453 | Breast cancer cell |
| SW48 | Human colon adenocarcinoma cell |
| GB-1 | Human brain glioblastoma cell |
| CFPAC-1 | Human pancreatic cancer cell |
| SF126 | Human glioblastoma multiforme cell |
| 786-O | Clear cell renal cell adenocarcinoma cell |
| D341 Med | Cerebellar medulloblastoma cell |
| Kelly | Brain neuroblastoma cell |
| NB-1 | Brain neuroblastoma cell |
| Daoy cell | Human medulloblastoma cell |

### Anti-tumor drug

The anti-tumor drug can be the compound of formula I, or an optical isomer thereof, or a racemate thereof, or a solvate thereof, or a pharmaceutically acceptable salt thereof, or a deuterated compound thereof of the present invention.

### Use

A use of the compound of formula I of the present invention for preventing and/or treating tumor is provided.

Specifically, the compound of present invention has more remarkable and excellent precison treatment effect on tumor with low expression, no expression, low activity or no activity of mitochondria permeability transition pore, low expression, no expression, low activity or no activity of peptidyl-prolyl cis-trans isomerase F, low or no expression of NNMT gene, high expression of DNA methylase, high expression of UHRFl, high methylation level of nucleotide site of NNMT gene, and/or high methylation level of DNA CpG site of NNMT gene. That is, the tumor with low expression, no expression, low activity or no activity of mitochondria permeability transition pore, low expression, no expression, low activity or no activity of peptidyl-prolyl cis-trans isomerase F, low or no expression of NNMT gene, high expression of DNA methylase, high expression of UHRFl, high methylation level of nucleotide site of NNMT gene, and/or high methylation level of DNA CpG site of NNMT gene is sensitive to the compound of the present invention. The expression level or activity of mitochondria permeability transition pore, the expression level or activity of peptidyl-prolyl cis-trans isomerase F, the expression level of NNMT gene, the expression level of DNA methylase, the expression level of UHRF1, the methylation level of nucleotide site of NNMT gene, and/or the methylation level of DNA CpG site of NNMT gene can be used as a marker for determining whether the compound of the present invention is suitable for use in the precision treatment on tumor, the biomarker can be used to effectively identify patient tumor sensitive to the compound of the present invention, improve treatment effects and avoid administrating the compound of the present invention to patient tumor insensitive to the compound of the present invention, thereby achieving precision treatment on tumor.

The present invention further provides a method for preventing and/or treating tumor, which comprises administering the compound of the present invention to a subject in need.

The compound of present invention has more remarkable and excellent precison treatment effect on tumor with low expression, no expression, low activity or no activity of mitochondria permeability transition pore, low expression, no expression, low activity or no activity of peptidyl-prolyl cis-trans isomerase F, low or no expression of NNMT gene, high expression of DNA methylase, high expression of UHRFl, high methylation level of nucleotide site of NNMT gene, and/or high methylation level of DNA CpG site of NNMT gene, therefore, during the prevention and/or treatment of tumor, firstly administering mitochondria permeability transition pore inhibitor, peptidyl-prolyl cis-trans isomerase F inhibitor, NNMT gene inhibitor, DNA methylase promoter, UHRF1 promoter, methylation promoter of nucleotide site of NNMT gene, and/or methylation promoter of DNA CpG site of NNMT gene to achieve low expression, no expression, low activity or no activity of mitochondria permeability transition pore, low expression, no expression, low activity or no activity of peptidyl-prolyl cis-trans isomerase F, low or no expression of NNMT gene, high expression of DNA methylase, high expression of UHRFl, high methylation level of nucleotide site of NNMT gene, and/or high methylation level of DNA CpG site of NNMT gene in the subject tumor, then administering the compound of the present invention to prevent and/or treat tumor. Therefore, the present invention develops a compound that can significantly enhance anti-tumor effects when combined with mitochondria permeability transition pore inhibitor, peptidyl-prolyl cis-trans isomerase F inhibitor, NNMT gene inhibitor, DNA methylase promoter, UHRF1 promoter, methylation promoter of nucleotide site of NNMT gene, and/or methylation promoter of DNA CpG site of NNMT gene. The compound of the present invention can be combined with mitochondria permeability transition pore inhibitor, peptidyl-prolyl cis-trans isomerase F inhibitor, NNMT gene inhibitor, DNA methylase promoter, UHRF1 promoter, methylation promoter of nucleotide site of NNMT gene, and/or methylation promoter of DNA CpG site of NNMT gene to significantly enhance the treatment effect of the compound on tumor.

In another preferred embodiment, the subject is human and non-human mammals (rodent, rabbit, monkey, livestock, dog, cat, etc.).

In the present invention, there are no special limitations to the method for achieving low expression, no expression, low activity or no activity of mitochondria permeability transition pore, low expression, no expression, low activity or no activity of peptidyl-prolyl cis-trans isomerase F, low or no expression of NNMT gene, high expression of DNA methylase, high expression of UHRFl, high methylation level of nucleotide site of NNMT gene, and/or high methylation level of DNA CpG site of NNMT gene in the tumor. For example, the expression and activity of mitochondria permeability transition pore and/or peptidyl-prolyl cis-trans isomerase F can be specifically inhibited using methods such as gene knockout, or gene silencing (e.g, shRNA transfection), etc.

In another preferred embodiment, the peptidyl-prolyl cis-trans isomerase F inhibitor comprises shRNA.

Preferably, the nucleotide sequence of shRNA is GTTCTTCATCTGCACCATAAA.

### Marker

The present invention provides a marker for determining whether the compound of present invention is suitable for use in the prevention and/or treatment of patient tumor, the marker comprises the expression level or activity of mitochondria permeability transition pore, the expression level or activity of peptidyl-prolyl cis-trans isomerase F, the expression level of NNMT gene, the expression level of DNA methylase, the expression level of UHRF1, the methylation level of nucleotide site of NNMT gene, and/or the methylation level of DNA CpG site of NNMT gene.

In a preferred embodiment, the expression level or activity of mitochondria permeability transition pore, the expression level or activity of peptidyl-prolyl cis-trans isomerase F, the expression level of NNMT gene, the expression level of DNA methylase, the expression level of UHRF1, the methylation level of nucleotide site of NNMT gene, and/or the methylation level of DNA CpG site of NNMT gene are used as a marker for determining whether the compound of present invention is suitable for use in the prevention and/or treatment of patient tumor, the method comprises as follows:
the compound of formula I, or an optical isomer thereof, or a racemate thereof, or a solvate thereof, or a pharmaceutically acceptable salt thereof, or a deuterated compound thereof according to the first aspect of the present invention is suitable for use in the prevention and/or treatment of patient tumor with low expression, no expression, low activity or no activity of mitochondria permeability transition pore, low expression, no expression, low activity or no activity of peptidyl-prolyl cis-trans isomerase F, low or no expression of NNMT gene, high expression of DNA methylase, high expression of UHRFl, high methylation level of nucleotide site of NNMT gene, and/or high methylation level of DNA CpG site of NNMT gene; and/or
the compound of formula I, or an optical isomer thereof, or a racemate thereof, or a solvate thereof, or a pharmaceutically acceptable salt thereof, or a deuterated compound thereof according to the first aspect of the present invention is not suitable for use in the prevention and/or treatment of patient tumor with high expression or high activity of mitochondria permeability transition pore, high expression or high activity of peptidyl-prolyl cis-trans isomerase F, high expression of NNMT gene, low expression of DNA methylase, low expression of UHRFl, low methylation level of nucleotide site of NNMT gene, and/or low methylation level of DNA CpG site of NNMT gene.

Specifically, the tumor with low expression, no expression, low activity or no activity of mitochondria permeability transition pore, and/or low expression, no expression, low activity or no activity of peptidyl-prolyl cis-trans isomerase F is as described above in the first aspect of the present invention.

Specifically, the tumor with low or no expression of NNMT gene, high expression of DNA methylase (e.g, NNMT1), high expression of UHRFl, high methylation level of nucleotide site of NNMT gene, and/or high methylation level of DNA CpG site of NNMT gene is as described above in the first aspect of the present invention.

Specifically, the tumor with high expression or high activity of mitochondria permeability transition pore, and/or high expression or high activity of peptidyl-prolyl cis-trans isomerase F is as described above in the second aspect of the present invention.

In a preferred embodiment, the tumor with high expression of NNMT gene, low expression of DNA methylase (e.g, NNMT1), low expression of UHRFl, low methylation level of nucleotide site of NNMT gene, and/or low methylation level of DNA CpG site of NNMT gene is as described above in the second aspect of the present invention.

The present invention further provides a use of the marker (or the expression level of marker) or its detection reagent in the preparation of reagent kit for determining whether the compound of the present invention is suitable for use in the prevention and/or treatment of patient tumor.

### Composition or preparation, active ingredient combination, medical kit and administration method

Preferably, the composition of the present invention is pharmaceutical composition. The compositions of the present invention can comprise a pharmaceutically acceptable carrier.

The term "pharmaceutically acceptable carrier" refers to one or more compatible solid, semi-solid, liquid or gel fillers, which are suitable for use in human or animal and must have sufficient purity and sufficiently low toxicity. The "compatible" means each component and drug active ingredient in the pharmaceutical composition can be blended with each other without significantly reducing the efficacy.

It should be understood that the pharmaceutically acceptable carrier is not particularly limited in the present invention, the carrier can be selected from materials commonly used in the art, or can be obtained by a conventional method, or is commercially available. Some examples of pharmaceutically acceptable carriers are cellulose and its derivatives (e.g., methylcellulose, ethylcellulose, hydroxypropylmethylcellulose, sodium carboxymethylcellulose, etc.), gelatin, talc, solid lubricants (e.g., stearic acid, magnesium stearate), calcium sulfate, plant oil (e.g., soybean oil, sesame oil, peanut oil, olive oil, etc.), polyols (e.g., propylene glycol, glycerin, mannitol, sorbitol, etc.), emulsifier (e.g., Tween), wetting agent (e.g., sodium lauryl sulfate), buffer agent, chelating agent, thickener, pH regulator, transdermal enhancer, colorant, flavoring agent, stabilizer, antioxidant, preservative, bacteriostatic agent, pyrogen-free water, etc.

In a preferred embodiment of the present invention, the dosage form of the composition or preparation is a solid preparation, liquid preparation or semi-solid preparation.

In a preferred embodiment of the present invention, the dosage form of the composition or preparation is oral preparation, external preparation or injection preparation

Typically, the dosage form of the composition or preparation is tablet, injection, infusion, paste, gel, solution, microsphere or film.

The pharmaceutical preparation should be matched with the mode of administration. The pharmaceutical preparation of the present invention can also be given together with other synergistic therapeutic drugs before, during or after the administration. When the pharmaceutical composition or preparation is administrated, a safe and effective amount of the drug is administered to a subject in need (e.g. human or non-human mammal). The safe and effective amount is usually at least about 10 µg/kg.bw, and does not exceed about 8 µg/kg.bw in most case, preferably, the dose is about 10 µg/kg.bw to 1 mg/kg.bw. Of course, the specific dose should also take into account the route of administration, the patient's health and other factors, which are within the skill range of skilled doctors.

### The main advantages of the present invention comprise:

1. The present has developed a compound, the compound has excellent precision treatment effect on tumor with low expression, no expression, low activity or no activity of mitochondria permeability transition pore, low expression, no expression, low activity or no activity of peptidyl-prolyl cis-trans isomerase F, low or no expression of NNMT gene, high expression of DNA methylase, high expression of UHRFl, high methylation level of nucleotide site of NNMT gene, and/or high methylation level of DNA CpG site of NNMT gene. The tumor with low expression, no expression, low activity or no activity of mitochondria permeability transition pore, low expression, no expression, low activity or no activity of peptidyl-prolyl cis-trans isomerase F, low or no expression of NNMT gene, high expression of DNA methylase, high expression of UHRFl, high methylation level of nucleotide site of NNMT gene, and/or high methylation level of DNA CpG site of NNMT gene is highly sensitive to the compound of present invention, ie, the compound of present invention has more remarkable and excellent treatment effect on tumor with low expression, no expression, low activity or no activity of mitochondria permeability transition pore, low expression, no expression, low activity or no activity of peptidyl-prolyl cis-trans isomerase F, low or no expression of NNMT gene, high expression of DNA methylase, high expression of UHRFl, high methylation level of nucleotide site of NNMT gene, and/or high methylation level of DNA CpG site of NNMT gene. Therefore, the compound of present invention can realize precise treatment on tumor with low expression, no expression, low activity or no activity of mitochondria permeability transition pore, low expression, no expression, low activity or no activity of peptidyl-prolyl cis-trans isomerase F, low or no expression of NNMT gene, high expression of DNA methylase, high expression of UHRFl, high methylation level of nucleotide site of NNMT gene, and/or high methylation level of DNA CpG site of NNMT gene, thus improving the treatment effect of the compound of present invention on tumor and avoiding administrating the compound of present invention to patient tumor insensitive to such anti-tumor drug. Therefore, the precision treatment of the compound of the present invention on tumor with low expression, no expression, low activity or no activity of mitochondria permeability transition pore, low expression, no expression, low activity or no activity of peptidyl-prolyl cis-trans isomerase F, low or no expression of NNMT gene, high expression of DNA methylase, high expression of UHRFl, high methylation level of nucleotide site of NNMT gene, and/or high methylation level of DNA CpG site of NNMT gene has advantages such a more excellent prevention and treatment effect on tumors, low drug dose, and minimal side effects, which improve the precision prevention and treatment effect on tumor, reduce the side effects and improve patient compliance.

The present invention will be further illustrated below with reference to the specific examples. It should be understood that the following specific Examples are based on the technical solution and provide detailed implementation methods and specific operation processes, but the scope of protection of the present invention is not limited to the Example.

### Example

Mitochondria permeability transition pore was abbreviated as mPTP.

Peptidyl-prolyl cis-trans isomerase F was abbreviated as PPIF.

DNMT3a refers to DNA methyltransferase 3a, NCBI entrez gene: 1788; Uniprotkb/Swiss-port: Q9Y6K1.

DNMT3b refers to DNA methyltransferase 3b, NCBI entrez gene: 1789; Uniprotkb/Swiss-port: Q9UBC3.

DNMT1 refers to DNA methyltransferase 1, NCBI entrez gene: 1786; Uniprotkb/Swiss-port: P26358.

UHRF1 refers to ubiquitin-like with PHD and ring finger domain 1.

NNMT refers to Nicotinamide N-Methyltransferase.

### Example 1 Synthesis of compound AB31882

The structure of compound AB31882 was as follows:

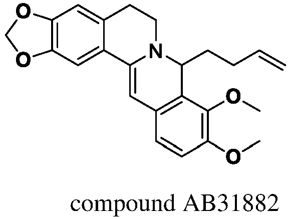

The synthesis method of compound AB31882 was as follows:

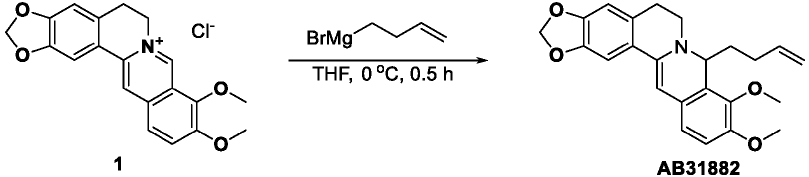

Compound 1 (200 mg, 0.54 mmol, 1 eq) was dissolved in anhydrous tetrahydrofuran (10 mL), and butenylmagnesium bromide (2.7 mL, 2.7 mmol, 5 eq, 1M) was slowly added dropwise at 0 °C under N₂. The mixture was reacted for 0.5 h, and the reaction was detected to be performed completely using LCMS. The reaction mixture was quenched with saturated aqueous ammonium chloride solution and then extracted with ethyl acetate, the organic phase was dried over anhydrous sodium sulfate, filtered and rotary dried. The crude product was purified by thin layer chromatography (DCM:MeOH=30:1) to afford compound AB31882.

### Compound AB31882:

MS-ESI: calculated [M+H]⁺: 392.18, Found: 392.20.

¹H NMR (400 MHz, CDC13) δ 7.14 (s, 1H), 6.75-6.72 (m, 2H), 6.59 (s, 1H), 5.94 (s, 2H), 5.81(s, 1H), 5.76-5.67 (m, 1H), 4.95-4.84 (m, 2H), 4.76-4.73 (m, 1H), 3.88 (s, 3H), 3.85 (s, 3H), 3.49-3.29 (m, 2H), 2.93-2.80 (m, 2H), 2.06-1.71 (m, 4H).

### Example 2 Synthesis of compound AB31883

The structure of compound AB31883 was as follows:

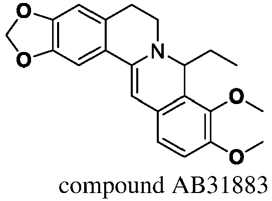

The synthesis method of compound AB31883 was as follows:

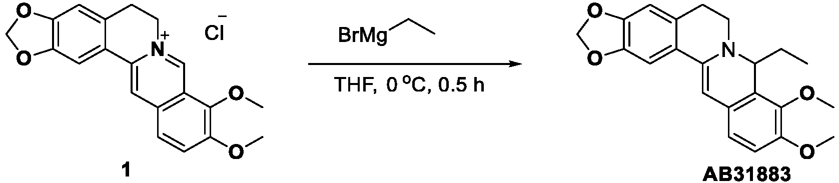

Compound 1 (200 mg, 0.54 mmol, 1 eq) was dissolved in anhydrous tetrahydrofuran (10 mL), and ethylmagnesium bromide (2.7 mL, 2.7 mmol, 5 eq, 1M) was slowly added dropwise at 0 °C under N₂. The mixture was reacted for 0.5 h, and the reaction was detected to be performed completely using LCMS. The reaction mixture was quenched with saturated aqueous ammonium chloride solution and then extracted with ethyl acetate, the organic phase was dried over anhydrous sodium sulfate, filtered and rotary dried. The crude product was purified by thin layer chromatography (DCM:MeOH=30:1) to afford compound AB31883.

### Compound AB31883

MS-ESI: calculated [M+H]⁺: 366.17, Found: 366.10.

¹H NMR (400 MHz, CDC13) δ 7.14 (s, 1H), 6.76-6.71 (m, 2H), 6.59 (s, 1H), 5.94 (s, 2H), 5.78 (s, 1H), 4.70-4.67 (m, 1H), 3.88 (s, 3H), 3.84 (s, 3H), 3.46-3.44 (m, 1H), 3.32-3.27 (m, 1H), 2.93-2.79 (m, 2H), 1.78-1.64 (m, 2H), 0.82-0.78 (m, 3H).

### Example 3 Synthesis of compound AB31884

The structure of compound AB31884 was as follows:

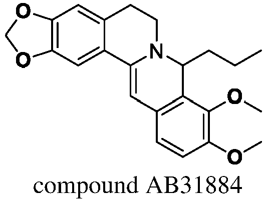

The synthesis method of compound AB31884 was as follows:

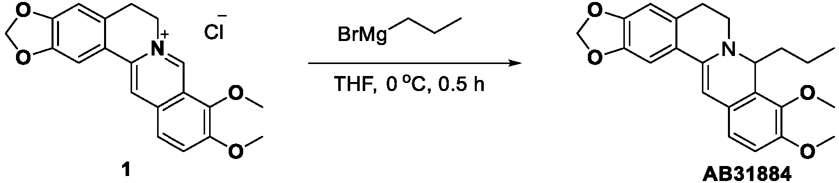

Compound 1 (200 mg, 0.54 mmol, 1 eq) was dissolved in anhydrous tetrahydrofuran (10 mL), and propylmagnesium bromide (2.7 mL, 2.7 mmol, 5 eq, 1M) was slowly added dropwise at 0 °C under N₂. The mixture was reacted for 0.5 h, and the reaction was detected to be performed completely using LCMS. The reaction mixture was quenched with saturated aqueous ammonium chloride solution and then extracted with ethyl acetate, the organic phase was dried over anhydrous sodium sulfate, filtered and rotary dried. The crude product was purified by thin layer chromatography (DCM:MeOH=30:1) to afford compound AB31884.

### Compound AB31884

MS-ESI: calculated [M+H]⁺: 380.18, Found: 380.10.

¹H NMR (400 MHz, CDC13) δ 7.14 (s, 1H), 6.76-6.73 (m, 2H), 6.59 (s, 1H), 5.94 (s, 2H), 5.80 (s, 1H), 4.73-4.70 (m, 1H), 3.88 (s, 3H), 3.84 (s, 3H), 3.47-3.20 (m, 2H), 2.87-2.79 (m, 2H), 1.73-1.59 (m, 2H), 1.29-1.20 (m, 2H), 0.81-0.78 (m, 3H).

### Example 4 Synthesis of compound AB31885

The structure of compound AB31885 was as follows:

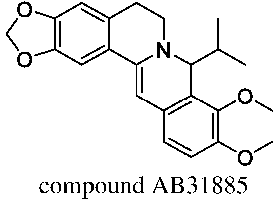

The synthesis method of compound AB31885 was as follows:

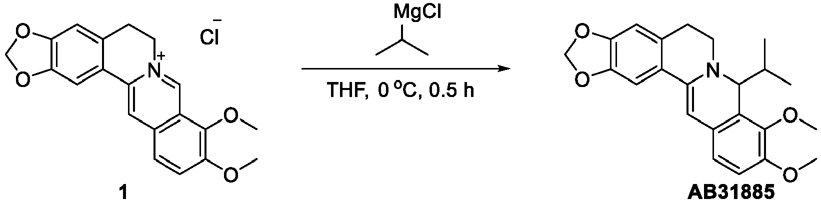

Compound 1 (200 mg, 0.54 mmol, 1 eq) was dissolved in anhydrous tetrahydrofuran (10 mL), and isopropylmagnesium bromide (2.7 mL, 2.7 mmol, 5 eq, 1M) was slowly added dropwise at 0 °C under N2. The mixture was reacted for 0.5 h, and the reaction was detected to be performed completely using LCMS. The reaction mixture was quenched with saturated aqueous ammonium chloride solution and then extracted with ethyl acetate, the organic phase was dried over anhydrous sodium sulfate, filtered and rotary dried. The crude product was purified by thin layer chromatography (DCM:MeOH=30:1) to afford compound AB31885.

### Compound AB31885:

MS-ESI: calculated [M+H]⁺: 380.18, Found: 380.20.

¹H NMR (400 MHz, CDC13) δ 7.13 (s, 1H), 6.78-6.71 (m, 2H), 6.59 (s, 1H), 5.94 (s, 2H), 5.77 (s, 1H), 5.43-5.42 (m, 1H), 3.87 (s, 3H), 3.84 (s, 3H), 3.51-3.45 (m, 2H), 2.97-2.92 (m, 1H), 2.81-2.74 (m, 1H), 2.07-2.02 (m, 1H), 0.90-0.86 (m, 6H).

### Example 5 Synthesis of compound AB31886

The structure of compound AB31886 was as follows:

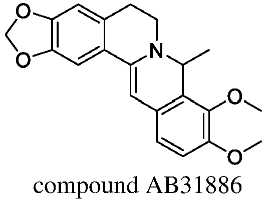

The synthesis method of compound AB31886 was as follows:

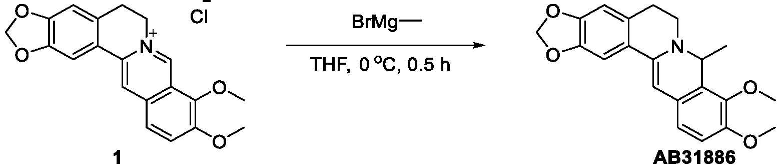

Compound 1 (1.0 g, 2.69 mmol, 1 eq) was dissolved in anhydrous tetrahydrofuran (30 mL), and methylmagnesium bromide (4.5 mL, 13.45 mmol, 5 eq, 3M) was slowly added dropwise at 0 °C under N₂. The mixture was reacted for 0.5 h, and the reaction was detected to be performed completely using LCMS. The reaction mixture was quenched with saturated aqueous ammonium chloride solution and then extracted with ethyl acetate, the organic phase was dried over anhydrous sodium sulfate, filtered and rotary dried. The crude product was purified by fast chromatography (100-200 mesh silica gel, DCM:MeOH=100:1) to afford compound AB31886.

### Compound AB31886:

MS-ESI: calculated [M+H]⁺: 352.15, Found: 352.10.

¹H NMR (400 MHz, CDC13) δ 7.15 (s, 1H), 6.74 (s, 2H), 6.59 (s, 1H), 5.94 (s, 2H), 5.85 (s, 1H), 4.87-4.83 (m, 1H), 3.90 (s, 3H), 3.84 (s, 3H), 3.41-3.32 (m, 2H), 2.94-2.77 (m, 2H), 1.18 (d, J= 8.0 Hz, 3H).

### Example 6 Synthesis of compound AB31891

The structure of compound AB31891 was as follows:

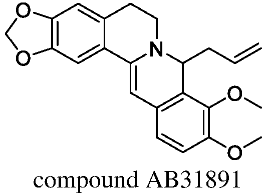

The synthesis method of compound AB31891 was as follows:

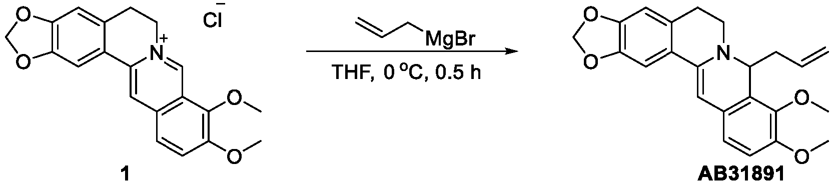

Compound 1 (200 mg, 0.54 mmol, 1 eq) was dissolved in anhydrous tetrahydrofuran (10 mL), and propenylmagnesium bromide (2.7 mL, 2.7 mmol, 5 eq, 1M) was slowly added dropwise at 0 °C under N₂. The mixture was reacted for 0.5 h, and the reaction was detected to be performed completely using LCMS. The reaction mixture was quenched with saturated aqueous ammonium chloride solution and then extracted with ethyl acetate, the organic phase was dried over anhydrous sodium sulfate, filtered and rotary dried. The crude product was purified by thin layer chromatography (DCM:MeOH=50:1) to afford compound AB31891.

### Compound AB31891:

MS-ESI: calculated [M+H]⁺: 378.17, Found: 378.35.

¹H NMR (400 MHz, CDC13) δ 7.11 (s, 1H), 6.72-6.69 (m, 2H), 6.56 (s, 1H), 5.92 (d, J= 8.0 Hz, 2H), 5.83-5.74 (m, 2H), 4.88-4.80 (m, 3H), 3.87 (s, 3H), 3.82 (s, 3H), 3.48-3.26 (m, 2H), 2.84-2.77 (m, 2H), 2.44-2.40 (m, 3H).

### Example 7 Synthesis of compound AB31893

The structure of compound AB31893 was as follows:

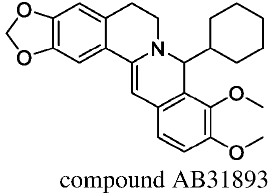

The synthesis method of compound AB31893 was as follows:

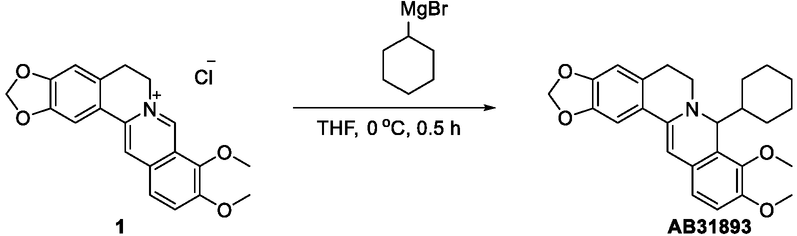

Compound 1 (200 mg, 0.54 mmol, 1 eq) was dissolved in anhydrous tetrahydrofuran (10 mL), and cyclohexylmagnesium bromide (2.7 mL, 2.7 mmol, 5 eq, 1M) was slowly added dropwise at 0 °C under N₂. The mixture was reacted for 0.5 h, and the reaction was detected to be performed completely using LCMS. The reaction mixture was quenched with saturated aqueous ammonium chloride solution and then extracted with ethyl acetate, the organic phase was dried over anhydrous sodium sulfate, filtered and rotary dried. The crude product was purified by thin layer chromatography (DCM:MeOH=50:1) to afford compound AB31893.

### Compound AB31893:

MS-ESI: calculated [M+H]⁺: 420.21, Found: 420.20.

¹H NMR (400 MHz, DMSO-d6) δ 7.23 (s, 1H), 6.82 (d, J= 8.0 Hz, 1H), 6.76 (s, 1H), 6.66 (d, J= 8.0 Hz, 1H), 5.99 (s, 2H), 5.85 (s, 1H), 4.46-4.45 (m, 1H), 3.75 (d, J= 8.0 Hz, 6H), 3.41-3.38 (m, 2H), 2.96-2.89 (m, 1H), 2.68-2.64 (m, 1H), 1.65-1.50 (m, 7H), 1.11-1.03 (m, 4H).

### Example 8 Synthesis of compound AB31900

The structure of compound AB31900 was as follows:

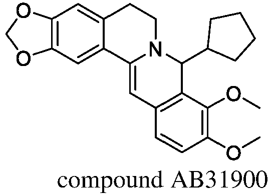

The synthesis method of compound AB31900 was as follows:

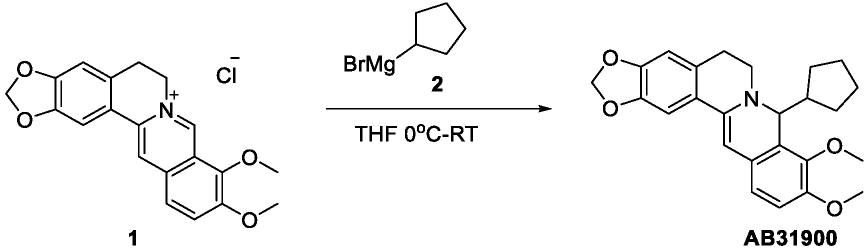

Compound 1 (200 mg, 0.54 mmol, 1 eq) was dissolved in anhydrous tetrahydrofuran (10 mL), and cyclopentylmagnesium bromide (2.7 mL, 2.7 mmol, 5 eq, 1M) was slowly added dropwise at 0 °C under N₂. The mixture was reacted for 0.5 h, and the reaction was detected to be performed completely using LCMS. The reaction mixture was quenched with saturated aqueous ammonium chloride solution and then extracted with ethyl acetate, the organic phase was dried over anhydrous sodium sulfate, filtered and rotary dried. The crude product was purified by thin layer chromatography (DCM:MeOH=30:1) to afford compound AB31900.

### Compound AB31900:

MS-ESI: calculated [M+H]⁺: 406.20, Found: 406.20.

¹H NMR (400 MHz, DMSO-d6) δ 7.20 (s, 1H), 6.79 (d, J= 8.0 Hz, 1H), 6.73 (s, 1H), 6.64 (d, J= 8.0 Hz, 1H), 5.96 (s, 2H), 5.87 (s, 1H), 4.51 (d, J= 8.0 Hz, 1H), 3.72 (s, 6H), 3.41-3.35 (m, 2H), 2.86-2.63 (m, 2H), 2.17-2.15 (m, 1H), 1.55-1.15 (m, 8H).

### Example 9 Synthesis of compound AB35403

The structure of compound AB35403 was as follows:

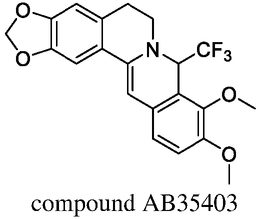

The synthesis method of compound AB35403 was as follows:

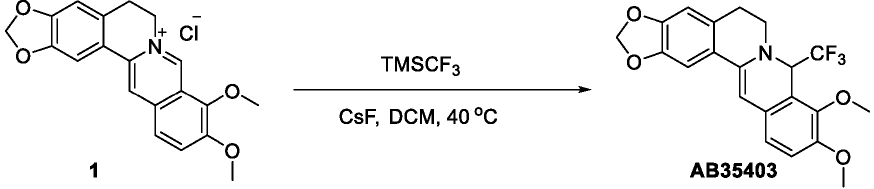

Compound 1 (500 mg, 1.34 mmol, 1 eq) was dissolved in dichloromethane (50 mL), and cesium fluoride (204 mg, 1.34 mmol, 1 eq) and (trifluoromethyl) trimethylsilane (96 mg, 0.67 mmol, 2 eq) were added. The mixture was reacted at 40 °C for overnight under N₂, and the reaction was detected to be performed completely using LCMS. The reaction mixture was diluted with wate and then extracted with dichloromethane, the organic phase was dried over anhydrous sodium sulfate, filtered and rotary dried. The crude product was purified by fast chromatography (dichloromethane) to afford compound AB35403.

### Compound AB35403:

MS-ESI: calculated [M+H]⁺: 406.12, Found: 406.10.

¹H NMR (400 MHz, DMSO-d6) δ 7.26 (s, 1H), 7.03 (d, J= 8.0 Hz, 1H), 6.82 (s, 1H), 6.79 (s, 1H), 6.03 (s, 1H), 6.01 (s, 2H), 5.52-5.46 (m, 1H), 3.82 (d, J= 8.0 Hz, 6H), 3.58-3.55 (m, 2H), 2.89-2.71 (m, 2H).

### Example 10 Synthesis of compound AB31875

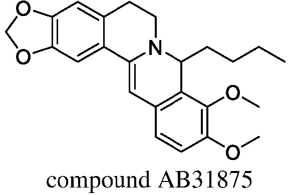

The synthesis method of compound AB31875 was as follows:

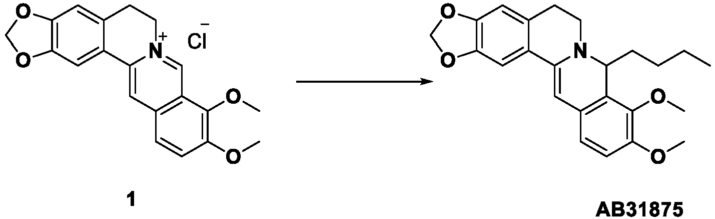

Compound 1 (336 mg, 1 mmol) was dissolved in 10mL n-butylmagnesium bromide. The mixture was stirred at 0 °C for 10 min, and the product was detected using LCMS. The reaction mixture was quenched with water, the tetrahydrofuran was added, and then the mixture was filtered to obtain filtrate, the organic solvent was removed by distillation under reduced pressure. The crude product was purified by preparation chromatography to afford compound AB31875.

MS-ESI 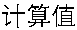 [M+1] ⁺ 394.48, Found: 394.20.

¹H NMR (400 MHz, dmso) δ 7.20 (s, 1H), 6.78 (s, 1H), 6.74 (s, 1H), 6.64 (s, 1H), 5.97 (s, 2H), 5.82 (s, 1H), 4.66 - 4.56 (m, 1H), 3.73 (s, 6H), 3.20 - 3.17 (m, 2H), 2.92 - 2.64 (m, 2H), 1.60 - 1.46 (m, 1H), 1.13 (s, 3H), 0.72 (s, 3H).

### Example 11 Synthesis of compound AB35435

The structure of compound AB35435 was as follows:

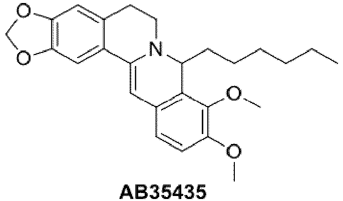

The synthesis method of compound AB35435 was as follows:

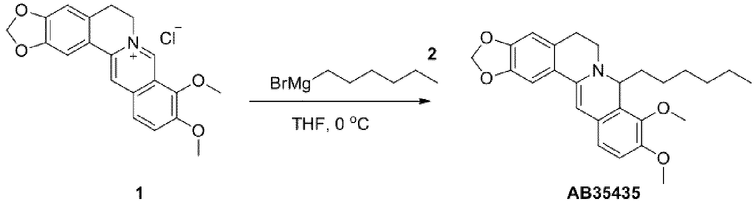

Compound 1 (150 mg, 0.40 mmol, 1.0 eq) was dissolved in anhydrous tetrahydrofuran (10 mL), and hexylmagnesium bromide (2 mL, 2.0 mmol, 5.0 eq, 1M) was added at 0 °C. The mixture was reacted at room temperature for 0.5 h, and the reaction was detected to be performed completely using TLC and LCMS. The reaction mixture was quenched with saturated aqueous ammonium chloride solution and then extracted with ethyl acetate twice, the organic phase was dried over anhydrous sodium sulfate, filtered and rotary dried. The crude product was purified by fast chromatography (dichloromethane/methanol=50/1) to afford compound AB35435.

MS-ESI: calculated [M+H]+: 422.23, Found: 422.40.

¹H NMR (400 MHz, DMSO-d6) δ 7.23 (s, 1H), 6.82 (d, J= 8.0 Hz, 1H), 6.77 (s, 1H), 6.66 (d, J= 8.0 Hz, 1H), 6.00 (d, J= 4.0 Hz, 2H), 5.85 (s, 1H), 4.66-4.63 (m, 1H), 3.76 (s, 6H), 3.31-3.26 (m, 2H), 2.85-2.67 (m, 2H), 1.62-1.49 (m, 2H), 1.20-1.13 (m, 8H), 0.80-0.77 (m,

### Example 12 Synthesis of compound AB31714

The structure of compound AB31714 was as follows:

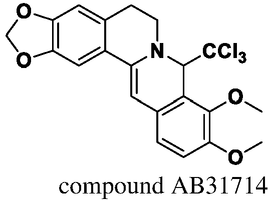

The synthesis method of compound AB31714 was as follows:

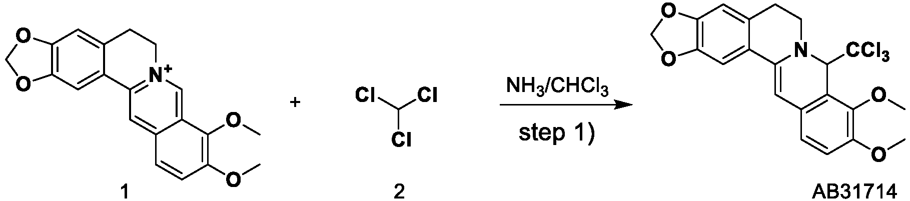

Compound 1 (2 g, 5.95 mmol) was dissolved in chloroform (60 mL), and aqueous aammonia (24 mL) was added dropwise. The mixture was stirred at room temperature for overnight, and new spot were detected on a plate. The reaction mixture was extracted, the filtrate was concentrated under reduced pressure to remove the organic solvent. The crude product was purified by preparation chromatography to afford solid compound AB31714

MS-ESI: 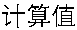 [M+1]+ 454.03: , Found: 454.10.

¹H NMR (400 MHz, DMSO-d6) δ 7.29 (s, 1H), 7.06 (d, J = 8.5 Hz, 1H), 6.86 (d, J = 8.5 Hz, 1H), 6.76 (s, 1H), 6.27 (s, 1H), 5.97 (d, J = 5.9 Hz, 2H), 5.61 (s, 1H), 3.78 (t, J = 6.8 Hz, 7H), 3.61 (t, J = 9.0 Hz, 1H), 3.26 - 3.13 (m, 2H), 2.68 (d, J = 16.0 Hz, 1H).

### Example 13 Synthesis of compound AB31713

The structure of compound AB31713 was as follows:

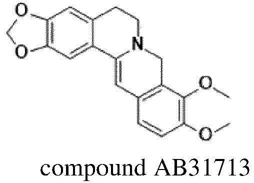

Compound AB31713 was obtained through commercial purchase.

### Example 14

### The activity of mitochondria permeability transition pore in related cell was investigated.

### Experimental background:

The mitochondria permeability transition pore (mPTP) was a non-specific channel on the inner membrane of mitochondria, which could allow small molecules with molecular less than 1.5KD to pass freely, and its activity was affected by peroxides (such as H₂O₂), pH and calcium ions in mitochondria. Some cells had active mitochondria permeability transition pore, while some cells had inactive mitochondria permeability transition pore. For cells with active mitochondria permeability transition pore, adding peroxide (such as H₂O₂) could increase the activity of mitochondria permeability transition pore, resulting in a decrease in mitochondria membrane potential. For cells with inactive mitochondria permeability transition pore, adding peroxide (such as H₂O₂) had no significant effect on the activity of the mitochondria permeability transition pore, and the mitochondria membrane potential had no significant change. Based on this principle, whether the mPTP was active or inactive in specific cell could be determined by measuring the change of potential difference of the mitochondria membrane under peroxide stimulation

### Experimental method and result:

Daoy cell (human medulloblastoma cell, ATCC no. HTB-186) were cultured in 10% fetal bovine serum-containing DMEM (+p/s), and then 1.5 µM Cyclosporin A (CSA, CSA could effectively inhibit the activity of mitochondria permeability transition pore) was added to the medium, and the cell cultured in medium without the addition of CsA were used as blank control. The mitochondria membrane potential difference was detected by Tetramethylrhodamine (TMRM), the high fluorescence intensity of TMRM represented the high membrane potential difference, the result was shown in Table 3.

**Table 3 Relative TMRM signal intensity (%) in Daoy cell after different treatments**

| **Cell** | **Daoy cell** | | | |
|---|---|---|---|---|
| CsA | - | - | + | + |
| H₂O₂ | - | + | - | + |
| mean value | 100 | 82 | 125 | 121 |
| Standard deviation | 7 | 11 | 14 | 11 |
| Repeat times | 3 | 3 | 3 | 3 |
| P value | | <0.05 | <0.01 | |

| | | | | |
|---|---|---|---|---|
| Remarks: "+" represented existence, "-" represented none. | | | | |

It could be seen from Table 3 that as for normal Daoy cell cultured in medium without the addition of CSA, the membrane potential was significantly down-regulated under the action of H₂O₂, indicating that the mitochondria permeability transition pore was active in Daoy cell. However, as for normal Daoy cell cultured in medium with the addition of CSA (CsA could inhibit the activity of mitochondria permeability transition pore), the membrane potential was not down-regulated, indicating that active mPTP of Daoy cell was inhibited by CsA and the mPTP of Daoy cell become inactive, H₂O₂ did not cause a decrease in membrane potential in this case.

Therefore, Table 3 showed that the mitochondria permeability transition pore was active in Daoy cell.

### Example 15

The inhibitory effect of the compounds prepared in the Examples of the present invention on the Daoy cell with normal activity of mPTP, and the Daoy cell with low activity of mPTP which was constructed by transfecting the Daoy cell with shRNA that targeted PPIF mRNA.

The peptidyl-prolyl cis-trans isomerase F was abbreviated as PPIF, the protein identification number was UniProtKB/Swiss Prot: P30405, and the gene identification number was NCBI Entrez Gene: 10105

### 1. Construction of Daoy cell with low activity of mitochondria permeability transition pore (mPTP)

### 1.1 Experimental background:

The activity of mitochondria permeability transition pore (mPTP) was regulated by the PPIF protein. When PPIF protein was inhibited, mPTP activity was significantly decreased. PPIF protein activity was restricted by the expression level of PPIF protein in cell. The PPIF protein expression level in Daoy cell can be specifically decreased by transfecting the Daoy cell with shRNA that could specificly induce the degradation of PPIF mRNA, thereby constructing Daoy cell having inactive mitochondria permeability transition pore (mPTP) (abbreviated as mPTP-inactive Daoy cell).

### 1.2 Experiment method and result:

A viral vector carrying shRNA that could specifically induces the degradation of PPIF mRNA was obtained using cloning technology, the shRNA (the nucleotide sequence was GTTCTTCATCTGCACCATAAA (SEQ ID NO: 2)) carried by viral vector could specifically induce the degradation of PPIF mRNA, the Daoy cell was tranfected with the viral vector carring shRNA that could specifically induce the degradation of PPIF mRNA, and the Daoy cell transfected with empty virus vector without carrying shRNA that can specificly induce the degradation of PPIF mRNA was used as control. The expression level of PPIF protein in Daoy cell was detected using Western blot technique, and the result was shown in Fig. 1. The Fig.1 showed the PPIF in the Daoy cell tranfected with the shRNA that could specifically induce the degradation of PPIF (mPTP regulated protein) mRNA was not expressed (i.e. PPIF shRNA in Fig.1), while the PPIF in the Daoy cell not tranfected with the shRNA that could specifically induce the degradation of PPIF mRN was normally expressed (i.e. con shRNA in Fig.1, as the control). The mitochondria membrane potential difference was detected in the Daoy cell transfected with empty virus vector without carrying shRNA that could specificly induce the degradation of PPIF mRNA and the Daoy cell transfected with virus vector carrying shRNA that could specificly induce the degradation of PPIF mRNA was detected by Tetramethylrhodamine (TMRM) according to the method described in Example 14 above, the high fluorescence intensity of TMRM represented the high membrane potential difference, the results were shown in Table 4 and Table 5.

**Table 4 Relative TMRM signal intensity (%) in the Daoy cell transfected with empty virus vector without carrying shRNA that could specificly induce the degradation of PPIF mRNA after different treatments**

| **Cell** | The Daoy cell transfected with empty virus vector without carrying shRNA that could specificly induce the degradation of PPIF mRNA | | | |
|---|---|---|---|---|
| CsA | - | - | + | + |
| H₂O₂ | - | + | - | + |
| mean value | 100 | 79 | 131 | 126 |
| Standard deviation | 6 | 7 | 10 | 8 |
| Repeat times | 3 | 3 | 3 | 3 |

**Table 5 Relative TMRM signal intensity (%) in the Daoy cell transfected with virus vector carrying shRNA that could specificly induce the degradation of PPIF mRNA after different treatments**

| **Cell** | The Daoy cell transfected with virus vector carrying shRNA that could specificly induce the degradation of PPIF mRNA | | | |
|---|---|---|---|---|
| CsA | - | - | + | + |
| H₂O₂ | - | + | - | + |
| mean value | 100 | 95 | 105 | 101 |
| Standard deviation | 5 | 6 | 8 | 11 |
| Repeat times | 3 | 3 | 3 | 3 |

The Table 4 and Table 5 showed the mitochondria permeability transition pore (mPTP) is active in the Daoy cell transfected with empty virus vector without carrying shRNA that could specificly induce the degradation of PPIF mRNA, the mitochondria permeability transition pore (mPTP) is inactive in the Daoy cell transfected with virus vector carrying shRNA that could specificly induce the degradation of PPIF mRNA, therefore, the Daoy cell having inactive mitochondria permeability transition pore (mPTP) was successfully constructed by transfecting the Daoy cell with shRNA that could specificly induce the degradation of PPIF (mPTP regulated protein) mRNA.

The cell viability of the mPTP-inactive Daoy cell constructed by transfecting the Daoy cell with shRNA that could specificly induce the degradation of PPIF mRNA and the mPTP-active Daoy cell not transfected with shRNA that could specificly induce the degradation of PPIF mRNAwas detected using the Promega CellTiter-Glo kit which reflected cell viability by measuring intracellular ATP content, the results was shown in Fig.2, it could be seen from Fig.2 that cell viability of mPTP-inactive Daoy cell constructed by transfecting the Daoy cell with shRNA that could specificly induce the degradation of PPIF mRNA and the mPTP-active Daoy cell not transfected with shRNA that could specificly induce the degradation of PPIF mRNA was almost the same, and the difference in cell viability was not statistically significant.

### 2. The correlation between the inhibitory effect of the compound sprepared in the Examples of the present invention on tumor cell and the activity level of mPTP was investigated

### 2.1 Experimental background:

The inhibitory effect (IC₅₀ value) of the compounds prepared in the Examples of the present invention on the mPTP-active Daoy cell not transfected with shRNA that could specificly induce the degradation of PPIF mRNA and the mPTP-inactive Daoy cell constructed by transfecting the Daoy cell with shRNA that could specificly induce the degradation of PPIF mRNA was detected using Promega CellTiter-Glo kit which refleced cell viability by directly measured intracellular ATP content.

### 2.2 Experiment method and result:

The mPTP-active Daoy cell not transfected with shRNA that could specificly induce the degradation of PPIF mRNA and the mPTP-inactive Daoy cell constructed by transfecting the Daoy cell with shRNA that could specificly induce the degradation of PPIF mRNA were cultured in 10% fetal bovine serum-containing DMEM (+p/s), the 50% inhibiting concentration (IC₅₀) of the compounds prepared in the Examples of present invention on the two types of cells was detected, and the experimental results were shown in Table 6:

**Table 6 the inhibitory effect of the compounds prepared in the Examples of present invention on the mPTP-active Daoy cell and mPTP-inactive Daoy cell (IC₅₀, µM)**

| Compound | mPTP-active Daoy cell (IC₅₀, µM) | mPTP-inactive Daoy cell (IC₅₀, µM ) |
|---|---|---|
| compound AB31886 | 14.33 | 5.74 |
| compound AB31883 | 5.64 | 2.45 |
| compound AB31884 | 5.76 | 2.71 |
| compound AB31885 | 6.43 | 3.17 |
| compound AB31891 | 6.22 | 3.12 |
| compound AB31875 | 14.58 | 6.25 |
| compound AB35435 | 1.32 | 0.69 |
| compound AB31882 | 19.33 | 9.22 |
| compound AB31714 | 26.69 | 12.46 |
| compound AB35403 | 60.12 | 28.62 |
| compound AB31900 | 5.37 | 2.56 |
| compound AB31893 | 5.14 | 2.50 |
| compound AB31713 | 25.04 | 12.50 |

| | | |
|---|---|---|
| Noted: IC₅₀ refered to 50% inhibiting concentration, ie, the concentration of the inhibitory compound when 50% inhibitory effect was achieved. The mPTP-active Daoy cell refered to the Daoy cell transfected with empty virus vector without carrying shRNA that can specificly induce the degradation of PPIF mRNA; The mPTP-inactive Daoy cell refered to the Daoy cell transfected with virus vector carrying shRNA that can specificly induce the degradation of PPIF mRNA. | | |

As was shown in Table 6, the compounds prepared in the Examples of the present invention had significant inhibitory effect on mPTP-inactive Daoy cell, while the compounds prepared in the Examples of the present invention had weaker inhibitory effect on mPTP-active Daoy cell. Decreasing the mPTP activity in Daoy cell could significantly improve the inhibitory effect of the compounds prepared in the Examples of the present invention on tumor, therefore, the compounds prepared in the Examples of the present invention had more significant inhibitory effect on mPTP-inactive Daoy cell, the mPTP-inactive Daoy cell was highly sensitive to the compounds prepared in the Examples of the present invention. Therefore, the compounds prepared in the Examples of the present invention had excellent precision treatment effect on mPTP-inactive Daoy cell. As mentioned above, when PPIF protein was inhibited, mPTP activity was significantly decreased, inhibiting the expression level of PPIF protein could decrease the mPTP activity in Daoy cell. Therefore, the compounds prepared in the Examples of the present invention had more significant inhibitory effect on Daoy cell with low expression of PPIF protein, while the compounds prepared in the Examples of the present invention had weaker inhibitory effect on Daoy cell with normal expression of PPIF protein, indicating that decreasing the expression level of PPIF protein could significantly improve the inhibitory effect of the compounds prepared in the Examples of the present invention on tumnor. Therefore, the compounds prepared in the Examples of the present invention had more significant inhibitory effect on Daoy cell with low expression of PPIF protein, i.e, the Daoy cell with low expression of PPIF protein was more sensitive to the compounds prepared in the Examples of the present invention compared with the Daoy cell with normal expression of PPIF protein. The compounds prepared in the Example of the present invention had excellent precision treatment on Daoy cell with low expression of PPIF protein.

### Example 16

The role of expression level of NNMT and DNMT1 in the sensitivity of NCI-H82 cell to the compounds prepared in the Examples of the present invention was determined

### Experiment method and result:

The expression of NNMT protein in NCI-H82 cell was overexpressed by inserting the NNMT gene into NCI-H82 cell using a viral vector, and the NNMT protein-overexpressing NCI-H82 cell (ov-NNMT NCI-H82 cell) was obtained. The expression of DNMT1 in NCI-H82 cell was knocked down by transfecting shRNA (the nucleotide sequence of shRNA was GATCCGGGATGAGTCCATCAAGGAAGATTCAAGAGATCTTCCTTGATGGACTCATCCT TTTTTG (SEQ ID No: 3)) using a viral vector, and the NCI-H82 cell with low expression of DNMT1 protein (sh-DNMT1 NCI-H82 cell) was obtained. The control NCI-H82 cell (Con-NCI-H82 cell) was obtained by transfecting NCI-H82 cell with empty virus vector without carrying NNMT gene and shRNA. The expressing content of NNMT protein and DNMT1 protein in the Con-NCI-H82 cell, the expressing content of NNMT protein in the ov-NNMT NCI-H82 cell, and the expressing content of DNMT1 protein in the sh-DNMT1 NCI-H82 cell were detected using Western Blot assay (as shown in Fig.3 and Fig.4 ), it could be seen from Fig.3 and Fig.4 that the NNMT protein was overexpressed in the ov-NNMT NCI-H82 cell and the expression of DNMT1 protein was knocked down (i.e, low expression) in the sh-DNMT1 NCI-H82 cell compared with the expression level of NNMT protein and DNMT protein in the Con-NCI-H82 cell.

The cell viability of Con-NCI-H82 cell, ov-NNMT NCI-H82 cell and sh-DNMT1 NCI-H82 cell was detected using the Promega CellTiter-Glo kit which reflected cell viability by measuring intracellular ATP content (as shown in Fig.5 and Fig.6) , it could be seen from Fig.5 and Fig.6 that the cell viability of Con-NCI-H82 cell, ov-NNMT NCI-H82 cell and sh-DNMT1 NCI-H82 cell was almost the same, and the difference in cell viability was not statistically significant.

The inhibitory effect (IC₅₀ value) of the compounds (dissolved in DMSO) prepared in the Examples of the present invention on Con-NCI-H82 cell, ov-NNMT NCI-H82 cell and sh-DNMT1 NCI-H82 cell was detected using Promega CellTiter-Glo kit which directly measured intracellular ATP content, the result was shown in Table 7 below:

**Table 7 the inhibitory effect of the compounds prepared in the Examples of the present invention on Con-NCI-H82 cell, ov-NNMT NCI-H82 cell and sh-DNMT1 NCI-H82 cell (IC₅₀, µM)**

| Compound | Con-NCI-H82 (µM) | ov-NNMT NCI-H82 (µM) | sh-DNMT1 NCI-H82 (µM) |
|---|---|---|---|
| compound AB31886 | 3.22 | 8.48 | 9.66 |
| compound AB31883 | 2.18 | 5.56 | 5.98 |
| compound AB31884 | 2.31 | 6.32 | 8.64 |
| compound AB31885 | 1.12 | 4.13 | 5.99 |
| compound AB31891 | 2.06 | 6.55 | 5.73 |
| compound AB31875 | 3.27 | 10.12 | 12.31 |
| compound AB35435 | 0.72 | 2.66 | 3.65 |
| compound AB31882 | 5.16 | 11.55 | 18.78 |
| compound AB31714 | 2.33 | 7.04 | 14.33 |
| compound AB35403 | 6.91 | 16.78 | 24.17 |
| compound AB31900 | 1.08 | 4.88 | 6.58 |
| compound AB31893 | 1.12 | 4.12 | 5.68 |
| compound AB31713 | 3.52 | 8.78 | 14.58 |

Note: IC₅₀ refered to 50% inhibiting concentration, ie, the concentration of the compound when 50% inhibitory effect was achieved. Con-NCI-H82 refered to the NCI-H82 cell transfected with empty virus vector without carrying NNMT gene and shRNA, which was used as control; ov-NNMT NCI-H82 refered to the NCI-H82 cell transfected with virus vector carrying NNMT gene, the NNMT protein was overexpressed in the ov-NNMT NCI-H82 cell; sh-DNMT1 NCI-H82 refered to the NCI-H82 cell transfected with virus vector carrying shRNA; the expression of DNMT1 was knocked down (i.e, low expression) in the sh-DNMT1 NCI-H82 cell.

As was shown in Table 7, the NNMT protein in NCI-H82 cell was overexpressed and the expression of DNMT1 in NCI-H82 cell was knocked down in the Example, the results further confirmed that the compounds prepared in the Examples of the present invention had significant inhibitory effect on tumor cell with low or no expression of NNMT gene and high expression of DNMT1 while the compounds prepared in the Examples of the present invention had weak inhibitory effect on tumor cells with high expression of NNMT gene and low expression of DNMT1. Decreasing the expression of NNMT gene and increasing the expression of DNMT1 in tumor could significantly improve the inhibitory effect of the compounds prepared in the Examples of the present invention on tumor on tumor, the expression level of NNMT gene in tumor cell was significantly negative correlation with the sensitivity of tumor cell to the compounds prepared in the Examples of the present invention, while the expression level of DNMT1 in tumor cell was significantly positive correlation with the sensitivity of tumor cell to the compounds prepared in the Examples of the present invention. Therefore, the compounds prepared in the Examples of the present invention had more significant inhibitory effect on tumor cell with low or no expression of NNMT gene and high expression of DNMT1, the tumor cell with low or no expression of NNMT gene and high expression of DNMT1 were highly sensitive to the compounds prepared in the Examples of the present invention. Therefore, the compounds prepared in the Examples of the present invention has excellent precision treatment on the tumor cell with low or no expression of NNMT gene and high expression of DNMT1.

### Example 17

The methylation level of DNA in cell was maintained by DNA methylation enzymes DNMT3a, DNMT3b and DNMT1. The original methylation of DNA was performed with DNMT3a and DNMT3b, DNMT1 could replicate and maintain methylated DNA with the help of protein UHRF1 (ubiquitin-like with PHD and ring finger domain 1). The correlation between the expression of NNMT and the expression of DNMT1, UHRF1, DNMT3a and DNMT3b in tumor was determined in the Example.

### Experiment method and result:

The expression of NNMT gene, DNMT1, UHRF1, DNMT3a and DNMT3b in various cells were obtained from a public database (Cancer Cell Line Encyclopedia, CCLE, 1019 cells in total). Then, the correlation between expression of NNMT and the expression of DNMT1, UHRF1, DNMT3a and DNMT3b in these cells was analyzed using bioinformatics, and the correlation between the expression level of NNMT gene and the expression level of DNMT1, UHRF1, DNMT3a and DNMT3b in each cell was analyzed, the experiment result was shown in Fig.7.

The Fig.7 showed the expression of NNMT was negatively correlated with the expression of DNA methylase and UHRF1 in each cell. Therefore, the tumor with high expression of DNA methylase and UHRF1 was highly sensitive to the compounds prepared in the Examples of the present invention, the compounds prepared in the Examples of the present invention has excellent precision treatment on the tumor with high expression of DNA methylase and UHRF1.

The above is an embodiment designed for a specific case of the present invention. It should be understood for the skilled in the art the improvements can be made without departing from the principles of the present invention, and these improvements should also be considered as the scope of protection of the present invention.

## Claims

1. A use of a compound of formula I, or an optical isomer thereof, or a racemate thereof, or a solvate thereof, or a pharmaceutically acceptable salt thereof, or a deuterated compound thereof in the preparation of a composition or a preparation for preventing and/or treating tumor; 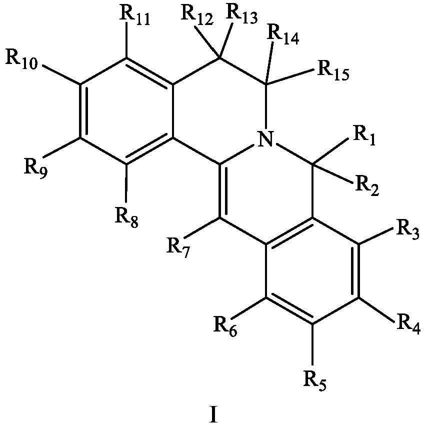 wherein,
R₁ and R₂ are each independently hydrogen, halogen, substituted or unsubstituted C1-C16 alkyl, substituted or unsubstituted C3-C16 cycloalkyl, substituted or unsubstituted 3-12 membered heterocycloalkyl, substituted or unsubstituted C1-C16 haloalkyl, substituted or unsubstituted C3-C16 halocycloalkyl, substituted or unsubstituted C6-C16 aryl-substituted or unsubstituted C1-C10 alkyl-, substituted or unsubstituted 5-16 membered heteroaryl-substituted or unsubstituted C1-C10 alkyl-, substituted or unsubstituted C2-C10 alkenyl-substituted or unsubstituted C1-C10 alkyl-;
R₃ and R₄ are each independently hydrogen, R₁₆-O-, R₁₆-S-;
R₅, R₆, R₇, R₈, R₁₁, R₁₂, R₁₃, R₁₄ and R₁₅ are each independently hydrogen, substituted or unsubstituted C1-C8 alkyl, halogen;
R₉ and R₁₀ are connected to form a substituted or unsubstituted 3-12 membered heterocycloalkane ring;
R₁₆ is hydrogen, substituted or unsubstituted C1-C16 alkyl, substituted or unsubstituted C3-C16 cycloalkyl;
the heterocyclic ring of the heterocycloalkyl, heteroaryl and heterocycloalkane ring has 1-4 (preferably 1, 2, 3 or 4) heteroatoms independently selected from the group consisting of N, O and S;
each "substituted" means that one or more (preferably 1, 2, 3, 4, 5, 6, 7, or 8) hydrogen atoms on the ring or group are independently substituted by substituent selected from the group consisting of C1-C8 alkyl, C3-C12 cycloalkyl, C1-C8 haloalkyl, C3-C8 halocycloalkyl, C3-C8 cycloalkoxyl, C3-C8 cycloalkylthio, C3-C8 halocycloalkoxyl, C3-C8 halocycloalkylthio, halogen, nitro, -CN, hydroxyl, sulfhydryl, amino, C1-C4 carboxyl, C2-C4 ester group, C2-C4 amide group, C1-C8 alkoxyl, C1-C8 alkylthio, C1-C8 haloalkoxyl, C1-C8 haloalkylthio, C6-C12 aryl, C6-C12 aryl-O-, 5-10 membered heteroaryl, 5-10 membered heteroaryl-O-, 5-10 membered heterocycloalkyl.

2. The use of claim 1, wherein the compound of formula I, or an optical isomer thereof, or a racemate thereof, or a solvate thereof, or a pharmaceutically acceptable salt thereof, or a deuterated compound thereof is selected from the following group:

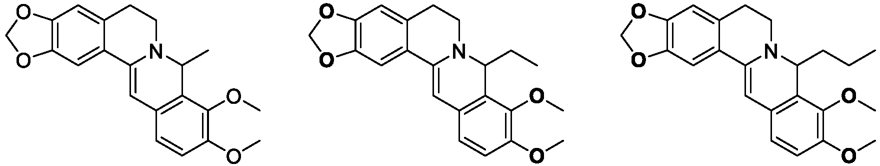

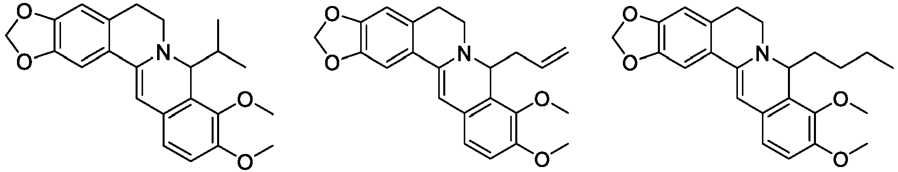

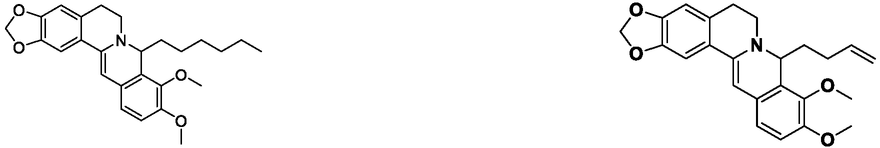

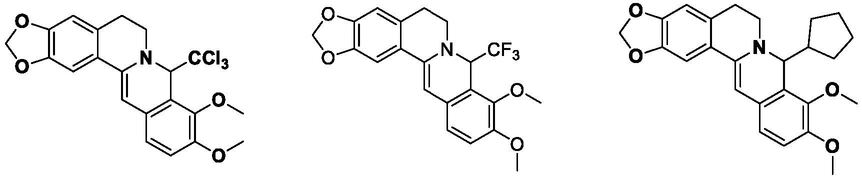

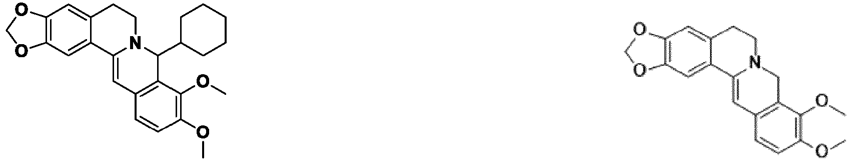

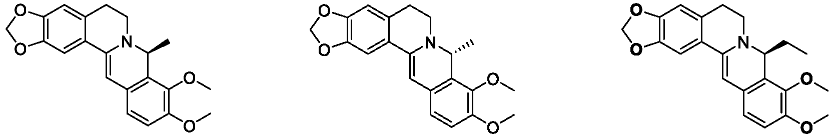

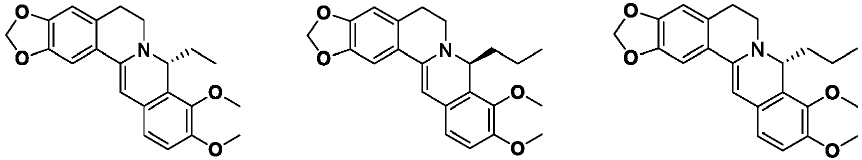

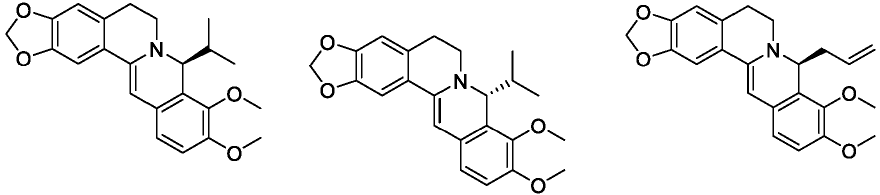

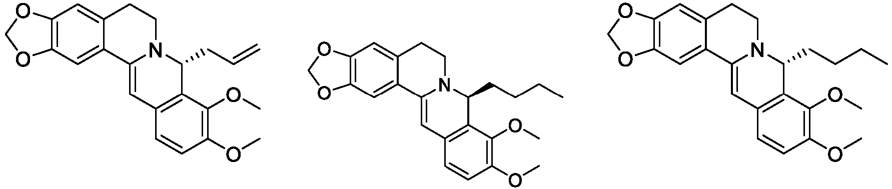

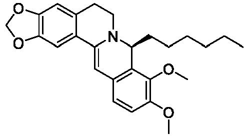

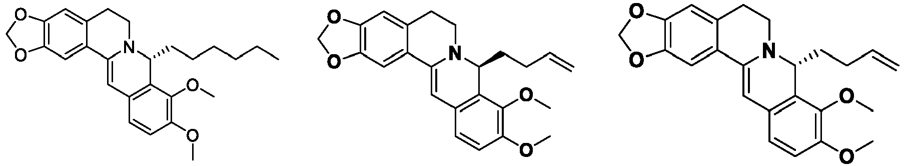

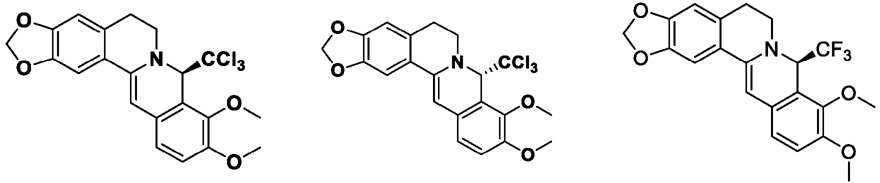

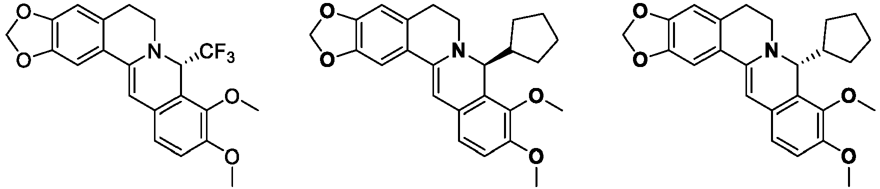

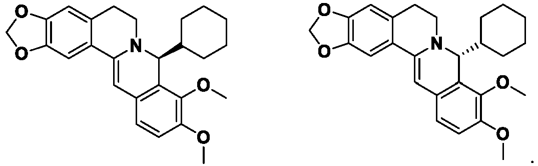

3. The use of claim 1, wherein the tumor comprises tumor with low expression, no expression, low activity or no activity of mitochondria permeability transition pore;
the tumor comprises tumor with low expression, no expression, low activity or no activity of peptidyl-prolyl cis-trans isomerase F;
the tumor comprises tumor with low or no expression of NNMT gene;
the tumor comprises tumor with high expression of DNA methylase;
the tumor comprises tumor with high expression of UHRFl;
the tumor comprises tumor with high methylation level of nucleotide site of NNMT gene; and/or
the tumor comprises tumor with high methylation level of DNA CpG site of NNMT gene.

4. The use of claim 3, wherein the DNA methylase is selected from the group consisting of DNMT1, DNMT3a, DNMT3b, and combinations thereof.

5. The use of claim 3, wherein the low expression or low activity of mitochondria permeability transition pore means the ratio (H1/H0) of the expression level or activity level Hlof mitochondria permeability transition pore in a cell ( e.g., tumor cell ) to the expression level or activity level H0 of mitochondria permeability transition pore in the same cell or a normal cell (e.g., para-tumor tissue cell) is <1.0, preferably ≤ 0.8, more preferably ≤ 0.7, more preferably ≤ 0.6, more preferably ≤ 0.5, more preferably ≤ 0.4, more preferably ≤ 0.3, more preferably ≤ 0.2, more preferably ≤ 0.1, more preferably ≤ 0.05, more preferably ≤ 0.01, more preferably ≤ 0.005, more preferably ≤ 0.001, more preferably ≤ 0.0001, more preferably ≤ 0.00001, more preferably ≤ 0.000001, more preferably ≤ 0.0000001;
the low expression or low activity of peptidyl-prolyl cis-trans isomerase F means the ratio (P1/P0) of the expression level or activity level Plof peptidyl-prolyl cis-trans isomerase F in a cell ( e.g., tumor cell) to the expression level or activity level P0 of peptidyl-prolyl cis-trans isomerase F in the same cell or a normal cell (e.g., para-tumor tissue cell) is <1.0, preferably ≤ 0.8, more preferably ≤ 0.7, more preferably ≤ 0.6, more preferably ≤ 0.5, more preferably ≤ 0.4, more preferably ≤ 0.3, more preferably ≤ 0.2, more preferably ≤ 0.1, more preferably ≤ 0.05, more preferably ≤ 0.01, more preferably ≤ 0.005, more preferably ≤ 0.001, more preferably ≤ 0.0001, more preferably ≤ 0.00001, more preferably ≤ 0.000001, more preferably ≤ 0.0000001;
the low or no expression of NNMT gene means the ratio (E1/E0) of the expression E1 of NNMT gene in a cell ( e.g., tumor cell ) to the expression E0 of NNMT gene in the same type of cell or a normal cell is <1.0, preferably ≤ 0.7, more preferably ≤ 0.6, more preferably ≤ 0.5, more preferably ≤ 0.4, more preferably ≤ 0.3, more preferably ≤ 0.2, more preferably ≤ 0.1, more preferably ≤ 0.05, more preferably ≤ 0.01, more preferably ≤ 0.005, more preferably ≤ 0.001, more preferably ≤ 0.0001, more preferably ≤ 0.00001, more preferably ≤ 0.000001, more preferably ≤ 0.0000001;
the tumor with high expression of DNA methylase means the ratio (A1/A0) of the expression level A1 of DNA methylase in the tumor cell to the expression level A0 of DNA methylase in the same type of cell or a normal cell is > 1.0, preferably ≥ 1.2, more preferably ≥ 1.5, more preferably ≥ 2, more preferably ≥ 3, more preferably ≥ 5, more preferably ≥ 8, more preferably ≥ 10, more preferably ≥ 15, more preferably ≥ 20, more preferably ≥ 30, more preferably ≥ 50, such as 2-50;
the tumor with high expression of UHRF1 means the ratio (F1/F0) of the expression level F1 of UHRF1 in the tumor cell to the expression level F0 of UHRF1 in the same type of cell or a normal cell is > 1.0, preferably ≥ 1.2, more preferably ≥ 1.5, more preferably ≥ 2, more preferably ≥ 3, more preferably ≥ 5, more preferably ≥ 8, more preferably ≥ 10, more preferably ≥ 15, more preferably ≥ 20, more preferably ≥ 30, more preferably ≥ 50, such as 2-50;
the high methylation level of nucleotide site of NNMT gene means the ratio (L1/L0) of the methylation level L1 of nucleotide site of NNMT gene in a cell ( e.g., tumor cell) to the methylation level L0 of nucleotide site of NNMT gene in the same type of cell or a normal cell (e.g., para-tumor tissue cell) is > 1.0, preferably ≥ 1.2, more preferably ≥ 1.5, more preferably ≥ 2, more preferably ≥ 3, more preferably ≥ 5, more preferably ≥ 8, more preferably ≥ 10, more preferably ≥ 15, more preferably ≥ 20, more preferably ≥ 30, more preferably ≥ 50, such as 2-50; and/or
the high methylation level of DNA CpG site of NNMT gene means the ratio (W1/W0) of the methylation level W1 of DNA CpG site of NNMT gene in a cell ( e.g., tumor cell) to the methylation level W0 of DNA CpG site of NNMT gene in the same type of cell or a normal cell (e.g., para-tumor tissue cell) is > 1.0, preferably ≥ 1.2, more preferably ≥ 1.5, more preferably ≥ 2, more preferably ≥ 3, more preferably ≥ 5, more preferably ≥ 8, more preferably ≥ 10, more preferably ≥ 15, more preferably ≥ 20, more preferably ≥ 30, more preferably ≥ 50, such as 2-50.

6. The use of claim 5, wherein the same cell comprises the same type of tumor cell with normal expression, high expression, normal activity or high activity of mitochondria permeability transition pore;
the same cell comprises the same type of tumor cell with normal expression, high expression, normal activity or high activity of peptidyl-prolyl cis-trans isomerase F;
the same type of cell comprises the same type of tumor cell with normal or high expression of NNMT gene;
the same type of cell comprises the same type of tumor cell with normal or low expression of DNA methylase;
the same type of cell comprises the same type of tumor cell with normal or low expression of UHRF1;
the same type of cell comprises the same type of tumor cell with normal or low methylation level of nucleotide site of NNMT gene; and/or
the same type of cell comprises the same type of tumor cell with normal or low methylation level of DNA CpG site of NNMT gene.

7. The use of claim 4, wherein the tumor is selected from the group consisting of lung cancer, renal carcinoma, breast cancer, colon cancer, lymphoma, leukemia, pancreatic cancer, brain tumor, liver cancer, prostate cancer, and combinations thereof.

8. A marker for determining whether the compound of formula I, or an optical isomer thereof, or a racemate thereof, or a solvate thereof, or a pharmaceutically acceptable salt thereof, or a deuterated compound thereof of claim 1 is suitable for use in the prevention and/or treatment of patient tumor, the marker comprises mitochondria permeability transition pore, peptidyl-prolyl cis-trans isomerase F, NNMT gene, DNA methylase, UHRF1, the methylation of nucleotide site of NNMT gene, and/or the methylation of DNA CpG site of NNMT gene.

9. A use of a detection kit in the preparation of concomitant diagnose kit for determining whether the compound of formula I, or an optical isomer thereof, or a racemate thereof, or a solvate thereof, or a pharmaceutically acceptable salt thereof, or a deuterated compound thereof of claim 1 is suitable for use in the prevention and/or treatment of patient tumor;
the detection kit comprises:
(i) a detection reagent for detecting the expression level or activity of mitochondria permeability transition pore, the expression level or activity of peptidyl-prolyl cis-trans isomerase F, the expression level of NNMT gene, the expression level of DNA methylase, the expression level of UHRF1, the methylation level of nucleotide site of NNMT gene, and/or the methylation level of DNA CpG site of NNMT gene;
the concomitant diagnose kit further comprises instruction or label, the instruction or label records as follows:
the compound of formula I, or an optical isomer thereof, or a racemate thereof, or a solvate thereof, or a pharmaceutically acceptable salt thereof, or a deuterated compound thereof according to the first aspect of the present invention is suitable for use in the prevention and/or treatment of patient tumor with low expression, no expression, low activity or no activity of mitochondria permeability transition pore, low expression, no expression, low activity or no activity of peptidyl-prolyl cis-trans isomerase F, low or no expression of NNMT gene, high expression of DNA methylase, high expression of UHRFl, high methylation level of nucleotide site of NNMT gene, and/or high methylation level of DNA CpG site of NNMT genel; and/or
the compound of formula I, or an optical isomer thereof, or a racemate thereof, or a solvate thereof, or a pharmaceutically acceptable salt thereof, or a deuterated compound thereof according to the first aspect of the present invention is not suitable for use in the prevention and/or treatment of patient tumor with high expression or high activity of mitochondria permeability transition pore, high expression or high activity of peptidyl-prolyl cis-trans isomerase F, high expression of NNMT gene, low expression of DNA methylase, low expression of UHRFl, low methylation level of nucleotide site of NNMT gene, and/or low methylation level of DNA CpG site of NNMT gene.

10. A medicine kit, wherein the medicine kit comprises:
(i) a detection reagent for detecting the expression level or activity of mitochondria permeability transition pore, the expression level or activity of peptidyl-prolyl cis-trans isomerase F, the expression level of NNMT gene, the expression level of DNA methylase, the expression level of UHRF1, the methylation level of nucleotide site of NNMT gene, and/or the methylation level of DNA CpG site of NNMT gene; and
(ii) the compound of formula I, or an optical isomer thereof, or a racemate thereof, or a solvate thereof, or a pharmaceutically acceptable salt thereof, or a deuterated compound thereof of claim 1.

11. A use of mitochondria permeability transition pore inhibitor, peptidyl-prolyl cis-trans isomerase F inhibitor, NNMT gene inhibitor, DNA methylase promoter, UHRF1 promoter, methylation promoter of nucleotide site of NNMT gene, and/or methylation promoter of DNA CpG site of NNMT gene in the preparation of a composition or a preparation for enhancing the anti-tumor effect of anti-tumor drug;
the anti-tumor drug is the compound of formula I, or an optical isomer thereof, or a racemate thereof, or a solvate thereof, or a pharmaceutically acceptable salt thereof, or a deuterated compound thereof of claim 1.

12. A composition, wherein the composition comprises:
(1) a first active ingredient, the first active ingredient comprises anti-tumor drug; and
(2) a second active ingredient, the second active ingredient comprises mitochondria permeability transition pore inhibitor, peptidyl-prolyl cis-trans isomerase F inhibitor, NNMT gene inhibitor, DNA methylase promoter, UHRF1 promoter, methylation promoter of nucleotide site of NNMT gene, and/or methylation promoter of DNA CpG site of NNMT gene;
the anti-tumor drug is the compound of formula I, or an optical isomer thereof, or a racemate thereof, or a solvate thereof, or a pharmaceutically acceptable salt thereof, or a deuterated compound thereof of claim 1.

13. A composition, wherein the composition comprises:
(1) a first active ingredient, the first active ingredient comprises anti-tumor drug; and
(2) a second active ingredient, the second active ingredient comprises mitochondria permeability transition pore inhibitor, peptidyl-prolyl cis-trans isomerase F inhibitor, NNMT gene inhibitor, DNA methylase promoter, UHRF1 promoter, methylation promoter of nucleotide site of NNMT gene, and/or methylation promoter of DNA CpG site of NNMT gene;
the anti-tumor drug is the compound of formula I, or an optical isomer thereof, or a racemate thereof, or a solvate thereof, or a pharmaceutically acceptable salt thereof, or a deuterated compound thereof of claim 1.
